(19) Europäisches Patentamt
European Patent Office
Office européen des brevets

(11) Veröffentlichungsnummer: **0 337 263 B1**

# (12) EUROPÄISCHE PATENTSCHRIFT

(45) Veröffentlichungstag der Patentschrift: **17.03.93**

(51) Int. Cl.[5]: **C07D 261/18**, C07D 275/02, A01N 43/80, C07D 413/12, C07D 413/04, C07D 417/12

(21) Anmeldenummer: **89105924.8**

(22) Anmeldetag: **05.04.89**

Die Akte enthält technische Angaben, die nach dem Eingang der Anmeldung eingereicht wurden und die nicht in dieser Patentschrift enthalten sind.

## (54) Isoxazol(Isothiazol)-5-carbonsäure-amide.

(30) Priorität: **13.04.88 DE 3812225**

(43) Veröffentlichungstag der Anmeldung: **18.10.89 Patentblatt 89/42**

(45) Bekanntmachung des Hinweises auf die Patenterteilung: **17.03.93 Patentblatt 93/11**

(84) Benannte Vertragsstaaten: **BE CH DE ES FR GB IT LI NL**

(56) Entgegenhaltungen:
**EP-A- 0 026 873**
**EP-A- 0 193 131**

**JOURNAL OF THE CHEMICAL SOCIETY, PERKIN TRANSACTIONS I, 1987, Seiten 1005-1009, London, GB; R. NESI et al.: "A new spiro annellation reaction in the isoxazole series: Applications and limits. Part 2. Reactivity of ethyl 4-nitro-3-phenylisoxazole-5-carboxylate with nitrogen binucleophiles"**

(73) Patentinhaber: **BASF Aktiengesellschaft**
**Carl-Bosch-Strasse 38**
**W-6700 Ludwigshafen(DE)**

(72) Erfinder: **Freund, Wolfgang, Dr.**
**Johann-Gottlieb-Fichte-Strasse 71**
**W-6730 Neustadt(DE)**
Erfinder: **Kuekenhoehner, Thomas, Dr.**
**Seidelstrasse 2**
**W-6710 Frankenthal(DE)**
Erfinder: **Hamprecht, Gerhard, Dr.**
**Rote-Turm-Strasse 28**
**W-6940 Weinheim(DE)**
Erfinder: **Wuerzer, Bruno, Dr.**
**Ruedigerstrasse 13**
**W-6701 Otterstadt(DE)**
Erfinder: **Westphalen, Karl-Otto, Dr.**
**Mausbergweg 58**
**W-6720 Speyer(DE)**
Erfinder: **Meyer, Norbert**
**Dossenheimer Weg 22**
**W-6802 Ladenburg(DE)**
Erfinder: **Theobald, Hans, Dr.**
**Oueichstrasse 6**
**W-6703 Limburgerhof(DE)**

EP 0 337 263 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**JOURNAL OF HETEROCYCLIC CHEMISTRY, Band 22, November-Dezember 1985, Seiten 1561-1565, US; A. CAMPARINI et al.: "Synthesis and photochemical reactivity of 3-methylisoxazolo[4,5-d]pyridazines"**

**JOURNAL OF THE CHEMICAL SOCIETY, 1959, Seiten 3061-3072, London, GB; A. ADAMS et al.: "Isothiazole: a new mononuclear heterocyclic system"**

**JOURNAL OF THE CHEMICAL SOCIETY, PERKIN TRANSACTIONS I, 1982, Seiten 2391-2394, London, GB; A. CAMPARINI et al.: "Syntheses and reactivities of 3-methylisoxazolo[4,5-b]pyridines"**

## Beschreibung

Die vorliegende Erfindung betrifft substituierte Isoxazol- und Isothiazol-5-carbonsäureamide und ihre Verwendung zur Bekämpfung unerwünschten Pflanzenwuchses.

Isoxazol- und Isothiazol-carbonsäuren bzw. deren Derivate sind bekannt. Dies sind die 5-Aminocarbonyl-3-methyl-4-isoxazolcarbonsäure, der 5-Aminocarbonyl-3-methyl-4-isoxazolcarbonsäureethylester, das 4,5-Isothiazoldicarboxamid sowie die 5-Carbamoyl-4-isothiazolcarbonsäure (J. Chem. Soc. Perkin Trans. I, 1982, 2391; J. Heterocyclic. Chem. 22, 1561 (1985); J. Chem. Soc. 1959, 3061). Mögliche Verwendungen dieser Substanzen sind nicht beschrieben.

Es wurde gefunden, daß Isoxazol(Isothiazol)-5-carbonsäureamide der Formel Ia

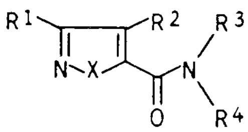 (Ia),

in der

X Sauerstoff oder Schwefel,

$R^1$ Wasserstoff,
gegebenenfalls durch $C_1$-$C_3$-Alkoxy, $C_1$-$C_3$-Halogenalkoxy, Halogen, Cyano oder Phenyl, das durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Cyano oder Nitro substituiert sein kann, substituiertes $C_1$-$C_{10}$-Alkyl,
$C_1$-$C_4$-Alkoxy,
gegebenenfalls durch $C_1$-$C_4$-Alkyl oder Halogen substituiertes $C_3$-$C_8$-Cycloalkyl,
einen 5- bis 6-gliedrigen heterocyclischen Rest mit einem oder zwei Heteroatomen, ausgewählt aus der Gruppe Sauerstoff, Schwefel und Stickstoff, der durch $C_1$-$C_4$-Alkyl, Carboxyl oder $C_1$-$C_4$-Alkoxycarbonyl substituiert sein kann,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes Phenyl,

$R^2$ Formyl, 4,5-Dihydro-oxazol-2-yl oder einen Rest der Formel $COYR^5$ oder $CONR^6R^7$, wobei

Y für Sauerstoff oder Schwefel,

$R^5$ für Wasserstoff,
$C_1$-$C_8$-Alkyl, das durch $C_1$-$C_4$-Alkoxy,
$C_1$-$C_4$-Alkoxy-$C_1$-$C_4$-alkoxy, Halogen, Cyano, Hydroxy, Trimethylsilyl, $C_1$-$C_4$-Alkylthio, $C_1$-$C_4$-Alkylamino, $C_1$-$C_4$-Dialkylamino, $C_1$-$C_4$-Alkylsulfinyl, $C_1$-$C_4$-Alkylsulfonyl, Carboxyl, $C_1$-$C_4$-Alkoxycarbonyl, $C_1$-$C_4$-Dialkylaminocarbonyl,
$C_1$-$C_4$-Dialkoxyphosphonyl, Alkaniminoxy, Benzyloxy, gegebenenfalls durch $C_1$-$C_4$-Alkyl, $C_1$-$C_3$-Alkoxy oder Halogen substituiertes Benzoyl oder gegebenenfalls durch $C_1$-$C_4$-Alkyl, $C_1$-$C_4$-Alkoxy, $C_1$-$C_4$-Halogenalkyl, Halogen, Nitro oder Cyano substituiertes Phenyl, durch Thienyl, Furyl, Tetrahydrofuryl, Phthalimido oder Pyridyl substituiert sein kann,
gegebenenfalls durch Phenyl, das durch $C_1$-$C_4$-Alkyl, $C_1$-$C_4$-Alkoxy, $C_1$-$C_4$-Halogenalkyl, Halogen, Nitro oder Cyano substituiert sein kann, substituiertes $C_3$-$C_8$-Alkenyl,
$C_3$-$C_6$-Halogenalkenyl,
gegebenenfalls durch Hydroxy oder $C_1$-$C_4$-Alkoxy substituiertes $C_3$-$C_8$-Alkinyl,
$C_3$-$C_6$-Cycloalkyl,
$C_5$-$C_6$-Cycloalkenyl,
gegebenenfalls durch $C_1$-$C_4$-Alkyl, $C_1$-$C_4$-Alkoxy, $C_1$-$C_4$-Halogenalkyl, Halogen, Nitro, Cyano, $C_1$-$C_4$-Alkoxycarbonyl oder Acylamino substituiertes Phenyl,
einen 5- bis 6-gliedrigen heterocyclischen Rest mit einem oder zwei Heteroatomen, ausgewählt aus der Gruppe Sauerstoff, Schwefel und Stickstoff oder einen Benzotriazolrest,
$C_6$-$C_7$-Cycloalkanimino, Phthalimido, Succinimido,
für die Reste

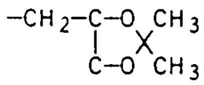 , 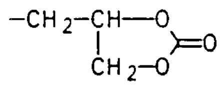 , $-CH_2-CH(OH)-CH_2(OH)$ ,

$-CH_2-CH(OH)-CH_2(OH)$,
für ein Äquivalent eines Kations aus der Gruppe der Alkali-, Erdalkalimetalle, Mangan, Kupfer, Eisen, Ammonium und substituiertes Ammonium oder für den Rest

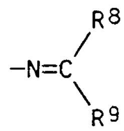 ,

wobei $R^8$ und $R^9$ unabhängig voneinander $C_1$-$C_4$-Alkyl, $C_2$-$C_6$-Alkoxyalkyl, $C_3$-$C_6$-Cycloalkyl, Phenyl, Furyl oder zusammen eine Methylenkette der Formel $-(CH_2)_m-$ mit $m = 4$ bis 7 Kettengliedern bedeuten und $R^9$ zusätzlich Wasserstoff bedeutet,

$R^6$ für Wasserstoff, $C_1$-$C_8$-Alkyl oder $C_3$-$C_8$-Cycloalkyl und

$R^7$ für Wasserstoff oder $C_1$-$C_8$-Alkyl stehen oder wobei

$R^6$ und $R^7$ eine Methylenkette mit 4 oder 5 Gliedern bilden,

$R^3$ Wasserstoff, gegebenenfalls durch Hydroxy, Halogen, $C_1$-$C_4$-Alkoxy, $C_1$-$C_4$-Alkylthio oder $C_1$-$C_4$-Dialkylamino substituiertes $C_1$-$C_8$-Alkyl
oder gegebenenfalls durch $C_1$-$C_4$-Alkyl, Halogen oder $C_1$-$C_4$-Halogenalkyl substituiertes $C_3$-$C_8$-Cycloalkyl und

$R^4$ Wasserstoff, Hydroxyl, $C_1$-$C_4$-Alkoxy,
gegebenenfalls durch $C_1$-$C_4$-Alkoxy, $C_1$-$C_4$-Halogenalkoxy, $C_1$-$C_4$-Alkylthio, $C_1$-$C_4$-Dialkylamino, Halogen, $C_3$-$C_6$-Cycloalkyl oder Phenyl, das durch Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio substituiert sein kann, substituiertes $C_1$-$C_{10}$-Alkyl,
gegebenenfalls durch Halogen oder $C_1$-$C_4$-Alkoxy substituiertes $C_3$-$C_{10}$-Alkenyl oder $C_3$-$C_{10}$-Alkinyl,
gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes $C_3$-$C_8$-Cycloalkyl,
$C_1$-$C_4$-Dialkylamino, einen gegebenenfalls durch $C_1$-$C_6$-Alkyl, $C_1$-$C_6$-Halogenalkyl oder Halogen substituierten 3- bis 6-gliedrigen heterocyclischen Rest mit einem oder zwei Heteroatomen, ausgewählt aus der Gruppe Sauerstoff, Schwefel und gegebenenfalls durch Methyl substituierter Stickstoff, Naphthyl,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio,
$C_1$-$C_6$-Halogenalkylthio, Halogen, Nitro, Cyano, Formyl, $C_1$-$C_6$-Alkanoyl oder $C_1$-$C_6$-Halogenalkanoyl substituiertes Phenyl bedeuten

oder

$R^3$ und $R^4$ gemeinsam einen Rest der Struktur $-(CH_2)_n-Y_p-(CH_2)_q-$, wobei n und q 1, 2 oder 3, p 0 oder 1 und Y Sauerstoff, Schwefel oder N-Methyl bedeuten, oder den Rest der Formel

$-(CH_2)_3-CO-$

bilden,
sowie deren umweltverträgliche Salze, herbizid wirksam sind.

Die Methyl-, Alkoxy-, Alkenyl- und Alkinylreste für $R^1$, $R^3$, $R^4$, $R^5$, $R^6$ und $R^7$ können unverzweigt oder verzweigt sein und enthalten vorzugsweise 1 bis 4 C-Atome. Entsprechendes gilt für die Alkylreste, die als Substituenten in den Resten $R^1$, $R^3$, $R^4$, $R^5$, $R^6$ und $R^7$ enthalten sein können, sowie für die Alkylgruppen in den Halogenalkyl-, Alkoxy-, Halogenalkoxy, Alkylthio-, Halogenalkylthio, Dialkylamino-, Alkanoyl-, Halogenalkanoyl- und Alkoxycarbonylresten.

Als Halogensubstituenten kommmen vorzugsweise Chlorsubstituenten in Betracht.

Die heterocyclischen Reste für $R^1$ sind gesättigt oder ungesättigt. In Betracht kommen beispielsweise Tetrahydropyranyl, Tetrahydrofuryl, Pyrazolyl, Thienyl, Furyl, Pyridyl und Tetrahydrofuryl. Diese Reste können durch $C_1$-$C_4$-Alkyl-, Carboxyl- oder $C_1$-$C_4$-Alkylcarbonylgruppen substituiert sein.

Die heterocyclischen Reste für $R^5$ können gegebenenfalls gesättigt oder ungesättigt sein. Geeignete Reste sind Thienyl, Furyl, Tetrahydrofuryl, Triazolyl, Imidazolyl, Tetrahydropyranyl, Pyridyl, Morpholino und Piperidino.

Gesättigte oder ungesättigte heterocyclische Reste für $R^4$ sind beispielsweise Tetrahydropyranyl, Tetrahydrofuryl, Thiazolyl, Pyridyl, Morpholino, Piperidino, Pyrimidyl.

Die erfindungsgemäßen Verbindungen I können beispielsweise mit anorganischen und organischen Säuren oder mit Alkylhalogeniden Additionssalze bilden oder sie können, sofern einer der Substituenten saure Eigenschaften hat, mit anorganischen und organischen Basen zu Salzen umgesetzt werden. Die entsprechenden Salze gehören ebenfalls zur Erfindung.

Als herbizide Wirkstoffe bevorzugte Isoxazol(Isothiazol)-5-carbonsäureamide sind solche der Formel Ia, in der $R^3$ Wasserstoff bedeutet.

Weiterhin sind Verbindungen der Formel Ia als Wirkstoffe bevorzugt, bei denen X Sauerstoff oder Schwefel, $R^1$ Wasserstoff oder $C_1$-$C_4$-Alkyl, $R^2$ $COYR^5$, $R^3$ Wasserstoff, $R^4$ $C_1$-$C_4$-Alkyl oder $C_3$-$C_8$-Cycloalkyl bedeuten. Bei diesen Verbindungen bedeutet $R^5$ vorzugsweise Wasserstoff, $C_1$-$C_4$-Alkyl, gegebenenfalls durch $C_1$-$C_4$-Alkyl, $C_1$-$C_4$-Halogenalkyl, $C_1$-$C_4$-Alkoxy, $C_1$-$C_4$ Alkylthio oder Halogen substituiertes Phenyl oder den Rest

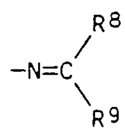

,

wobei wiederum $R^8$ und $R^9$ vorzugsweise für $C_1$-$C_4$-Alkyl stehen.

Isoxazol(Isothiazol)-5-carbonsäureamide der Formel

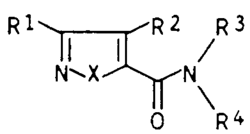

(I),

in der X, $R^1$, $R^2$, $R^3$ und $R^4$ die für Formel Ia angegebenen Bedeutungen haben, mit der Maßgabe, daß X Schwefel ist, wenn $R^1$ $CH_3$, $R^2$ COOH oder $COOC_2H_5$ und $R^3$ und $R^4$ Wasserstoff bedeuten, und daß X Sauerstoff ist, wenn $R^1$ Wasserstoff, $R^2$ COOH oder $CONH_2$ und $R^3$ und $R^4$ Wasserstoff bedeuten, sind neu.

Die Isoxazol(Isothiazol)-5-carbonsäureamide der Formel I bzw. Ia können auf folgenden Wegen hergestellt werden:

1. Ein Verfahren zur Herstellung von Verbindungen der Formel Ib und Ia, in der $R^2$ $COOR^5$ und $R^5$ Wasserstoff oder $C_1$-$C_8$-Alkyl bedeuten (vgl. Schema 1), beruht auf der Umsetzung eines Isoxazol- oder Isothiazol-4,5-dicarbonsäuredialkylesters II ($R^8$ = $C_1$-$C_8$-Alkyl) mit wäßriger Base und anschließende Umsetzung mit Mineralsäure zu einer Carbonsäure III. Als Dicarbonsäureester II kommen insbesondere Niedrigalkylester ($R^5$ = $R^8$ = $C_1$-$C_4$-Alkyl) in Betracht, wobei Dimethylester und Diethylester besonders bevorzugt sind.

Die Reaktion wird so durchgeführt, daß man einen Dicarbonsäuredialkylester II bei Temperaturen zwischen etwa 0 und 80 °C, vorzugsweise zwischen 0 und 50 °C, in einem organischen Lösungsmittel, z.B. Methanol oder Ethanol, mit einer starken Base, z.B. NaOH, KOH oder $Ca(OH)_2$, behandelt. Im allgemeinen wird dabei etwa 1 Äquivalent der starken Base in wäßriger Lösung eingesetzt. Nach erfolgter Umsetzung wird abgekühlt und mit einer starken Mineralsäure, z.B. Salzsäure oder Schwefelsäure, angesäuert. Die entstehende Carbonsäure III kann auf übliche Art und Weise z.B. durch Absaugen oder durch Extraktion mit einem organischen Lösungsmittel isoliert werden.

Zur Überführung der Carbonsäure III in das Carbonsäurehalogenid IV bringt man die Säure III in üblicher Art und Weise mit einem anorganischen Säurehalogenid, wie Thionylchlorid, Phosphortri- oder Phosphorpentahalogeniden, zur Reaktion, wobei die Chloride bevorzugt sind. Dabei wird zweckmäßigerweise das anorganische Säurehalogenid in 1 bis 5 Moläquivalenten, vorzugsweise 1 bis 2 Moläquivalenten, eingesetzt. Man kann ohne Lösungsmittel oder in Gegenwart eines inerten organischen Lösungsmittels wie z.B. Benzol oder Toluol bei Temperaturen zwischen Raumtemperatur und der Siedetemperatur des anorganischen Säurehalogenids bzw. des inerten organischen Lösungsmittels arbeiten. In manchen Fällen kann der Zusatz eines Katalysators, wie Dimethylformamid oder 4-Dimethylaminopyridin, von Vorteil sein. Nach Beendigung der Reaktion kann das Säurehalogenids IV auf übliche Art und Weise isoliert werden, z.B. durch Abdestillation des Überschusses an anorganischem Säurehalogenid und des organischen Lösungsmittels und nachfolgende Destillation des Säurechlorids IV bei Normaldruck oder vermindertem Druck.

Die Carbonsäureamide Ib erhält man aus den Carbonsäurehalogeniden durch Umsetzung mit einem Amin V. Dabei geht man zweckmäßigerweise so vor, daß man das Carbonsäurehalogenid in einem inerten organischen Lösungsmittel wie Dichlorethan, oder einem Ether wie Diethylether oder Methyl-tert.-butylether mit einem Amin V, ebenfalls gelöst in einem organischen Lösungsmittel, zur Reaktion bringt. Dabei setzt man das Amin V zweckmäßigerweise in 2- bis 5-fach molarer Menge, vorzugsweise 2- bis 3-fach molarer Menge ein, um den entstehenden Halogenwasserstoff zu binden. Man kann auch in Gegenwart einer Hilfsbase wie einem tertiären Amin, z.B. Triethylamin, arbeiten. In diesem Fall genügen 1 bis 1,5 Moläquivalente Amin V. Die Reaktionstemperatur kann zwischen 0 und 50 °C, vorzugsweise zwischen 0 und 20 °C betragen. Die Reaktion ist im allgemeinen nach 1 bis 12 Stunden beendet. Das Gemisch kann wie üblich aufgearbeitet werden, beispielsweise durch Hydrolyse mit Wasser und Extraktion des Produktes der Formel Ib mit einem organischen Lösungsmittel und Einengen des organischen Lösungsmittels. Zur Reinigung kann das Produkt der Formel Ib beispielsweise umkristalliert oder chromatographiert werden.

Aus den 4-Alkoxycarbonyl-isoxazol-5-carbonsäureamiden bzw. 4-Alkoxycarbonyl-isothiazol-5-carbonsäureamiden Ib lassen sich die freien Carbonsäuren Ic durch Umsetzung mit wäßrigen Basen und anschließender Umsetzung mit Mineralsäuren erhalten. Die Reaktion wird so durchgeführt, daß man den Ester Ib bei Temperaturen zwischen 0 und 80 °C, vorzugsweise zwischen 0 und 50 °C, in einem organischen Lösungsmittel wie z.B. Methanol oder Ethanol mit einer Base wie z.B. NaOH, KOH oder $Ca(OH)_2$ behandelt. Im allgemeinen werden dabei etwa 1 bis 3 Äquivalente, vorzugsweise 1 bis 1,5 Äquivalente der starken Base in wäßriger Lösung eingesetzt. Nach erfolgter Umsetzung wird unter Kühlung mit einer starken Mineralsäure, z.B. Salzsäure oder Schwefelsäure, angesäuert. Die entstehenden Carbonsäuren Ic können durch Absaugen oder durch Extraktion mit einem organischen Lösungsmittel und Einengen dieses organischen Lösungsmittels isoliert werden. Zur weiteren Reinigung der Säuren Ic können diese umkristallisiert oder chromatographiert werden.

Schema 1:

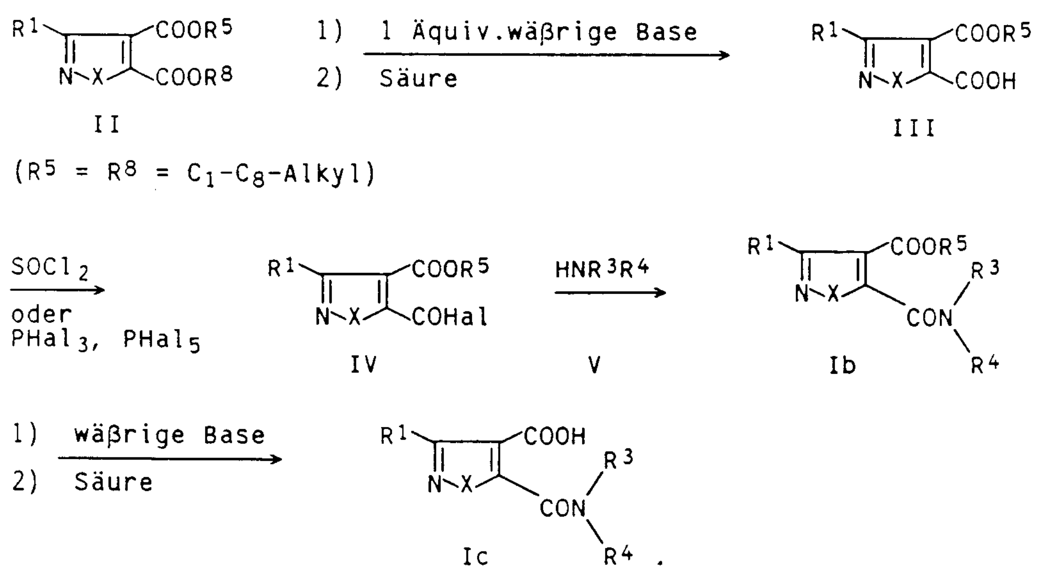


Die für dieses Verfahren als Ausgangsmaterial benötigten Isoxazol- und Isothiazol-4,5-dicarbonsäuredialkylester II sind literaturbekannt (J. Org. Chem. 43, 3736 (1978); Chem. Pharm. Bull. 28, 3296 (1980); Tetrahedron 30, 1365 (1974)) oder können nach allgemein literaturbekannten Methoden hergestellt

werden.

2. Ein weiteres Verfahren zur Herstellung der Verbindungen Id beruht auf der Umsetzung eines Isoxazol- bzw. Isothiazol-5-carbonsäurehalogenids VI mit einem Amin V. Als Carbonsäurehalogenide VI sind die Chloride bevorzugt. Dabei geht man zweckmäßigerweise so vor, daß man das Carbonsäurehalogenid in einem inerten organischen Lösungsmittel wie Dichlormethan, oder einem Ether wie Diethylether oder Methyl-tert.butylether mit einem Amin V, ebenfalls gelöst in einem organischen Lösungsmittel, zur Reaktion bringt. Dabei setzt man das Amin V zweckmäßigerweise in 2- bis 5-fach molarer Menge, vorzugsweise 2- bis 3-fach molarer Menge, ein, um den entstehenden Halogenwasserstoff zu binden. Man kann auch in Gegenwart einer Hilfsbase wie z.B. einem tertiären Amin (Triethylamin) arbeiten. In diesem Fall genügen 1 bis 1,5 Moläquivalente Amin V. Die Reaktionstemperatur kann zwischen 0 und 50 °C, vorzugsweise zwischen 0 und 20 °C betragen. Die Reaktion ist im allgemeinen nach 1 bis 12 Stunden beendet. Das Gemisch kann wie üblich aufgearbeitet werden, beispielsweise durch Hydrolyse mit Wasser und Extraktion des Produktes VII mit einem organischen Lösungsmittel und Einengen des organischen Lösungsmittels.

Aus den Isoxazol- bzw. Isothiazolamiden VII erhält man die 5-Aminocarbonyl-isoxazol-4-carbonsäuren bzw. 5-Aminocarbonyl-isothiazol-4-carbonsäuren der Formel Id, durch Umsetzung mit Alkyllithium, vorzugsweise unter Zugabe eines unter den Reaktionsbedingungen inerten Lösungsmittels, wie Diethylether oder Tetrahydrofuran. In der Regel arbeitet man unter einer Stickstoffatmosphäre bei Temperaturen zwischen -70 und -80 °C. Die Alkyllithiumverbindung wird bei diesem Verfahren im allgemeinen in 2 -3-fach molarer Menge bezogen auf eingesetztes Amid der Formel VII verwendet. Nach vollständiger Umsetzung wird das Gemisch mit Kohlendioxid behandelt, vorzugsweise in einem inerten Lösungsmittel wie Diethylether oder z.B. Tetrahydrofuran, wobei man die gewünschten Produkte der Formel Id, in der $R^2$ Carboxyl bedeutet, erhält.

Nach dem gleichen Verfahren können auch Isoxazol- und Isothiazolamide der Formel Id, bei denen $R^2$ für Formyl steht, erhalten werden, wenn anstelle des Kohlendioxids Dimethylformamid eingesetzt wird. Man erhält nach üblicher Aufarbeitung substituierte 4-Formyl-isoxazol-5-carbonamide bzw. 4-Formyl-isothiazol-5-carbonamide der Formel Id.

Schema 2:

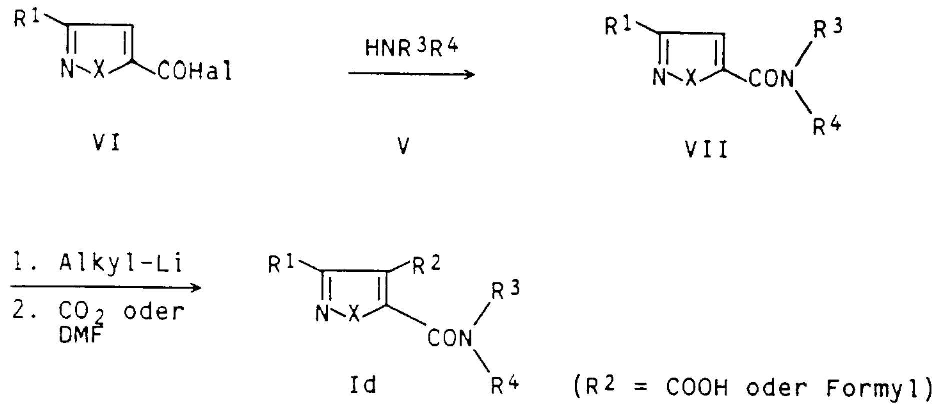


Die für dieses Verfahren als Ausgangsmaterial benötigten Isoxazol- und Isothiazol-5-carbonsäurehalogenide VI sind literaturbekannt oder können aus den entsprechenden Carbonsäuren VIII auf übliche Art und Weise, wie bereits oben beschrieben, hergestellt werden.

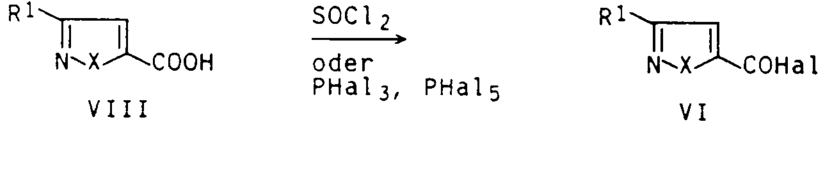


Die hierfür benötigten Carbonsäuren VIII sind ebenfalls literaturbekannt (Chemische Berichte 106, 3345 (1973), J. Chem. Soc. 1959, 3061, J. Chem. Soc. 1963, 2032; Adv. in Heterocyclic Chem. 14, 1 (1972)) oder können nach allgemein literaturbekannten Methoden, z.B. aus den entsprechenden Alkoholen oder Aldehyden durch Oxidation oder den entsprechenden Nitrilen durch Hydrolyse hergestellt werden.

3. Ein weiteres Verfahren führt zu Verbindungen Ie, in der $R^2$ $COOR^5$ und $R^5$ gegebenenfalls durch $C_1$-$C_4$-Alkoxy, $C_1$-$C_4$-Alkoxy-$C_1$-$C_4$-alkoxy, Halogen, $C_1$-$C_4$-Alkylthio, $C_1$-$C_4$-Alkylsulfinyl, $C_1$-$C_4$-Alkylsulfonyl, $C_1$-$C_4$Alkoxycarbonyl, Benzyloxy, oder durch Phenyl, das durch $C_1$-$C_4$-Alkyl, $C_1$-$C_4$-Alkoxy, $C_1$-$C_4$-Halogenalkyl, Halogen, Nitro oder Cyano substituiert sein kann, substituiertes $C_1$-$C_8$-Alkyl, $C_3$-$C_6$-Cycloalkyl, gegebenenfalls durch Phenyl, das durch $C_1$-$C_4$-Alkyl, $C_1$-$C_4$-Alkoxy, $C_1$-$C_4$-Halogenalkyl, Halogen, Nitro oder Cyano substituiert sein kann, substituiertes $C_3$-$C_8$-Alkenyl, $C_3$-$C_8$-Alkinyl, $C_6$-$C_7$-Cycloalkanimino, Succinimido oder einen Rest der Formel

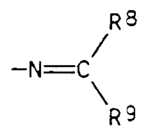

bedeuten, durch Umsetzung einer Säure Ic mit einem entsprechenden Alkohol IX in Gegenwart einer starken Mineralsäure wie z.B. Salzsäure oder Schwefelsäure bei einer Temperatur zwischen 0 und 100 ° C, vorzugsweise zwischen 20 und 50 ° C. In der Regel wird der Alkohol IX im Überschuß eingesetzt, es kann aber auch ein inertes Lösungsmittel verwendet werden.

Schema 3:

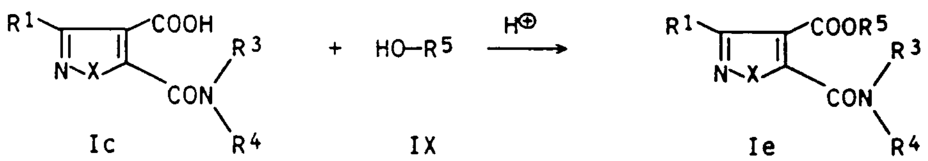

Ic IX Ie

4. Ein weiteres Verfahren zur Herstellung der Verbindungen der Formel Ie besteht in der Umsetzung einer Säure Ic mit einem Alkohol oder Thiol IX in Gegenwart eines wasserentziehenden Mittels (z.B. Dicyclohexylcarbodiimid (DCC)) bei einer Temperatur zwischen -20 und 50 ° C, vorzugsweise zwischen 0 und 30 ° C. In der Regel werden die Ausgangsmaterialien in etwa stöchiometrischer Menge zur Reaktion gebracht. Die Reaktion wird vorzugsweise in Gegenwart eines inerten Lösungsmittels, z.B. Tetrahydrofuran, Dichlormethan oder Toluol, durchgeführt.

Schema 4:

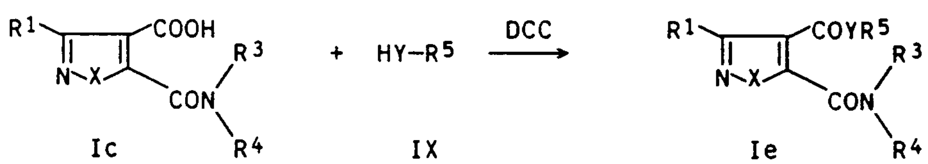

Ic IX Ie

(mit Y = O oder S).

5. Ein weiteres Verfahren zur Herstellung der Verbindungen Ic beruht auf der Umsetzung eines Carbonsäurealkylesters If mit einem Alkalimetallalkoxid X, wie Natrium- oder Kaliumalkoxid, mit einem entsprechenden Alkohol IX in an sich bekannter Art und Weise bei Temperaturen zwischen 20 ° C und der Siedetemperatur des gewählten Alkohols IX.

Schema 5:

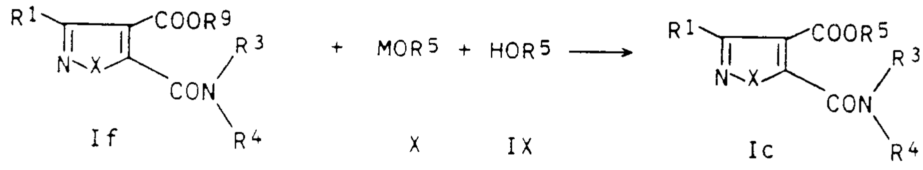

If X IX Ic

($R^9$ = Methyl oder Ethyl)

6. Verbindungen der Formel Ig, in der $R^2$ für $COOR^5$ steht, wobei $R^5$ ein salzbildendes Kation wie z.B. Alkalimetall, Erdalkalimetall, Ammonium oder substituiertes Ammonium bedeutet, werden durch Umsetzung einer substituierten Isoxazol- oder Isothiazol-4-carbonsäure Ic mit einem Äquivalent des salzbildenden Kations erhalten. Handelt es sich dabei um ein anorganisches Kation wie z.B. Natrium, Kalium oder Calcium, löst bzw. suspendiert man zweckmäßigerweise die Säure Ic in Wasser oder einem niederen Alkohol oder einer Mischung derselben und gibt ein Äquivalent des salzbildenden Kations zu. Das salzbildende Kation kann z.B. in Form seines Hydroxids, Carbonats oder Bicarbonats, vorzugsweise in Form seines Hydroxids, eingesetzt werden. Die Reaktion ist im allgemeinen nach wenigen Minuten beendet und kann wie üblich z.B. durch Ausfällen und Absaugen oder durch Einengen der Lösung aufgearbeitet werden. Zur Herstellung von Verbindungen Ig, bei denen $B^{\oplus}$ für Ammonium oder organisches Ammonium steht, löst bzw. suspendiert man die Säure Ic in einem organischen Lösungsmittel, wie z.B. Diethylether, Tetrahydrofuran oder Dioxan, und behandelt die Mischung mit einem Äquivalent Ammoniak, einem Amin oder einem Tetraalkylammoniumhydroxid.

Unter den Aminen, welche eingesetzt werden können, sollen die folgenden erwähnt werden: Methylamin, Ethylamin, n-Propylamin, Isopropylamin, n-Butylamin, Isobutylamin, sek.-Butylamin, n-Amylamin, Isoamylamin, Hexylamin, Heptylamin, Octylamin, Nonylamin, Decylamin, Undecylamin, Dodecylamin, Tridecylamin, Tetradecylamin, Pentadecylamin, Hexadecylamin, Heptadecylamin, Octadecylamin, Methylethylamin, Methylisopropylamin, Methylhexylamin, Methylnonylamin, Methylpentadecylamin, Methyloctadecylamin, Ethylbutylamin, Ethylheptylamin, Ethyloctylamin, Hexylheptylamin, Hexyloctylamin, Dimethylamin, Diethylamin, Di-n-propylamin, Diisopropylamin, Di-n-amylamin, Diisoamylamin, Dihexylamin, Diheptylamin, Dioctylamin, Trimethylamin, Triethylamin, Tri-n-propylamin, Triisopropylamin, Tri-n-butylamin, Triisobutylamin, Tri-sek.-butylamin, Tri-n-amylamin, Ethanolamin, n-Propanolamin, Isopropanolamin, Diethanolamin, N,N-Diethylethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Talgamin, Cyclopentylamin, Cyclohexylamin, Dicyclohexylamin, Piperidin, Morpholin und Pyrrolidin.

Bei den Tetraalkylammoniumhydroxiden können z.B. Tetramethyl-, Tetraethyl-oder Trimethylbenzylammoniumhydroxid eingesetzt werden. In der Regel fällt das Ammoniumsalz oder organische Ammoniumsalz aus der Lösung aus und kann nach üblichen Methoden isoliert werden. Alternativ kann das Salz der Formel Ig auch durch Einengen des Lösungsmittels erhalten werden.

Schema 6:

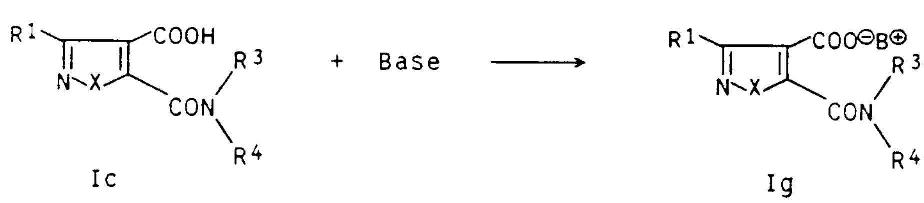


Ic Ig

7. Ein anderes Verfahren führt zu Verbindungen Ih, in der $R^2$ $CONR^6R^7$ bedeutet. Es besteht in der Umsetzung eines Esters Ib mit einem primären oder sekundären Amin XI. Das Verfahren wird so durchgeführt, daß man einen Ester Ib mit der 1- bis 50-fach molaren Menge Amin XI, gegebenenfalls in einem organischen Lösungsmittel, bei Temperaturen zwischen Raumtemperatur und der Siedetemperatur des Amins oder des organischen Lösungsmittels umsetzt. Als Ester Ib sind Niederalkylester, besonders die Methyl- und Ethylester bevorzugt. Die Reaktionsprodukte Ih können auf übliche Art und

Weise, wie z.B. durch Absaugen oder Einengen der Lösung isoliert werden und gegebenenfalls durch Umkristallisation oder Chromatographie weiter gereinigt werden.

Schema 7:

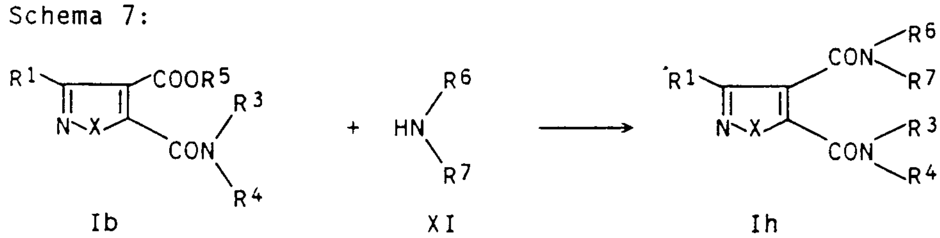

Ib XI Ih

8. Ein weiteres Verfahren zur Synthese der Verbindungen Ib besteht in der Umsetzung eines Isoxazol- oder Isothiazol-4,5-dicarbonsäuredialkylesters II mit einem Amin V.

Als Dialkylester II kommen insbesondere Niedrigalkylester, vorzugsweise Dimethylester oder Diethylester, in Betracht. Die Reaktion wird so durchgeführt, daß man einen Dicarbonsäuredialkylester II bei Temperaturen zwischen 0 und 100°C, vorzugsweise zwischen 50 und 80°C in einem organischen Lösungsmittel wie z.B. einem Alkohol wie etwa Methanol oder Ethanol mit ungefähr einem Äquivalent eines primären oder sekundären Amins V behandelt. Nach erfolgter Umsetzung wird abgekühlt und abgesaugt oder eingeengt. Das erhaltene Produkt der Formel Ib kann mit üblichen Standardmethoden, wie Umkristallisation oder Chromatographie, weiter gereinigt werden.

Schema 8:

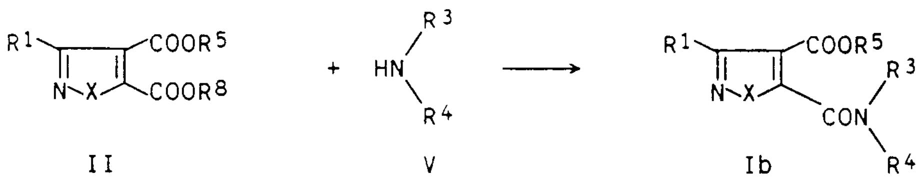

II V Ib

9. Verbindungen der Formel Ii können durch Umsetzung eines substituierten Isothiazol-4,5-dicarbonsäureanhydrids XII mit einem Amin V erhalten werden. Die Reaktion wird zweckmäßigerweise so durchgeführt, daß man das Anhydrid XII in einem inerten Lösungsmittel, wie einem Ether oder halogenierten Kohlenwasserstoff, vorlegt und etwa molare Mengen eines Amins V, gegebenenfalls ebenfalls gelöst in einem inerten Lösungsmittel, zutropft. Nach beendeter Reaktion wird das Reaktionsprodukt abgesaugt oder durch Einengen des verwendeten Lösungsmittels isoliert. In manchen Fällen können bei diesem Verfahren die isomeren Amide XIII entstehen, wobei im allgemeinen die Amide Ii die bevorzugten sind.

Schema 9:

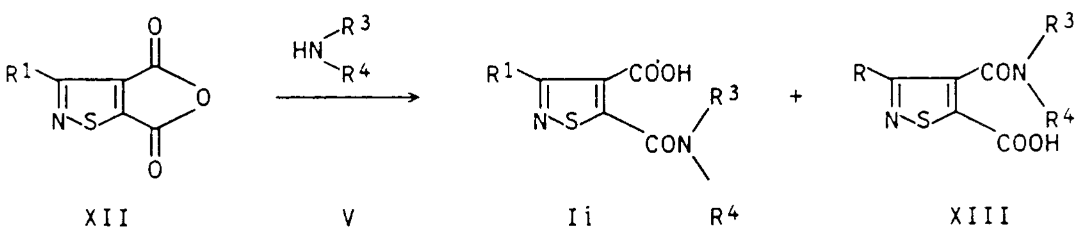

XII V Ii XIII

Die für dieses Verfahren als Ausgangsmaterial benötigten Isothiazol-4,5-dicarbonsäureanhydride XII sind literaturbekannt (J. Chem. Soc. 1959, 3061) oder können nach allgemein literaturbekannten Methoden synthetisiert werden.

10. Ein weiteres Verfahren zur Herstellung von Verbindungen der Formel Ik besteht in der Umsetzung einer Säure Ic mit einem Alkohol bzw. Thiol XIV in Anwesenheit von 1-Methyl-2-halogenpyridiniumiodiden bei einer Temperatur zwischen 20 und 80°C, vorzugsweise zwischen 30 und 40°C. Die Reaktion

wird in Gegenwart eines inerten Lösungsmittels, z.B. Dichlormethan oder Toluol, durchgeführt. Das Verfahren ist im Prinzip literaturbekannt (Chem. Lett., 1045 (1975); ibid., 13 (1976); ibid., 49/1976)).

Schema 10:

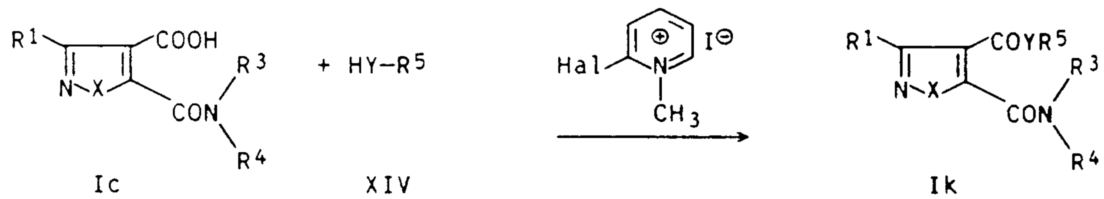

Ic XIV Ik

Die folgenden Beispiele erläutern die Herstellung der Vorprodukte für die Synthese der Verbindungen I bzw. Ia:

Beispiel a

Zu 10,0 g 3-Ethyl-isoxazol-5-carbonsäurechlorid in 150 ml Dichlormethan werden unter Eiskühlung 11,7 g Anilin zugetropft. Man rührt über Nacht bei Raumtemperatur nach, versetzt mit Wasser und konz. Salzsäure und trennt die organische Phase ab. Nach Waschen der organischen Phase mit Natriumbicarbonatlösung und Einengen erhält man 11,8 g 3-Ethyl-isoxazol-5-carbonsäureanilid als farblose Kristalle vom Fp. 122-124 ° C.

Auf analoge Weise können beispielsweise die Isoxazol-5-carbonsäureamide VII synthetisiert werden:

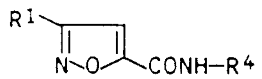

(VII)

| $R^1$ | $R^4$ | Fp [°C] |
|---|---|---|
| H | $i\text{-}C_3H_7$ | |
| H | $tert.\text{-}C_4H_9$ | 103-106 |
| H | $cyclo\text{-}C_3H_5$ | |
| H | $cyclo\text{-}C_6H_{11}$ | |
| H | $C_6H_5$ | |
| $CH_3$ | H | 167-171 |
| $CH_3$ | $i\text{-}C_3H_7$ | 92- 93 |
| $CH_3$ | $tert.\text{-}C_4H_9$ | 55- 60 |
| $CH_3$ | 1-Ethylcyclohexyl | öl |
| $CH_3$ | 4-Methyl-tetrahydro-pyran-4-yl | 63- 65 |
| $CH_3$ | $C_6H_5$ | 145-147 |
| $CH_3$ | $4\text{-}Cl\text{-}C_6H_4$ | 216-219 |
| $CH_3$ | $3\text{-}CF_3\text{-}C_6H_4$ | 146-148 |
| $C_2H_5$ | $i\text{-}C_3H_7$ | 85- 87 |
| $C_2H_5$ | $tert.\text{-}C_4H_9$ | 67- 70 |
| $C_2H_5$ | $4\text{-}Cl\text{-}C_6H_4$ | 170-173 |
| $C_2H_5$ | $3\text{-}CF_3\text{-}C_6H_4$ | 121-122 |
| $i\text{-}C_3H_7$ | H | |
| $i\text{-}C_3H_7$ | $CH_3$ | 69- 73 |
| $i\text{-}C_3H_7$ | $C_2H_5$ | 69- 72 |
| $i\text{-}C_3H_7$ | $n\text{-}C_3H_7$ | 79- 80 |
| $i\text{-}C_3H_7$ | $i\text{-}C_3H_7$ | 122-125 |
| $i\text{-}C_3H_7$ | $n\text{-}C_4H_9$ | 67- 68 |
| $i\text{-}C_3H_7$ | $sec.\text{-}C_4H_9$ | 133-135 |
| $i\text{-}C_3H_7$ | $i\text{-}C_4H_9$ | 85- 86 |
| $i\text{-}C_3H_7$ | $tert.\text{-}C_4H_9$ | 116-118 |
| $i\text{-}C_3H_7$ | $-C(CH_3)_2C_2H_5$ | 118-120 |
| $i\text{-}C_3H_7$ | $-C(CH_3)_2C_3H_7$ | 33- 34 |
| $i\text{-}C_3H_7$ | $-C(CH_3)_2CH_2C(CH_3)_3$ | 65- 66 |
| $i\text{-}C_3H_7$ | $-C(CH_3)_2CH_2SCH_3$ | 42 |
| $i\text{-}C_3H_7$ | $-CH_2CH_2SCH_3$ | |
| $i\text{-}C_3H_7$ | $-CH_2CH_2CH_2SCH_3$ | 34- 36 |
| $i\text{-}C_3H_7$ | $-CH_2CH_2OCH_3$ | öl |
| $i\text{-}C_3H_7$ | $-CH_2CH_2N(CH_3)_2$ | öl |
| $i\text{-}C_3H_7$ | $cyclo\text{-}C_3H_5$ | 88- 90 |
| $i\text{-}C_3H_7$ | $cyclo\text{-}C_6H_{11}$ | 152-154 |

| $R^1$ | $R^4$ | Fp [°C] |
|---|---|---|
| $i-C_3H_7$ | 1-Methyl-cyclohexyl | |
| $i-C_3H_7$ | 1-Ethylcyclohexyl | 50- 51 |
| $i-C_3H_7$ | 4-Methyl-tetrahydro-pyran-4-yl | 94- 96 |
| $i-C_3H_7$ | 4-Ethyl-tetrahydro-pyran-4-yl | 50- 51 |
| $i-C_3H_7$ | $-C(CH_3)_2$-cyclo$C_6H_{11}$ | 83 |
| $i-C_3H_7$ | $-CH_2CH=CH_2$ | 65- 66 |
| $i-C_3H_7$ | $-C(CH_3)_2CH=CH_2$ | 99-106 |
| $i-C_3H_7$ | $-C(CH_3)_2C\equiv CH$ | 85- 86 |
| $i-C_3H_7$ | $-CH_2-C_6H_5$ | 79- 81 |
| $i-C_3H_7$ | $-C(CH_3)_2C_6H_5$ | |
| $i-C_3H_7$ | $-CH_2-C(CH_3)_3$ | 87- 89 |
| $i-C_3H_7$ | $-C_6H_5$ | 106-108 |
| $i-C_3H_7$ | $4-Cl-C_6H_4$ | 176-178 |
| $i-C_3H_7$ | $3-CF_3-C_6H_4$ | 74- 78 |
| tert.-$C_4H_9$ | $i-C_3H_7$ | 120-122 |
| tert.-$C_4H_9$ | tert.-$C_4H_9$ | 129-133 |
| tert.-$C_4H_9$ | $C_6H_5$ | 121-122 |
| tert.-$C_4H_9$ | $4-Cl-C_6H_4$ | 158-161 |
| tert.-$C_4H_9$ | $3-CF_3-C_6H_4$ | 104-108 |
| cyclo-$C_6H_{11}$ | $i-C_3H_7$ | 139-140 |
| cyclo-$C_6H_{11}$ | tert.-$C_4H_9$ | 121-122 |
| cyclo-$C_6H_{11}$ | cyclo-$C_3H_5$ | 144-146 |
| cyclo-$C_6H_{11}$ | cyclo-$C_6H_{11}$ | |
| cyclo-$C_6H_{11}$ | $C_6H_5$ | 180-181 |
| Tetrahydropyran-3-yl | $i-C_3H_7$ | 102-104 |
| Tetrahydropyran-3-yl | tert.-$C_4H_9$ | 110-114 |
| Tetrahydropyran-3-yl | cyclo-$C_3H_5$ | 108-110 |
| Tetrahydropyran-3-yl | cyclo-$C_6H_{11}$ | |
| Tetrahydropyran-3-yl | $C_6H_5$ | 149-151 |
| $C_6H_5$ | $i-C_3H_7$ | |
| $C_6H_5$ | tert.-$C_4H_9$ | |
| $C_6H_5$ | cyclo-$C_3H_5$ | |
| $C_6H_5$ | cyclo-$C_6H_{11}$ | |
| $C_6H_5$ | $C_6H_5$ | |
| $4-Cl-C_6H_4$ | $i-C_3H_7$ | 166-171 |
| $4-Cl-C_6H_4$ | tert.-$C_4H_9$ | 128-132 |
| $4-Cl-C_6H_4$ | $C_6H_5$ | 229-232 |
| $4-Cl-C_6H_4$ | $4-Cl-C_6H_4$ | |
| $4-Cl-C_6H_4$ | $3-CF_3-C_6H_4$ | 163-165 |
| cyclo-$C_3H_5$ | $i-C_3H_7$ | 114-117 |
| cyclo-$C_3H_5$ | tert.-$C_4H_9$ | 106-107 |
| cyclo-$C_3H_5$ | $C_6H_5$ | 180-186 |

| $R^1$ | $R^4$ | Fp [°C]/$^1$H.NMR ($CDCl_3$) [ppm] |
|---|---|---|
| $i-C_3H_7$ | cyclo-$C_5H_9$ | 119-121 |
| $i-C_3H_7$ | Tetrahydrofur-3-yl | 75- 78 |
| $i-C_3H_7$ | Thiazol-2-yl | 165-168 |
| $i-C_3H_7$ | 5-Methyl-thiazol-2-yl | 149-153 |
| $i-C_3H_7$ | 5-Ethyl-thiazol-2-yl | 157-163 |
| $i-C_3H_7$ | 5-n-Propyl-thiazol-2-yl | 140-145 |
| $i-C_3H_7$ | $CH(CH_3)CH_2CN$ | 88- 92 |
| $i-C_3H_7$ | $-C(CH_3)_2-CH_2CN$ | 95- 97 |
| $i-C_3H_7$ | $OC_2H_5$ | 33- 35 |
| $i-C_3H_7$ | Pyrid-2-yl | 104-106 |
| $i-C_3H_7$ | Pyrid-3-yl | 150-152 |
| $i-C_3H_7$ | Pyrid-4-yl | 185-187 |
| $i-C_3H_7$ | Pyrimid-2-yl | 94- 99 |
| $n-C_3H_7$ | $i-C_3H_7$ | 108-110 |
| $n-C_3H_7$ | cyclo-$C_3H_5$ | 104-106 |
| $n-C_3H_7$ | tert.-$C_4H_9$ | 85- 86 |
| $n-C_3H_7$ | $C_6H_5$ | 118-119 |
| $n-C_3H_7$ | cyclo-$C_6H_{11}$ | 136-137 |
| $s-C_4H_9$ | $i-C_3H_7$ | 150-152 |
| $s-C_4H_9$ | cyclo-$C_3H_5$ | 107-111 |
| $s-C_4H_9$ | tert.-$C_4H_9$ | 138-142 |
| $s-C_4H_9$ | $C_6H_5$ | 99-101 |
| $i-C_3H_7$ | $N(CH_3)_2$ | 131-133 |
| $CH_3$ | $N(CH_3)_2$ | 111-113 |
| $CH_3$ | Morpholino | 190-192 |
| $CH_3$ | Piperidino | 158-161 |
| $CH_3$ | $CH_3$ | 146-148 |
| $i-C_3H_7$ | Piperidino | 133-135 |
| $i-C_3H_7$ | Morpholino | 178-179 |
| $CH_3$ | $C_2H_5$ | 97- 99 |
| neo-$C_5H_{11}$ | $CH_3$ | 128-130 |
| neo-$C_5H_{11}$ | $i-C_3H_7$ | 85- 88 |
| neo-$C_5H_{11}$ | cyclo-$C_3H_5$ | 109-112 |
| neo-$C_5H_{11}$ | tert.-$C_4H_9$ | 97- 99 |
| neo-$C_5H_{11}$ | $C_6H_5$ | 137-140 |
| $n-C_4H_9$ | $CH_3$ | 74- 76 |
| $n-C_4H_9$ | $i-C_3H_7$ | 97-100 |
| $n-C_4H_9$ | cyclo-$C_3H_5$ | 82- 86 |
| $n-C_4H_9$ | tert.-$C_4H_9$ | 60-64 |
| $n-C_4H_9$ | $C_6H_5$ | 118-120 |
| cyclo-$C_5H_9$ | tert.-$C_4H_9$ | 114-115 |
| cyclo-$C_5H_9$ | $CH_3$ | 88- 89 |

| $R^1$ | $R^4$ | Fp [°C]/$^1$H.NMR ($CDCl_3$) [ppm] |
|---|---|---|
| cyclo-$C_5H_9$ | cyclo-$C_3H_5$ | 108-109 |
| cyclo-$C_5H_9$ | $C_6H_5$ | 146-148 |
| n-$C_4H_9$ | $OCH_3$ | 62- 66 |
| $CH_3$-O-$CH_2$ | tert.-$C_4H_9$ | 50- 55 |
| $CH_3$-O-$CH_2$ | cyclo-$C_3H_5$ | 55- 60 |
| 2-Methoxyphenyl | tert.-$C_4H_9$ | 119-120 |
| 2-Methoxyphenyl | cyclo-$C_3H_5$ | 160-163 |
| i-$C_3H_7$ | $CH_2$-cyclo-$C_3H_5$ | 77- 80 |
| n-$C_4H_9$ | -$CH_2$-cyclo-$C_3H_5$ | 102-105 |
| $CH_3O$-CH($CH_3$)- | tert.-$C_4H_9$ | 76- 79 |
| cyclo-$C_3H_5$ | cyclo-$C_5H_9$ | 148-149 |
| 2,6-Difluorphenyl | tert.-$C_4H_9$ | 118-122 |
| 2,6-Difluorphenyl | cyclo-$C_3H_5$ | 128-132 |
| $CH_3$ | cyclo-$C_4H_7$ | 114-115 |
| i-$C_3H_7$ | cyclo-$C_4H_7$ | 84- 85 |
| $CH_3O$ | tert.-$C_4H_9$ | 65- 68 |

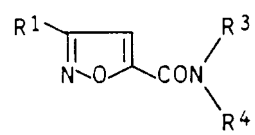

(VII)

| $R^1$ | $R^3$ | $R^4$ | Fp [°C] |
|---|---|---|---|
| i-$C_3H_7$ | $CH_3$ | $CH_3$ | |
| i-$C_3H_7$ | $C_2H_5$ | $C_2H_5$ | öl |

Beispiel b

Zu 18,9 g Kaliumhydroxid in 100 ml Wasser werden bei Raumtemperatur 65 g 3-Methyl-isoxazol-4,5-dicarbonsäurediethylester gelöst in 100 ml Ethanol zugetropft. Nach 16 Stunden wird auf 300 ml Wasser gegossen, mit Ether extrahiert und die wäßrige Phase mit konz. Salzsäure eingesäuert. Man extrahiert mit Dichlormethan und erhält nach dem Einengen 3-Methyl-4-ethoxycarbonyl-isoxazol-5-carbonsäure als farblose Kristalle vom Fp. 54-58 °C.

Beispiel c

Zu 85,5 g 3-Ethyl-isothiazol-4,5-dicarbonsäuredimethylester in 300 ml Methanol wird unter Eiskühlung eine Lösung aus 14,9 g NaOH in 120 ml Wasser und 150 ml Methanol zugetropft. Nach 2 Stunden wird eingeengt, zum Rückstand 1,5 l Wasser zugegeben, nachgerührt und mit Ether extrahiert. Die wäßrige Phase säuert man mit konz. Salzsäure an und schüttelt mit Dichlormethan aus. Einengen der organischen Phasen liefert 76,4 g 3-Ethyl-4-methoxycarbonyl-isothiazol-5-carbonsäure vom Fp. 43-45 °C.

Analog Beispielen b und c können beispielsweise die Isoxazol(Isothiazol)-5-carbonsäuren III synthetisiert werden:

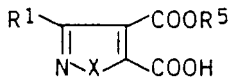

(III)

| $R^1$ | $R^5$ | X | Fp [°C] |
|---|---|---|---|
| H | $CH_3$ | O | |
| H | $C_2H_5$ | O | |
| H | $CH_3$ | S | |
| H | $C_2H_5$ | S | |
| $CH_3$ | $CH_3$ | O | |
| $CH_3$ | $CH_3$ | S | 75- 81 |
| $CH_3$ | $C_2H_5$ | S | |
| $C_2H_5$ | $CH_3$ | O | |
| $C_2H_5$ | $C_2H_5$ | O | |
| $C_2H_5$ | $C_2H_5$ | S | |
| n-$C_3H_7$ | $CH_3$ | O | |
| n-$C_3H_7$ | $C_2H_5$ | O | |
| n-$C_3H_7$ | $CH_3$ | S | |
| n-$C_3H_7$ | $C_2H_5$ | S | |
| i-$C_3H_7$ | $CH_3$ | O | |
| i-$C_3H_7$ | $C_2H_5$ | O | |
| i-$C_3H_7$ | $CH_3$ | S | Öl |
| i-$C_3H_7$ | $C_2H_5$ | S | |
| s-$C_4H_9$ | $CH_3$ | O | |
| s-$C_4H_9$ | $C_2H_5$ | O | |
| s-$C_4H_9$ | $CH_3$ | S | |
| s-$C_4H_9$ | $C_2H_5$ | S | |
| tert.-$C_4H_9$ | $CH_3$ | O | |
| tert.-$C_4H_9$ | $C_2H_5$ | O | |
| tert.-$C_4H_9$ | $CH_3$ | S | |
| tert.-$C_4H_9$ | $C_2H_5$ | S | |

| $R^1$ | $R^5$ | X | Fp [°C] |
|---|---|---|---|
| cyclo-$C_3H_5$ | $CH_3$ | O | |
| cyclo-$C_3H_5$ | $C_2H_5$ | O | |
| cyclo-$C_3H_5$ | $CH_3$ | S | |
| cyclo-$C_3H_5$ | $C_2H_5$ | S | |
| cyclo-$C_6H_{11}$ | $CH_3$ | O | |
| cyclo-$C_6H_{11}$ | $C_2H_5$ | O | |
| cyclo-$C_6H_{11}$ | $CH_3$ | S | |
| cyclo-$C_6H_{11}$ | $C_2H_5$ | S | |
| Tetrahydropyran-3-yl | $CH_3$ | O | |
| Tetrahydropyran-3-yl | $C_2H_5$ | O | |
| Tetrahydropyran-3-yl | $CH_3$ | S | |
| Tetrahydropyran-3-yl | $C_2H_5$ | S | |
| $C_6H_5$ | $CH_3$ | O | |
| $C_6H_5$ | $C_2H_5$ | O | |
| $C_6H_5$ | $CH_3$ | S | 139-141 |
| $C_6H_5$ | $C_2H_5$ | S | |

Herstellungsbeispiele für Verbindungen I bzw. Ia

Beispiel 1

Zu 10 g 3-Methyl-isoxazol-4,5-dicarbonsäurediethylester gelöst in 100 ml Methanol werden 2,9 g Isopropylamin zugetropft und anschließend zum Rückfluß erhitzt. Nach 7 Stunden wird eingeengt und das verbleibende Öl an Kieselgel chromatographiert (Toluol : Aceton = 9 : 1). Man erhält 5-iso-Propylaminocarbonyl-3-methyl-4-isoxazol-carbonsäuremethylester als farblose Kristalle vom Fp. 64-66°C (Verbindung Nr. 1005)

Beispiel 2

2,6 g Ester aus Beispiel 1 und 0,8 g Kaliumhydroxid werden in 20 ml Wasser und 20 ml Ethanol 16 Stunden bei Raumtemperatur gerührt. Anschließend wird mit Wasser verdünnt, mit konz. Salzsäure angesäuert und mit Dichlormethan ausgeschüttelt. Nach dem Einengen erhält man 1,8 g 5-iso-Propylaminocarbonyl-3-methyl-4-isoxazolcarbonsäure, farblose Kristalle vom Fp. 86-92°C (Verbindung Nr. 1004)

Beispiel 3

Zu 9,0 g Anilid aus Beispiel a, gelöst in 200 ml absolutem Tetrahydrofuran, werden bei -70°C 70 ml n-Butyllithium (1,6 molar in n-Hexan) zugetropft. Man rührt eine halbe Stunde nach und gießt das Reaktionsgemisch auf 500 g festes Kohlendioxid. Nach Stehen über Nacht wird eingeengt und der Rückstand zwischen $H_2O$, Natronlauge und Essigsäureethylester verteilt. Durch Einengen der Essigsäureethylesterphase lassen sich 2,0 g Ausgangsmaterial zurückgewinnen. Durch Ansäuern der wäßrigen Phase mit konz. Salzsäure und Absaugen erhält man 8,30 g 5-Anilinocarbonyl-3-ethyl-4-isoxazolcarbonsäure, farblose Kristalle vom Fp. 150-152°C (Verbindung Nr. 1015)

Beispiel 4

5,0 g 5-tert.-Butylaminocarbonyl-3-methyl-4-isoxazolcarbonsäure werden in 200 ml Methanol gelöst und 5 ml konz. $H_2SO_4$ zugegeben. Nach 2 Tagen wird eingeengt, der Rückstand zwischen Essigsäureethylester und Wasser verteilt und die organische Phase eingeengt. Man erhält 4,0 g 5-tert.-Butylaminocarbonyl-3-methyl-4-isoxazolcarbonsäuremethylester als farbloses Öl (Verbindung Nr. 1007).

H-NMR ($CDCl_3$): $\delta$ = 1,48 (s;9H), 2,50(s;3H), 3,99(s;1H), 9,42 (bs;1H,NH).

Beispiel 5

a) 3-Ethyl-4-methoxycarbonyl-isothiazol-5-carbonsäurechlorid

73 g Carbonsäure aus Beispiel c und 80 g Thionylchlorid werden in 200 ml Toluol in Gegenwart von etwas Dimethylformamid bis zur Beendigung der Gasentwicklung zum Rückfluß erhitzt. Das nach dem Einengen in quantitativer Ausbeute verbleibende rohe Carbonsäurechlorid wird direkt weiter umgesetzt.

b) 5-iso-Propylaminocarbonyl-3-ethyl-isothiazol-4-carbonsäuremethylester

Zu 16,5 g rohem Säurechlorid aus a) in 200 ml Dichlormethan werden unter Eiskühlung 8,4 g iso-Propylamin langsam zugetropft. Man rührt über Nacht nach, hydrolysiert mit 150 ml Wasser und trennt die organische Phase ab. Nach Waschen der organischen Phase mit Bicarbonatlösung, verdünnter Salzsäure und Wasser wird eingeengt. Man erhält 16,2 g 5-iso-Propylaminocarbonyl-3-ethyl-isothiazol-4-carbonsäuremethylester vom Fp. 55-56 °C (Verbindung Nr. 3007).

Beispiel 6

Zu 11 g Ester aus Beispiel 5 in 50 ml Ethanol werden 2,8 g KOH in 30 ml Wasser zugegeben und über Nacht bei Raumtemperatur nachgerührt. Man verdünnt mit 150 ml Wasser, extrahiert mit Ether und säuert die wäßrige Phase mit konz. Salzsäure an. Ausschütteln mit Dichlormethan und Einengen liefert 10 g 5-iso-Propylaminocarbonyl-3-ethyl-isothiazol-4-carbonsäure vom Fp. 138-140 °C (Verbindung Nr. 3006).

Beispiel 7

4,0 g 5-tert.-Butylaminocarbonyl-3-methyl-4-isoxazolcarbonsäuremethylester und 50 ml konz. Ammoniak werden 3 Stunden zum Rückfluß erhitzt. Nach dem Abkühlen wird mit Wasser verdünnt, mit Dichlormethan extrahiert und eingeengt. Man erhält 5-tert.-Butylaminocarbonyl-3-methyl-4-isoxazolcarbonamid als farblose Kristalle vom Fp. 155-158 °C (Verbindung Nr. 1).

Beispiel 8

Zu 8 g 3-Methyl-isoxazol-5-carbonsäure-tert.-butylamid in 150 ml Tetrahydrofuran werden bei -78 °C 56 ml Butyllithium (1,6 molar in n-Hexan) zugetropft. Man rührt 1 Stunde nach und tropft dann langsam 22 ml Dimethylformamid zu. Man läßt über Nacht auf Raumtemperatur kommen, hydrolysiert mit Wasser, neutralisiert mit konzentrierter Salzsäure und extrahiert mit Ether. Das nach dem Einengen verbleibende Öl wird an Kieselgel mit Cyclohexan/Essigester chromatographiert. Man erhält als erste Fraktion 4-Formyl-3-methylisoxazol-5-carbonsäure-tert.-butylamid, hellgelbe Kristalle vom Fp. 36-38 °C (Verbindung Nr. 2).

Beispiel 9

a) 3-Methyl-4-ethoxycarbonyl-isoxazol-5-carbonsäurechlorid

13,3 g Carbonsäure aus Beispiel b und 20 ml Thionylchlorid werden in Gegenwart von etwas Dimethylformamid bis zur Beendigung der Gasentwicklung zum Rückfluß erhitzt. Das nach dem Einengen in quantitativer Ausbeute verbleibende rohe Carbonsäurechlorid wird direkt weiter umgesetzt.

b) Zu 5 g rohem Säurechlorid aus a) in 100 g Dichlormethan werden unter Eiskühlung 3,4 g Diethylamin langsam zugetropft. Man rührt über Nacht nach, hydrolysiert mit Wasser und trennt die organische Phase ab. Nach Waschen der organischen Phase mit Bicarbonat wird eingeengt. Man erhält 5,0 g 5-Diethylaminocarbonyl-3-methyl-isoxazol-4-carbonsäureethylester als braunes Öl (Verbindung Nr. 3).

H-NMR ($CDCl_3$): $\delta$ = 1.15(t;3H), 1.27 und 1.31(2t;6H), 2.50(s;3H), 3.17(q;2H), 3.58(q;2H), 4.28-(q;2H).

Beispiel 10

5,0 g Ester aus Beispiel 9 und 1,4 g Kaliumhydroxid werden in 10 ml Wasser und 20 ml Ethanol bei Raumtemperatur gerührt. Nach beendeter Reaktion verdünnt man mit Wasser und extrahiert mit Dichlormethan. Anschließend wird die wäßrige Phase mit Salzsäure angesäuert, mit Dichlormethan extrahiert und eingeengt. Man erhält 3,0 g 5-Diethylaminocarbonyl-3-methyl-isoxazol-4-carbonsäure als helles Öl (Verbindung Nr. 4).

H-NMR ($CDCl_3$): $\delta$ = 1.28 und 1.32(2t;6H), 2.60(s;3H), 3.59 und 3.62(2q;4H), 10.50(bs;1H,COOH).

Beispiel 11

Zu einer gerührten Suspension von 4,1 g 1-Methyl-2-chlor-pyridiniumiodid in 40 ml Dichlormethan tropft man bei Raumtemperatur ein Gemisch aus 1,2 g 2-Methyl-propan-2-thiol, 3,0 g 5-tert.-Butylaminocarbonyl-3-methyl-isoxazol4-carbonsäure und 5,9 g Tri-n-butylamin in 20 ml Dichlormethan. Man erhitzt 3 h unter Rückfluß, zieht das Solvents im Vakuum ab und reinigt das Rohprodukt durch Säulenchromatographie an Kieselgel. Man erhält 2,1 g 5-tert.-Butylaminocarbonyl-3-methyl-isoxazol-4-carbonsäure-tert.-butylthioester als gelbes Öl (Verbindung Nr. 2003).

Analog den Beispielen 1 bis 10 können beispielsweise die in den folgenden Tabellen 1 bis 3 angeführten Verbindungen herstellt werden.

Tabelle 1

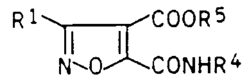

| Nr. | $R^1$ | $R^5$ | $R^4$ | Fp [°C]/$^1$H-NMR ($CDCl_3$) [ppm] |
|---|---|---|---|---|
| 1001 | H | H | i-$C_3H_7$ | |
| 1002 | H | H | tert.-$C_4H_9$ | öl 1.55(s;9H), 7.50 (bs; 1H, NH), 8.78(s;1H) |
| 1003 | $CH_3$ | H | H | 266-268 |
| 1004 | $CH_3$ | H | i-$C_3H_7$ | 86- 92 |
| 1005 | $CH_3$ | $CH_3$ | i-$C_3H_7$ | 64- 66 |
| 1006 | $CH_3$ | H | tert.-$C_4H_9$ | 92- 94 |
| 1007 | $CH_3$ | $CH_3$ | tert.-$C_4H_9$ | |
| 1008 | $CH_3$ | H | 1-Ethylcyclohexyl | 119-121 |
| 1009 | $CH_3$ | H | 4-Methyltetrahydro-pyran-4-yl | 80- 87 |
| 1010 | $CH_3$ | H | $C_6H_5$ | 204-210 |
| 1011 | $CH_3$ | H | 4-Cl-$C_6H_4$ | 233-237 |
| 1012 | $CH_3$ | H | 3-$CF_3$-$C_6H_4$ | 188-191 |
| 1013 | $C_2H_5$ | H | i-$C_3H_7$ | 63- 66 |
| 1014 | $C_2H_5$ | H | tert.-$C_4H_9$ | 53- 58 |
| 1015 | $C_2H_5$ | H | $C_6H_5$ | 150-152 |
| 1016 | $C_2H_5$ | H | 4-Cl-$C_6H_4$ | 193-196 |
| 1017 | $C_2H_5$ | H | 3-$CF_3$-$C_6H_4$ | 160-162 |
| 1018 | i-$C_3H_7$ | H | H | |
| 1019 | i-$C_3H_7$ | H | $CH_3$ | 147-148 |
| 1020 | i-$C_3H_7$ | H | $C_2H_5$ | 100-101 |
| 1021 | i-$C_3H_7$ | H | n-$C_3H_7$ | 85- 86 |
| 1022 | i-$C_3H_7$ | H | i-$C_3H_7$ | 98- 99 |
| 1023 | i-$C_3H_7$ | H | n-$C_4H_9$ | 96- 97 |
| 1024 | i-$C_3H_7$ | H | i-$C_4H_9$ | 112-114 |
| 1025 | i-$C_3H_7$ | H | sec-$C_4H_9$ | öl 1.00(t;3H), 1.32(d;3H), 1.37(d;6H), 1.64(quint; 2H), 3.78(sept; 1H), 4.15 (m;1H), 7.00 (bs;1H,NH) |

| Nr. | $R^1$ | $R^5$ | $R^4$ | Fp [°C]/$^1$H-NMR ($CDCl_3$) [ppm] |
|---|---|---|---|---|
| 1026 | i-$C_3H_7$ | H | tert.-$C_4H_9$ | 94- 98 |
| 1027 | i-$C_3H_7$ | H | $C(CH_3)_2C_2H_5$ | 44- 46 |
| 1028 | i-$C_3H_7$ | H | $C(CH_3)_2C_3H_7$ | 52- 53 |
| 1029 | i-$C_3H_7$ | H | $C(CH_3)_2CH_2C(CH_3)_3$ | 89-91 |
| 1030 | i-$C_3H_7$ | H | $C(CH_3)_2CH_2SCH_3$ | Öl 1.35(d;3H), 1.56(s;6H), 2.18(s;3H), 2.99(s;2H), 3.77(sept;1H), 7.30(bs;1H,NH) |
| 1031 | i-$C_3H_7$ | H | $CH_2CH_2SCH_3$ | |
| 1032 | i-$C_3H_7$ | H | $CH_2CH_2CH_2SCH_3$ | 116-118 |
| 1033 | i-$C_3H_7$ | H | $CH_2CH_2OCH_3$ | 69- 71 |
| 1034 | i-$C_3H_7$ | H | $CH_2CH_2N(CH_3)_2$ | |
| 1035 | i-$C_3H_7$ | H | cyclo-$C_3H_5$ | 74- 76 |
| 1036 | i-$C_3H_7$ | H | cyclo-$C_6H_{11}$ | 112-114 |
| 1037 | i-$C_3H_7$ | H | 1-Ethylcyclohexyl | 89- 90 |
| 1038 | i-$C_3H_7$ | H | 4-Methyltetrahydro-pyran-4-yl | 129-130 |
| 1039 | i-$C_3H_7$ | H | 1-$C(CH_3)_2$-cyclo$C_6H_{11}$ | 144-146 |
| 1040 | i-$C_3H_7$ | H | $CH_2CH=CH_2$ | 92- 94 |
| 1041 | i-$C_3H_7$ | H | $C(CH_3)_2CH=CH_2$ | Öl;1.35(d;6H), 1.62(s;6H), 3.78(sept;1H), 5.20(d;1H), 5.25(d;1H), 6.10(dd;1H), 7.10(bs;1H,NH) |
| 1042 | i-$C_3H_7$ | H | $CH_2C_6H_5$ | Öl 1.30(d;6H), 3.74(sept;1H), 4.70(d;2H), 7.35(bs;5H), 7.85(bt;1H,NH) |
| 1043 | i-$C_3H_7$ | H | $C(CH_3)_2C_6H_5$ | |
| 1044 | i-$C_3H_7$ | H | $CH_2C(CH_3)_3$ | 76 |
| 1045 | i-$C_3H_7$ | H | $C_6H_5$ | 138-140 |
| 1046 | i-$C_3H_7$ | H | 4-Cl-$C_6H_4$ | 170-173 |
| 1047 | i-$C_3H_7$ | H | 3-$CF_3$-$C_6H_4$ | 127 |
| 1048 | tert.-$C_4H_9$ | H | i-$C_3H_7$ | 84- 85 |
| 1049 | tert.-$C_4H_9$ | H | tert.-$C_4H_9$ | 129-133 |

| Nr. | $R^1$ | $R^5$ | $R^4$ | Fp [°C]/$^1H$-NMR ($CDCl_3$) [ppm] |
|---|---|---|---|---|
| 1050 | tert.-$C_4H_9$ | H | $C_6H_5$ | 132-137 |
| 1051 | tert.-$C_4H_9$ | H | 4-Cl-$C_6H_4$ | 188-191 |
| 1052 | tert.-$C_4H_9$ | H | 3-$CF_3$-$C_6H_4$ | 160-162 |
| 1053 | cyclo-$C_6H_{11}$ | H | i-$C_3H_7$ | 116-118 |
| 1054 | cyclo-$C_6H_{11}$ | H | tert.-$C_4H_9$ | 158-159 |
| 1055 | cyclo-$C_6H_{11}$ | H | cyclo-$C_3H_5$ | 142-143 |
| 1056 | cyclo-$C_6H_{11}$ | H | cyclo-$C_6H_{11}$ | |
| 1057 | cyclo-$C_6H_{11}$ | H | $C_6H_5$ | 198-199 |
| 1058 | 4-Cl-$C_6H_4$ | H | i-$C_3H_7$ | 165-168 |
| 1059 | 4-Cl-$C_6H_4$ | H | tert.-$C_4H_9$ | 165-168 |
| 1060 | 4-Cl-$C_6H_4$ | H | $C_6H_5$ | 220 |
| 1061 | 4-Cl-$C_6H_4$ | H | 4-Cl-$C_6H_4$ | |
| 1062 | 4-Cl-$C_6H_4$ | H | 3-$CF_3$-$C_6H_4$ | 209-211 |
| 1063 | i-$C_3H_7$ | Succinimido | cyclo-$C_3H_5$ | 108-109 |
| 1064 | $CH_3$ | H | $C(CH_3)_2C{\equiv}CH$ | 80- 87 |
| 1065 | $CH_3$ | $C_2H_5$ | $C(CH_3)_2C{\equiv}CH$ | 82- 86 |
| 1066 | $CH_3$ | $Na^{\oplus}$ | tert.-$C_4H_9$ | 220 |
| 1067 | $CH_3$ | $K^{\oplus}$ | tert.-$C_4H_9$ | 288 |
| 1068 | $CH_3$ | $H_3N^{\oplus}CH(CH_3)_2$ | tert.-$C_4H_9$ | 184-187 |
| 1069 | $CH_3$ | $H_3N^{\oplus}$-$CH_2CH_2OH$ | tert.-$C_4H_9$ | 124-126 |
| 1070 | $C_2H_5$ | $Na^{\oplus}$ | tert.-$C_4H_9$ | 150 |
| 1071 | $C_2H_5$ | $K^{\oplus}$ | tert.-$C_4H_9$ | 220 |
| 1072 | $C_2H_5$ | $H_3N^{\oplus}$-$CH(CH_3)_2$ | tert.-$C_4H_9$ | 170-172 |
| 1073 | $C_2H_5$ | $H_3N^{\oplus}$-$CH_2CH_2OH$ | tert.-$C_4H_9$ | 105-108 |
| 1074 | $C_2H_5$ | Succinimido | tert.-$C_4H_9$ | 163-165 |
| 1075 | $C_2H_5$ | $-N{=}C(CH_3)_2$ | tert.-$C_4H_9$ | 68- 70 |
| 1076 | $C_2H_5$ | $CH_2C{\equiv}CH$ | tert.-$C_4H_9$ | Öl 1.35(t;3H), 1.48(s;9H), 2.63(t;1H), 2.96; 4.98 (d;2H), 8.95(bs; 1H,NH) |
| 1077 | $C_2H_5$ | $CH_2CH_2OC_2H_5$ | tert.-$C_4H_9$ | 74- 76 |
| 1078 | cyclo-$C_3H_5$ | H | i-$C_3H_7$ | 78- 80 |
| 1079 | cyclo-$C_3H_5$ | H | tert.-$C_4H_9$ | 87- 88 |
| 1080 | cyclo-$C_3H_5$ | H | $C_6H_5$ | 162-163 |
| 1081 | cyclo-$C_6H_{11}$ | Succinimido | i-$C_3H_7$ | 126-127 |
| 1082 | cyclo-$C_6H_{11}$ | Succinimido | tert.-$C_4H9$ | 172-174 |
| 1083 | cyclo-$C_6H_{11}$ | Succinimido | $C_6H_5$ | 176-177 |
| 1084 | Tetrahydro-pyran-3-yl | H | i-$C_3H_7$ | 157-160 |

| Nr. | $R^1$ | $R^5$ | $R^4$ | Fp [°C]/$^1H$-NMR ($CDCl_3$) [ppm] |
|---|---|---|---|---|
| 1085 | Tetrahydro-pyran-3-yl | H | tert.-$C_4H_9$ | 91- 95 |
| 1086 | Tetrahydro-pyran-3-yl | H | cyclo-$C_3H_5$ | 158-160 |
| 1087 | Tetrahydro-pyran-3-yl | H | $C_6H_5$ | 152-157 |
| 1088 | $CH_3$ | Pyrid-3-yl-methyl | tert.-$C_4H_9$ | |
| 1089 | $CH_3$ | Thien-2-yl-methyl | tert.-$C_4H_9$ | |
| 1090 | $CH_3$ | $-CH_2-CH_2-N(CH_3)_2$ | tert.-$C_4H_9$ | |
| 1091 | $CH_3$ | $-CH_2-CH_2\overset{\oplus}{N}(CH_3)_3\overset{\ominus}{I}$ | tert.-$C_4H_9$ | |
| 1092 | $CH_3$ | $-CH_2-CF_3$ | tert.-$C_4H_9$ | |
| 1093 | $CH_3$ | $-CH_2-C(CH_3)=CH_2$ | tert.-$C_4H_9$ | |
| 1094 | $CH_3$ | $-CH_2C(Cl)=CH_2$ | tert.-$C_4H_9$ | |
| 1095 | $CH_3$ | $-CH_2-C\equiv C-CH_2OH$ | tert.-$C_4H_9$ | |
| 1096 | $CH_3$ | 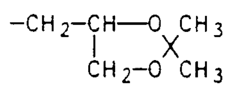 | tert.-$C_4H_9$ | |
| 1097 | $CH_3$ | 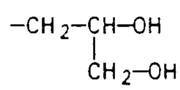 | tert.-$C_4H_9$ | |
| 1098 | $CH_3$ | 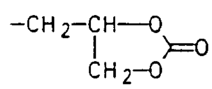 | tert.-$C_4H_9$ | |
| 1099 | $CH_3$ | Phenethyl | tert.-$C_4H_9$ | |
| 1100 | $CH_3$ | $-CH(C_6H_5)COOCH_3$ | tert.-$C_4H_9$ | |
| 1101 | $CH_3$ | cyclo-$C_6H_{11}$ | tert.-$C_4H_9$ | |
| 1102 | $CH_3$ | $-CH_2-OCH_2-C_6H_5$ | tert.-$C_4H_9$ | |
| 1103 | $CH_3$ | Tetrahydro-pyran-2-yl | tert.-$C_4H_9$ | |
| 1104 | $CH_3$ | Tetrahydro-fur-2-yl | tert.-$C_4H_9$ | |
| 1105 | $CH_3$ | (4-Brom-benzoyl)-methyl | tert.-$C_4H_9$ | |
| 1106 | $CH_3$ | (4-Methoxybenzoyl) methyl | tert.-$C_4H_9$ | |
| 1107 | $CH_3$ | $-CH(COOCH_3)_2$ | tert.-$C_4H_9$ | |

| Nr. | $R^1$ | $R^5$ | $R^4$ | Fp [°C]/$^1$H-NMR ($CDCl_3$) [ppm] |
|---|---|---|---|---|
| 1108 | $CH_3$ | Phthalimidomethyl | tert.-$C_4H_9$ | |
| 1109 | $CH_3$ | $-CH_2-CH_2-Si(CH_3)_3$ | tert.-$C_4H_9$ | |
| 1110 | $CH_3$ | $-CH_2-CH_2-O-N=C(CH_3)_2$ | tert.-$C_4H_9$ | |
| 1111 | $CH_3$ | $-CH_2-PO(OC_2H_5)_2$ | tert.-$C_4H_9$ | |
| 1112 | $CH_3$ | Fur-2-yl-methyl | tert.-$C_4H_9$ | |
| 1113 | $CH_3$ | Tetrahydrofur-2-yl-methyl | tert.-$C_4H_9$ | |
| 1114 | $CH_3$ | Pyrid-2-ylmethyl | tert.-$C_4H_9$ | |
| 1115 | $CH_3$ | Pyrid-4-ylmethyl | tert.-$C_4H_9$ | |
| 1116 | $CH_3$ | Piperidino | tert.-$C_4H_9$ | |
| 1117 | $CH_3$ | Phthalimido | tert.-$C_4H_9$ | |
| 1118 | $CH_3$ | 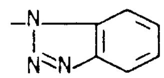 | tert.-$C_4H_9$ | |
| 1119 | $CH_3$ | $-N=CH-C_6H_5$ | tert.-$C_4H_9$ | |
| 1120 | $CH_3$ | 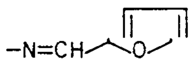 | tert.-$C_4H_9$ | |
| 1121 | $CH_3$ | $-CH(CH_3)CH(OCH_3)_2$ | tert.-$C_4H_9$ | |
| 1122 | $CH_3$ | $-CH_2-CON(C_2H_5)_2$ | tert.-$C_4H_9$ | |
| 1123 | $CH_3$ | $N(C_2H_5)_2$ | tert.-$C_4H_9$ | |
| 1124 | $C_2H_5$ | cyclo-$C_6H_{11}$ | tert.-$C_4H_9$ | |
| 1125 | $C_2H_5$ | $-CH_2-OCH_2-C_6H_5$ | tert.-$C_4H_9$ | |
| 1126 | $C_2H_5$ | Tetrahydro-pyran-2-yl | tert.-$C_4H_9$ | |
| 1127 | $C_2H_5$ | Tetrahydro-fur-2-yl | tert.-$C_4H_9$ | |
| 1128 | $C_2H_5$ | (4-Brom-benzoyl)-methyl | tert.-$C_4H_9$ | |
| 1129 | $C_2H_5$ | (4-Methoxybenzoyl) methyl | tert.-$C_4H_9$ | |
| 1130 | $C_2H_5$ | $-CH(COOCH_3)_2$ | tert.-$C_4H_9$ | |

| Nr. | $R^1$ | $R^5$ | $R^4$ | Fp [°C]/$^1$H-NMR ($CDCl_3$) [ppm] |
|---|---|---|---|---|
| 1131 | $C_2H_5$ | Phthalimidomethyl | tert.-$C_4H_9$ | |
| 1132 | $C_2H_5$ | $-CH_2-CH_2-Si(CH_3)_3$ | tert.-$C_4H_9$ | |
| 1133 | $C_2H_5$ | $-CH_2-CH_2-O-N=C(CH_3)_2$ | tert.-$C_4H_9$ | |
| 1134 | $C_2H_5$ | $-CH_2-PO(OC_2H_5)_2$ | tert.-$C_4H_9$ | |
| 1135 | $C_2H_5$ | Fur-2-ylmethyl | tert.-$C_4H_9$ | |
| 1136 | $C_2H_5$ | Tetrahydrofur-2-yl-methyl | tert.-$C_4H_9$ | |
| 1137 | $C_2H_5$ | Pyrid-2-yl-methyl | tert.-$C_4H_9$ | |
| 1138 | $C_2H_5$ | Pyrid-4-yl-methyl | tert.-$C_4H_9$ | |
| 1139 | $C_2H_5$ | Pyrid-3-yl-methyl | tert.-$C_4H_9$ | |
| 1140 | $C_2H_5$ | Thien-2-yl-methyl | tert.-$C_4H_9$ | |
| 1141 | $C_2H_5$ | $-CH_2-CH_2-N(CH_3)_2$ | tert.-$C_4H_9$ | |
| 1142 | $C_2H_5$ | $-CH_2-CH_2\overset{\oplus}{N}(CH_3)_3\overset{\ominus}{I}$ | tert.-$C_4H_9$ | |
| 1143 | $C_2H_5$ | $-CH_2-CF_3$ | tert.-$C_4H_9$ | |
| 1144 | $C_2H_5$ | $-CH_2-C(CH_3)=CH_2$ | tert.-$C_4H_9$ | |
| 1145 | $C_2H_5$ | $-CH_2C(Cl)=CH_2$ | tert.-$C_4H_9$ | |
| 1146 | $C_2H_5$ | $-CH_2-C≡C-CH_2OH$ | tert.-$C_4H_9$ | |
| 1147 | $C_2H_5$ | 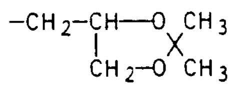 | tert.-$C_4H_9$ | |
| 1148 | $C_2H_5$ | 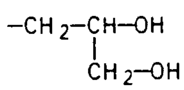 | tert.-$C_4H_9$ | |
| 1149 | $C_2H_5$ | 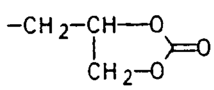 | tert.-$C_4H_9$ | |
| 1150 | $C_2H_5$ | Phenethyl | tert.-$C_4H_9$ | |
| 1151 | $C_2H_5$ | $-CH(C_6H_5)COOCH_3$ | tert.-$C_4H_9$ | |
| 1152 | $C_2H_5$ | Piperidino | tert.-$C_4H_9$ | |
| 1153 | $C_2H_5$ | Phthalimido | tert.-$C_4H_9$ | |

| Nr. | $R^1$ | $R^5$ | $R^4$ | Fp [°C]/$^1$H-NMR ($CDCl_3$) [ppm] |
|---|---|---|---|---|
| 1154 | $C_2H_5$ | 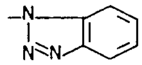 | tert.-$C_4H_9$ | |
| 1155 | $C_2H_5$ | $-N=CH-C_6H_5$ | tert.-$C_4H_9$ | |
| 1156 | $C_2H_5$ | $-N=CH-$ 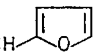 | tert.-$C_4H_9$ | |
| 1157 | $C_2H_5$ | $-CH(CH_3)CH(OCH_3)_2$ | tert.-$C_4H_9$ | |
| 1158 | $C_2H_5$ | $-CH_2-CON(C_2H_5)_2$ | tert.-$C_4H_9$ | |
| 1159 | $C_2H_5$ | $N(C_2H_5)_2$ | tert.-$C_4H_9$ | |
| 1160 | i-$C_3H_7$ | cyclo-$C_6H_{11}$ | tert.-$C_4H_9$ | |
| 1161 | i-$C_3H_7$ | $-CH_2-OCH_2-C_6H_5$ | tert.-$C_4H_9$ | |
| 1162 | i-$C_3H_7$ | Tetrahydro-pyran-2-yl | tert.-$C_4H_9$ | |
| 1163 | i-$C_3H_7$ | Tetrahydro-fur-2-yl | tert.-$C_4H_9$ | |
| 1164 | i-$C_3H_7$ | (4-Brom-benzoyl)-methyl | tert.-$C_4H_9$ | |
| 1165 | i-$C_3H_7$ | (4-Methoxybenzoyl) methyl | tert.-$C_4H_9$ | |
| 1166 | i-$C_3H_7$ | $-CH(COOCH_3)_2$ | tert.-$C_4H_9$ | |
| 1167 | i-$C_3H_7$ | Phthalimidomethyl | tert.-$C_4H_9$ | |
| 1168 | i-$C_3H_7$ | $-CH_2-CH_2-Si(CH_3)_3$ | tert.-$C_4H_9$ | 64- 69 |
| 1169 | i-$C_3H_7$ | $-CH_2-CH_2-O-N=C(CH_3)_2$ | tert.-$C_4H_9$ | |
| 1170 | i-$C_3H_7$ | $-CH_2-PO(OC_2H_5)_2$ | tert.-$C_4H_9$ | |
| 1171 | i-$C_3H_7$ | Fur-2-ylmethyl | tert.-$C_4H_9$ | |
| 1172 | i-$C_3H_7$ | Tetrahydrofur-2-yl-methyl | tert.-$C_4H_9$ | |
| 1173 | i-$C_3H_7$ | Pyrid-2-yl-methyl | tert.-$C_4H_9$ | Öl; 1.30(s;6H) 1.44 (s;9H), 3.40 (sept;1H) 5.52 (s;2H) 7.20-8,64 (m;4H), 8.60 (bs;1H,NH) |
| 1174 | i-$C_3H_7$ | Pyrid-4-yl-methyl | tert.-$C_4H_9$ | |
| 1175 | i-$C_3H_7$ | Pyrid-3-yl-methyl | tert.-$C_4H_9$ | |
| 1176 | i-$C_3H_7$ | Thien-2-yl-methyl | tert.-$C_4H_9$ | |
| 1177 | i-$C_3H_7$ | $-CH_2-CH_2-N(CH_3)_2$ | tert.-$C_4H_9$ | |
| 1178 | i-$C_3H_7$ | $-CH_2-CH_2\overset{\oplus}{N}(CH_3)_3\overset{\ominus}{I}$ | tert.-$C_4H_9$ | |
| 1179 | i-$C_3H_7$ | $-CH_2-CF_3$ | tert.-$C_4H_9$ | |
| 1352 | i-$C_3H_7$ | $-CH_2-C(CH_3)=CH_2$ | tert.-$C_4H_9$ | |

| Nr. | $R^1$ | $R^5$ | $R^4$ | Fp [°C]/$^1$H-NMR ($CDCl_3$) [ppm] |
|---|---|---|---|---|
| 1353 | i-$C_3H_7$ | $-CH_2C(Cl)=CH_2$ | tert.-$C_4H_9$ | |
| 1354 | i-$C_3H_7$ | $-CH_2-C{\equiv}C-CH_2OH$ | tert.-$C_4H_9$ | |
| 1355 | i-$C_3H_7$ | 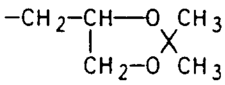 | tert.-$C_4H_9$ | |
| 1356 | i-$C_3H_7$ | 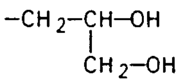 | tert.-$C_4H_9$ | |
| 1357 | i-$C_3H_7$ | 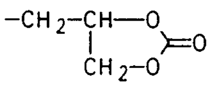 | tert.-$C_4H_9$ | |
| 1180 | i-$C_3H_7$ | Phenethyl | tert.-$C_4H_9$ | |
| 1181 | i-$C_3H_7$ | $-CH(C_6H_5)COOCH_3$ | tert.-$C_4H_9$ | |
| 1182 | i-$C_3H_7$ | Piperidino | tert.-$C_4H_9$ | |
| 1183 | i-$C_3H_7$ | Phthalimido | tert.-$C_4H_9$ | |
| 1184 | i-$C_3H_7$ | 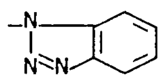 | tert.-$C_4H_9$ | |
| 1185 | i-$C_3H_7$ | $-N=CH-C_6H_5$ | tert.-$C_4H_9$ | |
| 1186 | i-$C_3H_7$ | 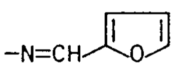 | tert.-$C_4H_9$ | |
| 1187 | i-$C_3H_7$ | $-CH(CH_3CH)(OCH_3)_2$ | tert.-$C_4H_9$ | |
| 1188 | i-$C_3H_7$ | $-CH_2-CON(C_2H_5)_2$ | tert.-$C_4H_9$ | 91- 93 |
| 1189 | i-$C_3H_7$ | $N(C_2H_5)_2$ | tert.-$C_4H_9$ | |
| 1190 | $CH_3$ | $-N=C(CH_3)_2$ | tert.-$C_4H_9$ | 108-109 |
| 1191 | $CH_3$ | Cyclohexanimino | tert.-$C_4H_9$ | 91- 92 |
| 1192 | $CH_3$ | $-N=C(cyclo-C_3H_5)_2$ | tert.-$C_4H_9$ | 50- 52 |
| 1193 | $CH_3$ | H | $-N(CH_3)_2$ | 225-227 |
| 1194 | $CH_3$ | H | Piperidino | 162-164 |
| 1195 | $CH_3$ | $CH_2-C{\equiv}CH$ | tert.-$C_4H_9$ | 90- 95 |
| 1197 | $CH_3$ | $2-NO_2-4-F-C_6H_3$ | tert.-$C_4H_9$ | Öl;1.44(s;9H), 2.59(s;3H), 7.24 und 8.30 (m;3H), 8.16 (bs;1H,NH) |

| Nr. | $R^1$ | $R^5$ | $R^4$ | Fp [°C]/$^1$H-NMR ($CDCl_3$) [ppm] |
|---|---|---|---|---|
| 1198 | $CH_3$ | 3,5-$(CF_3)_2$-$C_6H_3$ | tert.-$C_4H_9$ | 156-159 |
| 1199 | $CH_3$ | H | $CH_3$ | 192-197 |
| 1200 | $CH_3$ | H | $-OC_2H_5$ | 145-148 |
| 1201 | $CH_3$ | H | cyclo-$C_4H_7$ | 141-142 |
| 1202 | $CH_3$ | H | cyclo-$C_3H_5$ | 135-137 |
| 1203 | $CH_3$ | $-N=C(CH_3)_2$ | cyclo-$C_3H_5$ | 91- 93 |
| 1204 | $CH_3$ | H | $C_2H_5$ | 151-154 |
| 1205 | $CH_3$ | $-N=C(CH_3)_2$ | cyclo-$C_4H_7$ | 77- 79 |
| 1207 | $CH_3$ | $CH_2CO_2CH_3$ | tert.-$C_4H_9$ | 88- 89 |
| 1208 | $C_2H_5$ | Succinimido | i-$C_3H_7$ | 132-136 |
| 1209 | n-$C_3H_7$ | H | cyclo-$C_6H_{11}$ | 132-134 |
| 1210 | n-$C_3H_7$ | H | tert.-$C_4H_9$ | 82- 83 |
| 1211 | n-$C_3H_7$ | $-N=C(CH_3)_2$ | tert.-$C_4H_9$ | 66- 68 |
| 1212 | n-$C_3H_7$ | Succinimido | tert.-$C_4H_9$ | 126-129 |
| 1213 | n-$C_3H_7$ | Succinimido | cyclo-$C_3H_5$ | 104-106 |
| 1214 | n-$C_3H_7$ | $-N=C(CH_3)_2$ | cyclo-$C_3H_5$ | Öl 0.70(m;2H), 0.90(m;2H), 1.00(t;3H), 1.78(m;2H), 2.16 und 2.19 (2s;6H), 2.92 (t;2H), 3.00 (m;1H), 9.24 (bs;1H,NH) |
| 1215 | n-$C_3H_7$ | H | cyclo-$C_3H_5$ | 104-106 |
| 1216 | n-$C_3H_7$ | H | i-$C_3H_7$ | 70- 71 |
| 1217 | n-$C_3H_7$ | $-N=C(CH_3)_2$ | i-$C_3H_7$ | 72- 73 |
| 1218 | n-$C_3H_7$ | $-N=C(CH_3)_2$ | cyclo-$C_6H_{11}$ | 110-111 |
| 1219 | n-$C_3H_7$ | H | $C_6H_5$ | 165-166 |
| 1220 | i-$C_3H_7$ | $-N=C(CH_3)_2$ | tert.-$C_4H_9$ | 112-113 |
| 1221 | i-$C_3H_7$ | 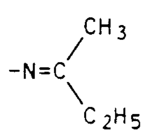 | tert.-$C_4H_9$ | 83- 86 |
| 1222 | i-$C_3H_7$ | Cyclohexanimino | tert.-$C_4H_9$ | 91- 94 |
| 1223 | i-$C_3H_7$ | -N=C(cyclo-$C_3H_5)_2$ | tert.-$C_4H_9$ | 70- 75 |
| 1224 | i-$C_3H_7$ | $-N=C(CH_3)_2$ | Tetrahydrofur-3-yl | 104-106 |
| 1225 | i-$C_3H_7$ | Succinimido | Tetrahydrofur-3-yl | 160-162 |

| Nr. | $R^1$ | $R^5$ | $R^4$ | Fp [°C]/$^1$H-NMR ($CDCl_3$) [ppm] |
|---|---|---|---|---|
| 1226 | $i-C_3H_7$ | H | Tetrahydrofur-3-yl | Öl 1.33(d;6H), 2.40(m;2H), 3.75(sept;1H), 4.00(m;4H), 4.75 (m;1H), 8.25 (d;1H,NH) |
| 1227 | $i-C_3H_7$ | H | $OC_2H_5$ | 134-135 |
| 1228 | $i-C_3H_7$ | Succinimido | $OC_2H_5$ | 146-148 |
| 1229 | $i-C_3H_7$ | H | Thiazol-2-yl | 195 |
| 1230 | $i-C_3H_7$ | H | 5-Methyl-thiazol-2-yl | 248 |
| 1231 | $i-C_3H_7$ | H | 5-Ethyl-thiazol-2-yl | 228-230 |
| 1232 | $i-C_3H_7$ | H | 5-n-Propyl-thiazol-2-yl | 160-163 |
| 1233 | $i-C_3H_7$ | Succinimido | tert.-$C_4H_9$ | 141-144 |
| 1234 | $i-C_3H_7$ | H | cyclo-$C_4H_7$ | 95- 96 |
| 1235 | $i-C_3H_7$ | $-N=C(CH_3)_2$ | cyclo-$C_4H_7$ | 100-101 |
| 1236 | $i-C_3H_7$ | $-N=C(CH_3)_2$ | $-N(CH_3)_2$ | 129-131 |
| 1237 | $i-C_3H_7$ | H | $-N(CH_3)_2$ | 163-165 |
| 1238 | $i-C_3H_7$ | H | Piperidino | 167-168 |
| 1239 | $i-C_3H_7$ | H | Morpholino | 177-179 |
| 1240 | $i-C_3H_7$ | H | cyclo-$C_5H_9$ | 62- 65 |
| 1241 | $i-C_3H_7$ | H | Cyclopropylmethyl | 88- 90 |
| 1242 | $i-C_3H_7$ | H | s-$C_4H_9$ | öl;1.00(t;3H), 1.34(d;3H), 1.37(d;6H), 1.66(quint;2H), 3.78 (sept;1H), 4.17(m;1H), 7.04 (d,1H,NH) |
| 1243 | $i-C_3H_7$ | $-N=C(CH_3)_2$ | s-$C_4H_9$ | öl;0.98 (t;3H), 1,26 (d,3H), 1,39 (d,6H) 1,64 (quint; 2H), 2,16 und 2,18 (2s; 6H) 3,44 (sept;1H), 4,10 (m,1H), 8,54 (d,1H,NH) |

| Nr. | $R^1$ | $R^5$ | $R^4$ | Fp [°C]/$^1H$-NMR ($CDCl_3$) [ppm] |
|---|---|---|---|---|
| 1244 | $i-C_3H_7$ | H | Gemisch aus 4-Methyl-5-carboxy-thiazol-2-yl und 4-Methyl-thiazol-2-yl | 197 |
| 1245 | $i-C_3H_7$ | $CH_2-CH=CH-C_6H_5$ | tert.-$C_4H_9$ | 59- 63 |
| 1246 | $i-C_3H_7$ | $4-CO_2CH_3C_6H_4$ | tert.-$C_4H_9$ | 143-145 |
| 1247 | $i-C_3H_7$ | $CH_2-CH_2-CN$ | tert.-$C_4H_9$ | 67- 71 |
| 1248 | $i-C_3H_7$ | $CH_2-CCl_3$ | tert.-$C_4H_9$ | 72- 75 |
| 1249 | $i-C_3H_7$ | $4-NHCOCH_3-C_6H_4$ | tert.-$C_4H_9$ | 212-214 |
| 1250 | $i-C_3H_7$ | $2,4-Cl_2-C_6H_3$ | tert.-$C_4H_9$ | 140-141 |
| 1251 | $i-C_3H_7$ | Cyclooctanimino | tert.-$C_4H_9$ | öl, 1,37(d,6H), 1,47(s;9H), 1,28-1,93 (m;10H), 2,54 (m;4H), 3,44 (sept,1H), 8,44 (bs;1H,NH) |
| 1252 | $i-C_3H_7$ | $(CH_2)_2O(CH_2)_2OCH_3$ | tert.-$C_4H_9$ | öl, 1,34(d;6H), 1,47 (s;9H), 3,39 (s;3H), 3,45 (sept;1H), 3,60 (m;4H), 3,84 und 4,50 (m;4H), 8,94 (bs;1H,NH) |
| 1253 | $i-C_3H_7$ | $CH_2-CH_2-S-CH_3$ | tert.-$C_4H_9$ | 46- 48 |
| 1254 | $i-C_3H_7$ | Pyrid-2-yl | tert.-$C_4H_9$ | 155-163 |
| 1255 | $i-C_3H_7$ | $CH_2-CH_2-Cl$ | tert.-$C_4H_9$ | 70- 72 |
| 1263 | $n-C_4H_9$ | H | $CH_3$ | 146-149 |
| 1264 | $n-C_4H_9$ | H | $i-C_3H_7$ | 60- 63 |
| 1265 | $n-C_4H_9$ | H | cyclo-$C_3H_5$ | 112-114 |
| 1266 | $n-C_4H_9$ | H | $C_6H_5$ | 145-150 |

| Nr. | $R^1$ | $R^5$ | $R^4$ | Fp [°C]/$^1H$-NMR ($CDCl_3$) [ppm] |
|---|---|---|---|---|
| 1267 | n-$C_4H_9$ | H | tert.-$C_4H_9$ | 52- 54 |
| 1268 | n-$C_4H_9$ | $-N=C(CH_3)_2$ | tert.-$C_4H_9$ | 58- 62 |
| 1269 | n-$C_4H_9$ | $-CH_2CCl_3$ | tert.-$C_4H_9$ | öl 0,92 (t;3H), 1,43 (m,2H), 1,48(s;9H), 1,74(m;2H) 3,00(t;2H), 5,01(s;2H), 8,80(bs;1H;NH) |
| 1270 | n-$C_4H_9$ | 2,6-$Br_2$-4-CN-$C_6H_2$ | tert.-$C_4H_9$ | 165-170 |
| 1271 | n-$C_4H_9$ | $-CH_2-CH=CH_2$ | tert.-$C_4H_9$ | öl 0,92(t;3H), 1,41(m;2H), 1,46(s;9H), 1,66(m;2H), 2,89(t;2H), 4,88(d;2H), 5,40(m;2H), 6,01(m;1H), 9,19(bs;1H,NH) |
| 1272 | n-$C_4H_9$ | 2,4-Dichlorbenzyl | tert.-$C_4H_9$ | 81- 86 |
| 1273 | n-$C_4H_9$ | H | Cyclopropylmethyl | 65- 70 |
| 1274 | s-$C_4H_9$ | H | i-$C_3H_7$ | öl 0,92 (t,3H), 1,34(d,3H), 1,39(d;6H), 1,76(m;2H), 3,65(m;1H), 4,32(m;1H), 7,08 (bs;1H,NH) |
| 1275 | s-$C_4H_9$ | $-N=C(CH_3)_2$ | i-$C_3H_7$ | 80 - 84 |
| 1276 | s-$C_4H_9$ | H | cyclo-$C_3H_7$ | 78- 85 |
| 1277 | s-$C_4H_9$ | $-N=C(CH_3)_2$ | cyclo-$C_3H_5$ | öl 0,70(m,;2H), 0,90 (m;2H), 0,92 (t;3H), 1,34 (d;3H), 1,79 (m;2H), 2,14 und 2,18 (2s;6H), 2,97 (m;1H), 3,24 (m;1H), 8,80 (bs,1H,NH) |

| Nr. | $R^1$ | $R^5$ | $R^4$ | Fp [°C]/$^1H$-NMR ($CDCl_3$) [ppm] |
|---|---|---|---|---|
| 1278 | s-$C_4H_9$ | Succinimido | cyclo-$C_3H_5$ | 112-115 |
| 1279 | s-$C_4H_9$ | H | tert.-$C_4H_9$ | 93- 95 |
| 1280 | s-$C_4H_9$ | -N=C($CH_3$)$_2$ | tert.-$C_4H_9$ | Öl 0,92 (t,3H), 1,34 (d;3H), 1,48 (s;9H), 1,80 (m;2H), 2,12 und 2,16 (2s;6H), 3,26 (m,1H), 8,29 (bs,1H,NH) |
| 1281 | s-$C_4H_9$ | H | $C_6H_5$ | 117-120 |
| 1282 | s-$C_4H_9$ | -N=C($CH_3$)$_2$ | $C_6H_5$ | Öl, 0,93(t;3H), 1,36 (d;3H), 1,80 (m;2H), 2,14 und 2,18 (2s;6H), 3,28 (m;1H), 7,10-7,80(m;5H) 10,90(bs;1H),NH) |
| 1283 | tert.-$C_4H_9$ | Succinimido | i-$C_3H_7$ | 137-140 |
| 1284 | tert.-$C_4H_9$ | Succinimido | 4-Cl-$C_6H_4$ | 238-242 |
| 1285 | tert.-$C_4H_9$ | Succinimido | tert.-$C_4H_9$ | 144-146 |
| 1286 | tert.-$C_4H_9$ | -N=C($CH_3$)$_2$ | tert.-$C_4H_9$ | 86- 90 |
| 1287 | tert.-$C_4H_9$ | H | cyclo-$C_3H_5$ | 75- 77 |
| 1288 | tert.-$C_4H_9$ | -N=C($CH_3$)$_2$ | cyclo-$C_3H_5$ | 93- 98 |
| 1290 | neo-$C_5H_{11}$ | H | $CH_3$ | 130-133 |
| 1291 | neo-$C_5H_{11}$ | H | i-$C_3H_7$ | 100-104 |
| 1292 | neo-$C_5H_{11}$ | H | cyclo-$C_3H_5$ | 133-136 |
| 1293 | neo-$C_5H_{11}$ | -N=C($CH_3$)$_2$ | cyclo-$C_3H_5$ | 56- 62 |
| 1294 | neo-$C_5H_{11}$ | H | tert.-$C_4H_9$ | 112-117 |
| 1295 | neo-$C_5H_{11}$ | -N=C($CH_3$)$_2$ | tert.-$C_4H_9$ | 107-111 |
| 1296 | neo-$C_5H_{11}$ | H | $C_6H_5$ | 205-207 |
| 1297 | cyclo-$C_3H_5$ | Succinimido | i-$C_3H_7$ | 131-133 |
| 1298 | cyclo-$C_3H_5$ | Succinimido | tert.-$C_4H_9$ | 167-168 |
| 1299 | cyclo-$C_3H_5$ | Succinimido | $C_6H_5$ | 168-170 |
| 1300 | cyclo-$C_3H_5$ | H | cyclo-$C_3H_5$ | 139-140 |
| 1301 | cyclo-$C_3H_5$ | -N=C($CH_3$)$_2$ | cyclo-$C_3H_5$ | Öl 0,80 (m;8H), 2,02 und 2,04 (2s; 6H), 2,30 (m;1H), 2,86 (m;1H), 9,20 (d;1H,NH) |

| Nr. | $R^1$ | $R^5$ | $R^4$ | Fp [°C]/$^1$H-NMR ($CDCl_3$) [ppm] |
|---|---|---|---|---|
| 1302 | cyclo-$C_3H_5$ | -N=C(cyclo-$C_3H_5$)$_2$ | cyclo-$C_3H_7$ | 106-108 |
| 1303 | cyclo-$C_3H_5$ | H | cyclo-$C_5H_9$ | 106-109 |
| 1304 | cyclo-$C_3H_5$ | -N=C($CH_3$)$_2$ | cyclo-$C_5H_9$ | 125-127 |
| 1305 | cyclo-$C_5H_9$ | H | $C_6H_5$ | 170-171 |
| 1306 | cyclo-$C_5H_9$ | H | cyclo-$C_3H_5$ | 118-120 |
| 1307 | cyclo-$C_5H_9$ | -N=C($CH_3$)$_2$ | cyclo-$C_3H_5$ | 55- 57 |
| 1308 | cyclo-$C_5H_9$ | -N=C($CH_3$)$_2$ | $CH_3$ | 100-101 |
| 1309 | cyclo-$C_5H_9$ | H | $CH_3$ | 166-167 |
| 1310 | cyclo-$C_5H_9$ | H | tert.-$C_4H_9$ | 125-126 |
| 1311 | cyclo-$C_5H_9$ | -N=C($CH_3$)$_2$ | tert.-$C_4H_9$ | 112-114 |
| 1312 | Tetrahydro-pyran-3-yl | Succinimido | $C_6H_5$ | 80 |
| 1313 | 2-$CH_3O$-$C_6H_4$ | H | tert.-$C_4H_9$ | 179-184 |
| 1314 | 2-$CH_3O$-$C_6H_4$ | H | cyclo-$C_4H_5$ | 177-180 |
| 1315 | 2,6$F_2$-$C_6H_3$ | H | tert.-$C_4H_9$ | 128-135 |
| 1316 | 2,6$F_2$-$C_6H_3$ | H | cyclo-$C_3H_5$ | 134-138 |
| 1317 | $CH_3$-O- | H | tert.-$C_4H_9$ | Öl 1,52 (s;9H), 4,12(s;3H), 7,16(bs;1H,NH) |
| 1318 | $CH_3$-O-$CH_2$ | H | tert.-$C_4H_9$ | 95-100 |
| 1319 | $CH_3$-O-$CH_2$ | H | cyclo-$C_3H_5$ | 90- 95 |
| 1320 | $CH_3$-O-$CH_2$ | -N=C($CH_3$)$_2$ | tert.-$C_4H_9$ | 65- 70 |
| 1321 | $CH_3$-O-CH($CH_3$)- | -N=C($CH_3$)$_2$ | tert.-$C_4H_9$ | Öl 1,45 (s;9H) 1,60 (d;3H), 2,16 und 2,18 (2s;6H), 3,34 (s;3H), 4,87 (quart.; 1H) 8,10(bs;1H,NH) |
| 1322 | $CH_3$-O-CH($CH_3$)- | H | tert.-$C_4H_9$ | 69 - 71 |
| 1323 | $CH_3$-O-CH($CH_3$)- | 2,6-$Br_2$-4-CN-$C_6H_2$ | tert.-$C_4H_9$ | 118-120 |
| 1324 | $CH_3$ | CH($CH_3$)$CO_2CH_3$ | tert.-$C_4H_9$ | Öl 1,46 (s;9H) 2,54 (s;3H) 3,82 (s;3H), 5,40(quart.;1H) 9,00(bs;1H,NH) |

| Nr. | $R^1$ | $R^5$ | $R^4$ | Fp [°C]/$^1$H-NMR ($CDCl_3$) [ppm] |
|---|---|---|---|---|
| 1325 | $CH_3$ | 2,6-$Br_2$-4-CN-$C_6H_2$ | tert.-$C_4H_9$ | 143-146 |
| 1326 | 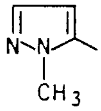 | H | tert.-$C_4H_9$ | 168-170 |
| 1327 | 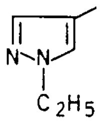 | H | tert.-$C_4H_9$ | 157 |
| 1328 | 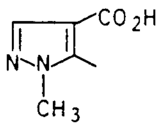 | H | tert.-$C_4H_9$ | 254 |
| 1329 | 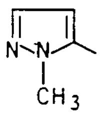 | $CH_3$ | tert.-$C_4H_9$ | Öl, 1,5 (s,9H)<br>3,8;(s;3H),<br>3,9 (s;3H),<br>6,5 (d;1H),<br>7,6 (d;1H),<br>8,7 (bs, 1H,NH) |
| 1330 | 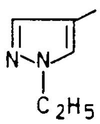 | $CH_3$ | tert.-$C_4H_9$ | 120-122°C |
| 1331 | 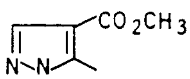 | $CH_3$ | tert.-$C_4H_9$ | Öl, 1,5 (s;9H),<br>3,7 (s;3H),<br>3,8 (s;3H),<br>3,9 (s;3H),<br>8,0 (s;1H),<br>9,2 (bs,1H,NH) |
| 1332 | 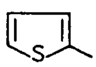 | H | tert.-$C_4H_9$ | |
| 1333 | 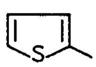 | -N=C($CH_3$)$_2$ | tert.-$C_4H_9$ | |
| 1334 | 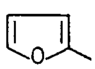 | H | tert.-$C_4H_9$ | |
| 1335 | 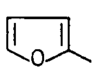 | -N=C($CH_3$)$_2$ | tert.-$C_4H_9$ | |

| Nr. | $R^1$ | $R^5$ | $R^4$ | Fp [°C]/$^1$H-NMR ($CDCl_3$) [ppm] |
|---|---|---|---|---|
| 1336 | Pyrid-2-yl | H | tert.-$C_4H_9$ | |
| 1337 | Pyrid-2-yl | $-N=C(CH_3)_2$ | tert.-$C_4H_9$ | |
| 1338 | Pyrid-3-yl | H | tert.-$C_4H_9$ | |
| 1339 | Pyrid-3-yl | $-N=C(CH_3)_2$ | tert.-$C_4H_9$ | |
| 1340 | Pyrid-4-yl | H | tert.-$C_4H_9$ | |
| 1341 | Pyrid-4-yl | $-N=C(CH_3)_2$ | tert.-$C_4H_9$ | |
| 1342 | $4-F-C_6H_4-CH_2$ | H | tert.-$C_4H_9$ | |
| 1343 | $4-F-C_6H_4-CH_2$ | $-N=C(CH_3)_2$ | tert.-$C_4H_9$ | |
| 1344 | $CH_3$ | n-$C_4H_9$ | tert.-$C_4H_9$ | |
| 1345 | $CH_3$ | $C_6H_5$ | tert.-$C_4H_9$ | |
| 1346 | $CH_3$ | tert.-$C_4H_9$ | tert.-$C_4H_9$ | |
| 1347 | $C_2H_5$ | n-$C_4H_9$ | tert.-$C_4H_9$ | |
| 1348 | $C_2H_5$ | $C_6H_5$ | tert.-$C_4H_9$ | |
| 1349 | i-$C_3H_7$ | n-$C_4H_9$ | tert.-$C_4H_9$ | |
| 1350 | i-$C_3H_7$ | $C_6H_5$ | tert.-$C_4H_9$ | |
| 1351 | i-$C_3H_7$ | Cyclopentanimino | tert.-$C_4H_9$ | 113-115 |

Tabelle 2

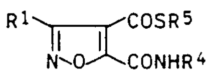

| Nr. | $R^1$ | $R^5$ | $R^4$ | Fp [°C]/$^1$H-NMR ($CDCl_3$) [ppm] |
|---|---|---|---|---|
| 2001 | $CH_3$ | n-$C_4H_9$ | tert.-$C_4H_9$ | |
| 2002 | $CH_3$ | $C_6H_5$ | tert.-$C_4H_9$ | |
| 2003 | $CH_3$ | tert.-$C_4H_9$ | tert.-$C_4H_9$ | Öl; 1,46 (s;9H)<br>1,60 (s; 9H),<br>2,50 (s; 3H),<br>7,94 (bs;<br>1H, NH) |
| 2004 | $C_2H_5$ | n-$C_4H_9$ | tert.-$C_4H_9$ | |
| 2005 | $C_2H_5$ | $C_6H_5$ | tert.-$C_4H_9$ | |
| 2006 | i-$C_3H_7$ | $C_6H_5$ | tert.-$C_4H_9$ | |
| 2007 | i-$C_3H_7$ | n-$C_4H_9$ | tert.-$C_4H_9$ | |

Tabelle 3

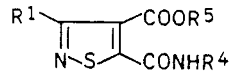

| Nr. | $R^1$ | $R^5$ | $R^4$ | Fp [°C]/H-NMR ($CDCl_3$) [ppm] |
|---|---|---|---|---|
| 3001 | $CH_3$ | H | i-$C_3H_7$ | 153-154 |
| 3002 | $CH_3$ | H | tert.-$C_4H_9$ | 158-159 |
| 3003 | $CH_3$ | H | $C_6H_5$ | 178-183 |
| 3004 | $CH_3$ | H | 4-Cl-$C_6H_4$ | 231 |
| 3005 | $CH_3$ | H | 3-$CF_3$-$C_6H_4$ | 221-223 |
| 3006 | $C_2H_5$ | H | i-$C_3H_7$ | 138-140 |
| 3007 | $C_2H_5$ | $CH_3$ | i-$C_3H_7$ | 55- 56 |
| 3008 | $C_2H_5$ | H | tert.-$C_4H_9$ | 155-157 |
| 3009 | $C_2H_5$ | $CH_3$ | tert.-$C_4H_9$ | 39 |
| 3010 | $C_2H_5$ | H | $C_6H_5$ | 164 |
| 3011 | $C_2H_5$ | $CH_3$ | $C_6H_5$ | 130-131 |
| 3012 | $C_2H_5$ | H | 4-Cl-$C_6H_4$ | 202-204 |
| 3013 | $C_2H_5$ | $CH_3$ | 4-Cl-$C_6H_4$ | 158-159 |
| 3014 | $C_2H_5$ | H | 3-$CF_3$-$C_6H_4$ | 191-195 |
| 3015 | $C_2H_5$ | $CH_3$ | 3-$CF_3$-$C_6H_4$ | 84 |
| 3016 | i-$C_3H_7$ | H | i-$C_3H_7$ | 160-162 |
| 3017 | i-$C_3H_7$ | $CH_3$ | i-$C_3H_7$ | 90- 91 |
| 3018 | i-$C_3H_7$ | H | tert.-$C_4H_9$ | 178-179 |
| 3019 | i-$C_3H_7$ | $CH_3$ | tert.-$C_4H_9$ | 38- 41 |
| 3020 | i-$C_3H_7$ | H | $C_6H_5$ | 171-172 |
| 3021 | i-$C_3H_7$ | $CH_3$ | $C_6H_5$ | 92- 93 |
| 3022 | i-$C_3H_7$ | H | 4-Cl-$C_6H_4$ | 185-186 |
| 3023 | i-$C_3H_7$ | $CH_3$ | 4-Cl-$C_6H_4$ | 92- 93 |
| 3024 | i-$C_3H_7$ | H | 3-$CF_3$-$C_6H_4$ | 177-179 |
| 3025 | i-$C_3H_7$ | $CH_3$ | 3-$CF_3$-$C_6H_4$ | 37- 45 |
| 3026 | $C_6H_5$ | H | i-$C_3H_7$ | 144 |
| 3027 | $C_6H_5$ | $CH_3$ | i-$C_3H_7$ | 98-100 |
| 3028 | $C_6H_5$ | H | tert.-$C_4H_9$ | 209 |
| 3029 | $C_6H_5$ | $CH_3$ | tert.-$C_4H_9$ | 130-131 |
| 3030 | $C_6H_5$ | H | $C_6H_5$ | 204 |
| 3031 | $C_6H_5$ | $CH_3$ | $C_6H_5$ | 100-101 |
| 3032 | $C_6H_5$ | H | 4-Cl-$C_6H_4$ | 209 |
| 3033 | $C_6H_5$ | $CH_3$ | 4-Cl-$C_6H_4$ | 157-158 |
| 3034 | $C_6H_5$ | H | 3-$CF_3$-$C_6H_4$ | 217 |
| 3035 | $C_6H_5$ | $CH_3$ | 3-$CF_3$-$C_6H_4$ | 131-132 |
| 3036 | i-$C_3H_7$ | Succinimido | i-$C_3H_7$ | 98-100 |
| 3037 | i-$C_3H_7$ | Succinimdio | tert.-$C_4H_9$ | 75- 76 |

| Nr. | R1 | R5 | R4 | Fp [°C]/H-NMR ($CDCl_3$) [ppm] |
|---|---|---|---|---|
| 3038 | $i-C_3H_7$ | $Na^{\oplus}$ | tert.-$C_4H_9$ | 300 |
| 3039 | $i-C_3H_7$ | $K^{\oplus}$ | tert.-$C_4H_9$ | 110 |
| 3040 | $i-C_3H_7$ | $Na^{\oplus}$ | $C_6H_5$ | 330 |
| 3041 | $i-C_3H_7$ | $K^{\oplus}$ | $C_6H_5$ | 300 |
| 3042 | $i-C_3H_7$ | $\overset{\oplus}{H_3N}-CH(CH_3)_2$ | $C_6H_5$ | 154-157 |
| 3043 | $i-C_3H_7$ | $\overset{\oplus}{H_3N}-CH_2-CH_2-OH$ | $C_6H_5$ | 162-164 |
| 3044 | $i-C_3H_7$ | Succinimido | $C_6H_5$ | 161 |
| 3045 | $CH_3$ | $-N=C(CH_3)_2$ | tert.-$C_4H_9$ | 97- 98 |

Analog können darüber hinaus beispielsweise weitere Verbindungen hergestellt werden mit der allgemeinen Struktur

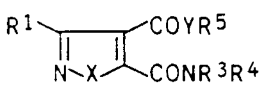

wobei für X und Y Sauerstoff oder Schwefel, und beispielsweise
$R^1$ für einen Rest aus der Gruppe Q1 bis Q61,
$R^5$ für einen Rest aus der Gruppe M1 bis M78,
$R^3$ für einen Rest aus der Gruppe $P_1$-$P_{11}$
$R^4$ für einen Rest aus der Gruppe L1 bis L195 stehen und
die Reste X, Y, P, Q, M und L beliebig kombiniert werden können.

$R^1$, $R^5$, $R^3$ und $R^4$ können beispielsweise die folgenden Reste bedeuten:

| Verb. Nr. | $R^1$ | Verb. Nr. | $R^1$ |
|---|---|---|---|
| Q1 | H | Q24 | Tetrahydropyran-3-yl |
| Q2 | $CH_3$ | | |
| Q3 | $C_2H_5$ | Q25 | Tetrahydropyran-3-yl |
| Q4 | $n-C_3H_7$ | Q26 | $C_6H_5$ |
| Q5 | $i-C_3H_7$ | Q27 | $2-F-C_6H_4$ |
| Q6 | $n-C_4H_9$ | Q28 | $3-F-C_6H_4$ |
| Q7 | $i-C_4H_9$ | Q29 | $4-F-C_6H_4$ |
| Q8 | $s-C_4H_9$ | Q30 | $2-Cl-C_6H_4$ |
| Q9 | tert.-$C_4H_9$ | Q31 | $3-Cl-C_6H_4$ |
| Q10 | cyclo-$C_3H_5$ | Q32 | $4-Cl-C_6H_4$ |
| Q11 | cyclo-$C_4H_7$ | Q33 | $2-CH_3-C_6H_4$ |
| Q12 | cyclo-$C_5H_9$ | Q34 | $3-CH_3-C_6H_4$ |
| Q13 | cyclo-$C_6H_{11}$ | Q35 | $4-CH_3-C_6H_4$ |
| Q14 | cyclo-$C_7H_{13}$ | Q36 | $2-CF_3-C_6H_4$ |

| Verb. Nr. | $R^1$ | Verb. Nr. | $R^1$ |
|---|---|---|---|
| Q15 | cyclo-$C_8H_{15}$ | Q37 | 3-$CF_3$-$C_6H_4$ |
| Q16 | $CF_3$ | Q38 | 4-$CF_3$-$C_6H_4$ |
| Q17 | $CH_2OCH_3$ | Q39 | 2-$OCH_3$-$C_6H_4$ |
| Q18 | $CH(CH_3)OCH_3$ | Q40 | 3-$OCH_3$-$C_6H_4$ |
| Q19 | $CH(CH_3)CH_2OCH_3$ | Q41 | 4-$OCH_3$-$C_6H_4$ |
| Q20 | $CH_2OC_2H_5$ | Q42 | 4-$OCF_3$-$C_6H_4$ |
| Q21 | Tetrahydrofur-2-yl | Q43 | 4-$SCH_3$-$C_6H_4$ |
| | | Q44 | 4-$SCF_3$-$C_6H_4$ |
| Q22 | Tetrahydrofur-2-yl | Q45 | 4-$NO_2$-$C_6H_4$ |
| | | Q46 | 4-CN-$C_6H_4$ |
| Q23 | Tetrahydrofur-2-yl | | |

| | | | |
|---|---|---|---|
| Q47 | neo-$C_5H_{11}$ | Q56 | 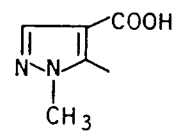 |
| Q48 | $CH_3O$ | | |
| Q49 | $C_2H_5O$ | | |
| Q50 | $C_6H_5-CH_2$ | Q57 | 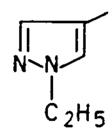 |
| Q51 | 4-F-$C_6H_4-CH_2$ | | |
| Q51 | 4-$CH_3$-$C_6H_4$-$CH_2$- | | |
| Q52 | 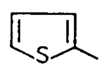 | Q58 | 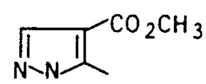 |
| Q53 | 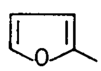 | Q59 | Pyrid-2-yl |
| | | Q60 | Pyrid-3-yl |
| Q54 | 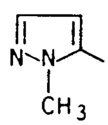 | Q61 | Pyrid-4-yl |
| | | Q62 | 2,6-$F_2$-$C_6H_3$ |
| Q55 | 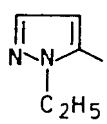 | | |

| Verb. Nr. | $R^5$ |
|---|---|
| M1 | H |
| M2 | $CH_3$ |
| M3 | $C_2H_5$ |
| M4 | $n-C_3H_7$ |
| M5 | $i-C_3H_7$ |
| M6 | $n-C_4H_9$ |
| M7 | $s-C_4H_9$ |
| M8 | $t-C_4H_9$ |
| M9 | $CH(CH_3)C_6H_{13}$ |
| M10 | $CH_2CH_2OCH_3$ |
| M11 | $CH_2CH_2OC_2H_5$ |
| M12 | Succinimido |
| M13 | $Li^{\oplus}$ |
| M14 | $Na^{\oplus}$ |
| M15 | $K^{\oplus}$ |
| M16 | $NH_4^{\oplus}$ |
| M17 | $H_3N^{\oplus}i-C_3H_7$ |
| M18 | $H_2N^{\oplus}(i-C_3H_7)_2$ |
| M19 | $H_3N^{\oplus}CH_2CH_2OH$ |
| M20 | $CH_2CH=CH_2$ |
| M21 | $CH_2-C(CH_3)=CH_2$ |
| M22 | $CH_2-C(Cl)=CH_2$ |
| M23 | $CH_2-C\equiv CH$ |
| M24 | $CH_2-C\equiv C-CH_2OH$ |
| M25 | $-N=C(CH_3)_2$ |
| M26 | $-N=C(C_2H_5)_2$ |
| M27 | $CH_2-CH_2-N(CH_3)_2$ |
| M28 | $CH_2-CH_2-N(C_2H_5)_2$ |
| M29 | $CH_2-CH_2N^{\oplus}(CH_3)_3I^{\ominus}$ |
| M30 | $CH_2-CF_3$ |
| M31 | Phenyl |
| M32 | Phenylethyl |
| M33 | $CH_2-CH_2-Si(CH_3)_3$ |
| M34 | $CH_2-CH_2-ON=C(CH_3)_2$ |
| M35 | $CH_2-PO(OC_2H_5)_2$ |
| M36 | $CH(CH_3)CH(OCH_3)_2$ |
| M37 | $CH_2-CON(C_2H_5)_2$ |
| M38 | $N(C_2H_5)_2$ |
| M39 | $CH_2-OCH_2-C_6H_5$ |

| Verb. Nr. | $R^5$ |
|---|---|
| M40 | $CH(COOCH_3)_2$ |
| M41 | $-N=C(cyclo-C_3H_5)_2$ |
| M42 | 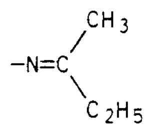 |
| M43 | Cyclohexanimino |
| M44 | Cyclooctanimino |
| M45 | $CH_2-CH_2-Cl$ |
| M46 | $CH_2-CH_2-CN$ |
| M47 | $CH_2-CCl_3$ |
| M48 | Pyrid-3-ylmethyl |
| M49 | Thien-2-yl-methyl |
| M50 | 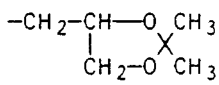 |
| M51 | 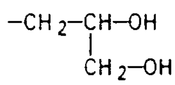 |
| M52 | 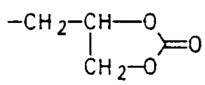 |
| M53 | $-CH(C_6H_5)COOCH_3$ |
| M54 | cyclo-$C_6H_{11}$ |
| M55 | $-CH_2-OCH_2-C_6H_5$ |
| M56 | Tetrahydropyran-2-yl |
| M57 | Tetrahydrofur-2-yl |
| M58 | (4-Brom-benzoyl)methyl |
| M59 | (4-Methoxybenzoyl)methyl |
| M60 | $-CH(COOCH_3)_2$ |
| M61 | Phthalimidomethyl |
| M62 | Fur-2-ylmethyl |
| M63 | Tetrahydrofur-2-yl-methyl |
| M64 | Pyrid-2-ylmethyl |
| M65 | Pyrid-4-ylmethyl |
| M66 | Pyrid-3-yl-methyl |
| M67 | Thien-2-yl-methyl |
| M68 | $-CH(C_6H_5)COOCH_3$ |
| M69 | Piperidino |
| M70 | Phthalimido |

| Verb. Nr. | $R^5$ |
|---|---|
| M71 | 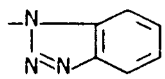 |
| M72 | $-N=CH-C_6H_5$ |
| M73 | 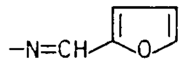 |
| M74 | $2-NO_2-4-F-C_6H_3$ |
| M75 | $3,5-(CF_3)_2-C_6H_3$ |
| M76 | $CH_2-CH_2-S-CH_3$ |
| M77 | $4-NHCOCH_3-C_6H_4$ |
| M78 | 2,4-Dichlorbenzyl |

| Verb. Nr. | $R^3$ | Verb. Nr. | $R^3$ |
|---|---|---|---|
| P1 | H | P12 | $CH_2OC_2H_5$ |
| P2 | $CH_3$ | P13 | $CH_2CH_2OCH_3$ |
| P3 | $C_2H_5$ | P14 | $CH_2SCH_3$ |
| P4 | $n-C_3H_7$ | P15 | $CH_2SC_2H_5$ |
| P5 | $i-C_3H_7$ | P16 | $CH_2CH_2SCH_3$ |
| P6 | $n-C_4H_9$ | P17 | $CH_2-CH_2-N(CH_3)_2$ |
| P7 | $s-C_4H_9$ | P18 | $CH_2CH_2-N(C_2H_5)_2$ |
| P8 | $t-C_4H_9$ | P19 | $cyclo-C_3H_5$ |
| P9 | $CH_2-CH_2OH$ | P20 | $cyclo-C_6H_{11}$ |
| P10 | $CH_2-CH_2Cl$ | P21 | $1-Methyl-cyclo-C_6H_{10}$ |
| P11 | $CH_2OCH_3$ | | |

| Verb. Nr. | $R^4$ | Verb. Nr. | $R^4$ |
|---|---|---|---|
| L1 | H | L21 | $-CH(C_2H_5)C_5H_{11}$ |
| L2 | $CH_3$ | L22 | $-C(CH_3)_2CH_2C(CH_3)_3$ |
| L3 | $C_2H_5$ | L23 | cyclo-$C_3H_5$ |
| L4 | n-$C_3H_7$ | L24 | cyclo-$C_4H_7$ |
| L5 | i-$C_3H_7$ | L25 | cyclo-$C_5H_9$ |
| L6 | n-$C_4H_9$ | L26 | cyclo-$C_6H_{11}$ |
| L7 | i-$C_4H_9$ | L27 | cyclo-$C_7H_{13}$ |
| L8 | sec-$C_4H_9$ | L28 | cyclo-$C_8H_{15}$ |
| L9 | tert.-$C_4H_9$ | L29 | 1-Methylcyclohexyl |
| L10 | n-$C_5H_{11}$ | L30 | 1-Ethylcyclohexyl |
| L11 | $-CH(CH_3)C_3H_7$ | L31 | 3,5-Dimethylcyclohexyl |
| L12 | $-CH(C_2H_5)C_2H_5$ | L32 | 3-Trifluormethylcyclohexyl |
| L13 | n-$C_6H_{13}$ | L33 | Tetrahydropyran-4-yl |
| L14 | $-CH(CH_3)C_4H_9$ | L34 | 4-Methyl-tetrahydropyran |
| L15 | $-CH(C_2H_5)C_3H_7$ | | |
| L16 | n-$C_7H_{15}$ | | |
| L17 | $-CH(CH_3)C_5H_{11}$ | | |
| L18 | $-CH(C_2H_5)C_4H_9$ | | |
| L19 | n-$C_8H_{17}$ | | |
| L20 | $-CH(CH_3)C_6H_{13}$ | | |

| Verb. Nr. | $R^4$ | Verb. Nr. | $R^4$ |
|---|---|---|---|
| L35 | 4-Methyl-tetrahydropyran-4-yl | L72 | $-CH_2CH_2CH_2OCH_3$ |
| | | L73 | $-CH_2CH_2CH_2N(CH_3)_2$ |
| L36 | $-CH_2-CH=CH_2$ | L74 | $-CH_2CH_2CH_2N(C_2H_5)_2$ |
| L37 | $-CH(CH_3)CH=CH_2$ | L75 | $2-CH_3-C_6H_4$ |
| L38 | $-C(CH_3)_2CH=CH_2$ | L76 | $3-CH_3-C_6H_4$ |
| L39 | $-C(CH_3,C_2H_5)CH=CH_2$ | L77 | $4-CH_3-C_6H_4$ |
| L40 | $-C(CH_3)_2-C_2H_5$ | L78 | $2-C_2H_5-C_6H_4$ |
| L41 | $-C(CH_3,C_2H_5)C_2H_5$ | L79 | $3-C_2H_5-C_6H_4$ |
| L42 | $-C(CH_3)_2C_3H_7$ | L80 | $4-C_2H_5-C_6H_4$ |
| L43 | $-C(CH_3)_2cycloC_6H_{11}$ | L81 | 3-tert.-$C_4H_9-C_6H_4$ |
| L44 | $-CH_2-C(CH_3)=CH_2$ | L82 | 4-tert.-$C_4H_9-C_6H_4$ |
| L45 | $-CH_2CH=CHCH_3$ | L83 | $2,3-(CH_3)_2-C_6H_3$ |
| L46 | $-CH(CH_3)CH=CHCH_3$ | L84 | $2,4-(CH_3)_2-C_6H_3$ |
| L47 | $-C(CH_3)_2CH=CHCH_3$ | L85 | $2,5-(CH_3)_2-C_6H_3$ |
| L48 | $-CH_2C{\equiv}CH$ | L86 | $2,6-(CH_3)_2-C_6H_3$ |
| L49 | $-CH(CH_3)C{\equiv}CH$ | L87 | $3,4-(CH_3)_2-C_6H_3$ |
| L50 | $-C(CH_3)_2C{\equiv}CH$ | L88 | $3,5-(CH_3)_2-C_6H_3$ |
| L51 | $-C(CH_3,C_2H_5)C{\equiv}CH$ | L89 | $2,3,4-(CH_3)_3-C_6H_2$ |
| L52 | $-C(C_2H_5)_2C{\equiv}CH$ | L90 | $2,3,5-(CH_3)_3-C_6H_2$ |
| L53 | $-CH_2C{\equiv}CCH_3$ | L91 | $2,4,5-(CH_3)_3-C_6H_2$ |
| L54 | $-CH(CH_3)C{\equiv}CCH_3$ | L92 | $2,4,6-(CH_3)_3-C_6H_2$ |
| L55 | $-C(CH_3)_2C{\equiv}CCH_3$ | L93 | $3,4,5-(CH_3)_3-C_6H_2$ |
| L56 | $-CH_2C_6H_5$ | L94 | $2-CF_3-C_6H_4$ |
| L57 | $-CH(CH_3)C_6H_5$ | L95 | $3-CF_3-C_6H_4$ |
| L58 | $-C(CH_3)_2C_6H_5$ | L96 | $4-CF_3-C_6H_4$ |
| L59 | $-CH_2CH_2C_6H_5$ | L97 | $2-F-C_6H_4$ |
| L60 | $-CH_2CH_2SCH_3$ | L98 | $3-F-C_6H_4$ |
| L61 | $-CH(CH_3)CH_2SCH_3$ | L99 | $4-F-C_6H_4$ |
| L62 | $-C(CH_3)_2CH_2SCH_3$ | L100 | $2-Cl-C_6H_4$ |
| L63 | $-CH_2CH_2CH_2SCH_3$ | L101 | $3-Cl-C_6H_4$ |
| L64 | $-CH_2CH_2Cl$ | L102 | $4-Cl-C_6H_4$ |
| L65 | $-CH(CH_3)CH_2Cl$ | L103 | $2-Br-C_6H_4$ |
| L66 | $-C(CH_3)_2CH_2Cl$ | L104 | $3-Br-C_6H_4$ |
| L67 | $-CH_2CH_2OCH_3$ | L105 | $4-Br-C_6H_4$ |
| L68 | $-CH(CH_3)CH_2OCH_3$ | L106 | $2,3-F_2-C_6H_3$ |
| L69 | $-C(CH_3)_2CH_2OCH_3$ | L107 | $2,4-F_2-C_6H_3$ |
| L70 | $-CH_2CH_2N(CH_3)_2$ | L108 | $2,5-F_2-C_6H_3$ |
| L71 | $-CH_2CH_2N(C_2H_5)_2$ | L109 | $2,6-F_2-C_6H_3$ |
| | | L110 | $2,3-Cl_2-C_6H_3$ |

| Verb. Nr. | $R^4$ | Verb. Nr. | $R^4$ |
|---|---|---|---|
| L111 | $2,4\text{-}Cl_2\text{-}C_6H_3$ | L149 | $3\text{-}SCH_3\text{-}C_6H_4$ |
| L112 | $2,5\text{-}Cl_2\text{-}C_6H_3$ | L150 | $4\text{-}SCH_3\text{-}C_6H_4$ |
| L113 | $2,6\text{-}Cl_2\text{-}C_6H_3$ | L151 | $2\text{-}SC_2H_5\text{-}C_6H_4$ |
| L114 | $3,4\text{-}Cl_2\text{-}C_6H_3$ | L152 | $3\text{-}SC_2H_5\text{-}C_6H_4$ |
| L115 | $3,5\text{-}Cl_2\text{-}C_6H_3$ | L153 | $4\text{-}SC_2H_5\text{-}C_6H_4$ |
| L116 | $2,3,4\text{-}Cl_3\text{-}C_6H_2$ | L154 | $2\text{-}S\text{-}i\text{-}C_3H_7\text{-}C_6H_4$ |
| L117 | $2,3,5\text{-}Cl_3\text{-}C_6H_2$ | L155 | $3\text{-}S\text{-}i\text{-}C_3H_7\text{-}C_6H_4$ |
| L118 | $2,4,6\text{-}Cl_3\text{-}C_6H_2$ | L156 | $4\text{-}S\text{-}i\text{-}C_3H_7\text{-}C_6H_4$ |
| L119 | $3,4,5\text{-}Cl_3\text{-}C_6H_2$ | L157 | $2,4\text{-}(SCH_3)_2\text{-}C_6H_3$ |
| L120 | $2\text{-}CN\text{-}C_6H_4$ | L158 | $2\text{-}SCF_3\text{-}C_6H_4$ |
| L121 | $3\text{-}CN\text{-}C_6H_4$ | L159 | $3\text{-}SCF_3\text{-}C_6H_4$ |
| L122 | $4\text{-}CN\text{-}C_6H_4$ | L160 | $4\text{-}SCF_3\text{-}C_6H_4$ |
| L123 | $2\text{-}OCH_3\text{-}C_6H_4$ | L161 | $2\text{-}NO_2\text{-}C_6H_4$ |
| L124 | $3\text{-}OCH_3\text{-}C_6H_4$ | L162 | $3\text{-}NO_2\text{-}C_6H_4$ |
| L125 | $4\text{-}OCH_3\text{-}C_6H_4$ | L163 | $4\text{-}NO_2\text{-}C_6H_4$ |
| L126 | $2\text{-}OC_2H_5\text{-}C_6H_4$ | L164 | $2,3\text{-}(NO_2)_2\text{-}C_6H_3$ |
| L127 | $3\text{-}OC_2H_5\text{-}C_6H_4$ | L165 | $2,4\text{-}(NO_2)_2\text{-}C_6H_3$ |
| L128 | $4\text{-}OC_2H_5\text{-}C_6H_4$ | L166 | $2,5\text{-}(NO_2)_2\text{-}C_6H_3$ |
| L129 | $2\text{-}O\text{-}n\text{-}C_3H_7\text{-}C_6H_4$ | L167 | $2,6\text{-}(NO_2)_2\text{-}C_6H_3$ |
| L130 | $3\text{-}O\text{-}n\text{-}C_3H_7\text{-}C_6H_4$ | L168 | $3,4\text{-}(NO_2)_2\text{-}C_6H_3$ |
| L131 | $4\text{-}O\text{-}n\text{-}C_3H_7\text{-}C_6H_4$ | L169 | $3,5\text{-}(NO_2)_2\text{-}C_6H_3$ |
| L132 | $2\text{-}O\text{-}i\text{-}C_3H_7\text{-}C_6H_4$ | L170 | $2\text{-}CHO\text{-}C_6H_4$ |
| L133 | $3\text{-}O\text{-}i\text{-}C_3H_7\text{-}C_6H_4$ | L171 | $3\text{-}CHO\text{-}C_6H_4$ |
| L134 | $4\text{-}O\text{-}i\text{-}C_3H_7\text{-}C_6H_4$ | L172 | $4\text{-}CHO\text{-}C_6H_4$ |
| L135 | $2,3\text{-}(OCH_3)_2\text{-}C_6H_3$ | L173 | $2\text{-}\underset{\overset{\|}{O}}{C}CH_3\text{-}C_6H_4$ |
| L136 | $2,4\text{-}(OCH_3)_2\text{-}C_6H_3$ | | |
| L137 | $2,5\text{-}(OCH_3)_2\text{-}C_6H_3$ | L174 | $3\text{-}\underset{\overset{\|}{O}}{C}CH_3\text{-}C_6H_4$ |
| L138 | $2,6\text{-}(OCH_3)_2\text{-}C_6H_3$ | | |
| L139 | $3,4\text{-}(OCH_3)_2\text{-}C_6H_3$ | | |
| L140 | $3,5\text{-}(OCH_3)_2\text{-}C_6H_3$ | L175 | $4\text{-}\underset{\overset{\|}{O}}{C}CH_3\text{-}C_6H_4$ |
| L141 | $3,4,5\text{-}(OCH_3)_3\text{-}C_6H_2$ | | |
| L142 | $2\text{-}OCF_3\text{-}C_6H_4$ | L176 | $2\text{-}\underset{\overset{\|}{O}}{C}C_2H_5\text{-}C_6H_4$ |
| L143 | $3\text{-}OCF_3\text{-}C_6H_4$ | | |
| L144 | $4\text{-}OCF_3\text{-}C_6H_4$ | | |
| L145 | $2\text{-}OCF_2CHF_2\text{-}C_6H_4$ | L177 | $3\text{-}\underset{\overset{\|}{O}}{C}C_2H_5\text{-}C_6H_4$ |
| L146 | $3\text{-}OCF_2CHF_2\text{-}C_6H_4$ | | |
| L147 | $4\text{-}OCF_3CHF_2\text{-}C_6H_4$ | L178 | $4\text{-}\underset{\overset{\|}{O}}{C}C_2H_5\text{-}C_6H_4$ |
| L148 | $2\text{-}SCH_3\text{-}C_6H_4$ | | |

| Verb. Nr. | $R^4$ |
|---|---|
| L179 | $2\text{-}C(=O)\text{-}n\text{-}C_3H_7\text{-}C_6H_4$ |
| L180 | $3\text{-}C(=O)\text{-}n\text{-}C_3H_7\text{-}C_6H_4$ |
| L181 | $4\text{-}C(=O)\text{-}n\text{-}C_3H_7\text{-}C_6H_4$ |
| L182 | $2\text{-}C(=O)CF_3\text{-}C_6H_4$ |
| L183 | $3\text{-}C(=O)CF_3\text{-}C_6H_4$ |
| L184 | $4\text{-}C(=O)CF_3\text{-}C_6H_4$ |
| L185 | 1-Naphthyl |
| L186 | 2-Naphthyl |
| L187 | $C_6H_5$ |
| L188 | Piperidino |
| L189 | Tetrahydrofur-3-yl |
| L190 | Thiazol-2-yl |
| L191 | 5-Methyl-thiazol-2-yl |
| L192 | 5-Ethyl-thiazol-2-yl |
| L193 | 5-n-Propyl-thiazol-2-yl |
| L194 | 4-Methyl-5-carboxy-thiazol-2-yl |
| L195 | Cyclopropylmethyl |

Die Verbindungen Ia bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Verbindungen Ia eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-,

Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:

I. Man vermischt 90 Gew.-Teile der Verbindung Nr. 1006 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1006 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1022 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion, die 0,02 Gew.% des Wirkstoffs enthält.

IV. 20 Gew.-Teile der Verbindung Nr. 1049 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion, die 0,02 Gew.% des Wirkstoffs enthält.

V. 20 Gew.-Teile des Wirtkstoffs Nr. 3038 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

VI. 3 Gew.-Teile der Verbindung Nr. 1075 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile des Wirkstoffs Nr. 1220 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gew.-Teile des Wirkstoffs Nr. 1026 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenol-Formaldehyd-Kondensats und 68 Gew.-Teilen eines paraffinischen Mineralöls innig vermischt. Man erhält eine stabile ölige Dispersion.

Die Formulierungen enthalten zwischen 0,1 und 95 Gew.%, vorzugsweise zwischen 0,5 und 90 Gew.%, Wirkstoff.

Die Applikation der herbiziden Mittel bzw. der Wirkstoffe kann im Vorauflauf-oder vorzugsweise im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5, vorzugsweise 0,01 bis 1 kg/ha.

In Anbetracht des erfaßbaren Wirkungsspektrums zur Bekämpfung der unerwünschten Pflanzen der Verträglichkeit für Kulturpflanzen oder der erwünschten Beeinflussung des Wachstums derselben sowie angesichts der Vielfalt der Applikationsmethoden können die Verbindungen der Formel Ia sowie die sie enthaltenden herbiziden Mittel in einer großen Zahl von Kulturpflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum, Gossypium herbaceum,Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |

| Botanischer Name | Deutscher Name |
|---|---|
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |

| Botanischer Name | Deutscher Name |
|---|---|
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Isoxazol-(Isothiazol)-5-carbonsäureamide der Formel Ia mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäuren, Sulfonylharnstoffe, Imidazolinone, (Hetero)Aryloxyphenoxypropionsäuren, deren Salze, Ester und Amide und andere Wirkstoffe in Betracht.

Außerdem kann es von Nutzen sein, die Verbindungen der Formel Ia allein oder in Kombination mit anderen Herbiziden auch noch mit weiteren Pflanzenschutzmitteln gemischt gemeinsam zubringen, beispielsweise mit Mitteln zur Bekämpfung von Schädlingen oder phytopatogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate hergestellt werden.

Anwendungsbeispiele

Die Wirkung der Isoxazol(Isothiazol)-5-carbonsäureamide der Formel Ia auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3 % Humus als Substrat. Die Samen der Testpflanzen werden nach Arten getrennt flach eingesät.

Bei Vorauflaufbehandlung werden die aufbereiteten Wirkstoffe unmittelbar danach auf die Erdoberfläche aufgebracht. Sie werden hierzu in Wasser als Verteilungsmittel suspendiert oder emulgiert und mittels fein verteilender Düsen gespritzt. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen auflaufen. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung werden entweder direkt gesäte oder in den gleichen Gefäßen aufgewachsene Pflanzen ausgewählt oder die Pflanzen werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 1,0 und 3,0 kg/ha a.S.

Die Versuchsgefäße werden dann im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Abutilon theophrasti | Chinesischer Hanf |
| Centaurea cyanus | Kornblume |
| Chenopodium album | Weißer Gänsefuß |
| Chrysanthemum coronarium | Kranenwucherblume |
| Echinochloa crus-galli | Hühnerhirse |
| Galium aparine | Klettenlabkraut |
| Ipomoea ssp. | Prunkwindearten |
| Lolium multiflorum | Ital. Raygras |
| Mentha piperita | Pfefferminze |
| Mercurialis annua | einj. Bingelkraut |
| Solanum nigrum | Schwarzer Nachtschatten |
| Triticum aestivum | Sommerweizen |
| Viola spp. | Stiefmütterchen |
| Zea mays | Mais |

Der beispielhaft ausgewählte Wirkstoff Nr. 1006 bekämpft unerwünschte Pflanzen im Vorauflaufverfahren bei einer Aufwandmenge von 3,0 kg/ha sehr gut.

Bei Nachauflaufanwendung zeigen u.a. die Wirkstoffe Nr. 1004, 1014, 3018, 1026, 1006, 1049 und 1022 bei 1 bis 3 kg/ha Aufwandmenge herbizide Wirkung gegen ein breites Spektrum unerwünschter Pflanzen. Außerdem zeigen die Verbindungen Nr. 1075 und 3038 eine sehr gute herbizide Wirkung an breitblättrigen Pflanzen bei gleichzeitiger Selektivität in Mais. Die Verbindung Nr. 1211 zeigt neben ausgezeichneter herbizider Aktivität Selektivität in Weizen.

**Patentansprüche**

**Patentansprüche für folgende Vertragsstaaten : BE, CH, DE, FR, GB, IT, LI, NL**

**1.** Isoxazol(Isothiazol)-5-carbonsäureamide der Formel

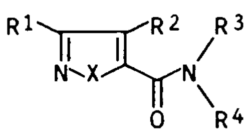

(I),

in der

x Sauerstoff oder Schwefel,

$R^1$ Wasserstoff,
gegebenenfalls durch $C_1$-$C_3$-Alkoxy, $C_1$-$C_3$-Halogenalkoxy, Halogen, Cyano oder Phenyl, das durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Cyano oder Nitro substituiert sein kann, substituiertes $C_1$-$C_{10}$-Alkyl,
$C_1$-$C_4$-Alkoxy,
gegebenenfalls durch $C_1$-$C_4$-Alkyl oder Halogen substituiertes $C_3$-$C_8$-Cycloalkyl,
einen 5- bis 6-gliedrigen heterocyclischen Rest mit einem oder zwei Heteroatomen, ausgewählt aus der Gruppe Sauerstoff, Schwefel und Stickstoff, der durch $C_1$-$C_4$-Alkyl, Carboxyl oder $C_1$-$C_4$-Alkoxycarbonyl substituiert sein kann,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes Phenyl,

$R^2$ Formyl, 4,5-Dihydro-oxazol-2-yl oder einen Rest der Formel $COYR^5$ oder $CONR^6R^7$,

wobei

Y für Sauerstoff oder Schwefel,

$R^5$ für Wasserstoff,
$C_1$-$C_8$-Alkyl, das durch $C_1$-$C_4$-Alkoxy, $C_1$-$C_4$-Alkoxy-$C_1$-$C_4$-alkoxy, Halogen, Cyano, Hydroxy, Trimethylsilyl, $C_1$-$C_4$-Alkylthio, $C_1$-$C_4$-Alkylamino, $C_1$-$C_4$-Dialkylamino, $C_1$-

$C_4$-Alkylsulfinyl, $C_1$-$C_4$-Alkylsulfonyl, Carboxyl, $C_1$-$C_4$-Alkoxycarbonyl, $C_1$-$C_4$-Dialkylaminocarbonyl, $C_1$-$C_4$-Dialkoxyphosphonyl, Alkaniminoxy, Benzyloxy, gegebenenfalls durch $C_1$-$C_4$-Alkyl, $C_1$-$C_3$-Alkoxy oder Halogen substituiertes Benzoyl oder gegebenenfalls durch $C_1$-$C_4$-Alkyl, $C_1$-$C_4$-Alkoxy, $C_1$-$C_4$-Halogenalkyl, Halogen, Nitro oder Cyano substituiertes Phenyl, durch Thienyl, Furyl, Tetrahydrofuryl, Phthalimido oder Pyridyl substituiert sein kann,
gegebenenfalls durch
Phenyl, das durch $C_1$-$C_4$-Alkyl, $C_1$-$C_4$-Alkoxy, $C_1$-$C_4$-Halogenalkyl, Halogen, Nitro oder Cyano substituiert sein kann, substituiertes $C_3$-$C_8$-Alkenyl,
$C_3$-$6_6$-Halogenalkenyl,
gegebenenfalls durch Hydroxy oder $C_1$-$C_4$-Alkoxy substituiertes $C_3$-$C_8$-Alkinyl,
$C_3$-$C_6$-Cycloalkyl,
$C_5$-$C_6$-Cycloalkenyl,
gegebenenfalls durch $C_1$-$C_4$-Alkyl, $C_1$-$C_4$-Alkoxy,
$C_1$-$C_4$-Halogenalkyl, Halogen, Nitro, Cyano,
$C_1$-$C_4$-Alkoxycarbonyl oder Acylamino substituiertes Phenyl, einen 5- bis 6-gliedrigen heterocyclischen Rest mit einem oder zwei Heteroatomen, ausgewählt aus der Gruppe Sauerstoff, Schwefel und Stickstoff, oder einen Benzotriazolrest
$C_6$-$C_7$-Cycloalkanimino, Phthalimido, Succinimido, für die Reste

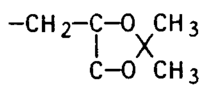 , 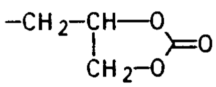 ,

$-CH_2-CH(OH)-CH_2(OH)$ ,
für ein Äquivalent eines Kations aus der Gruppe der Alkali-, Erdalkalimetalle, Mangan, Kupfer, Eisen, Ammonium und substituiertes Ammonium oder für den Rest

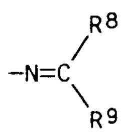 ,

wobei $R^8$ und $R^9$ unabhängig voneinander $C_1$-$C_4$-Alkyl, $C_2$-$C_6$-Alkoxyalkyl, $C_3$-$C_6$-Cycloalkyl, Phenyl, Furyl oder zusammen eine Methylenkette der Formel $-(CH_2)_m-$ mit m = 4 bis 7 Kettengliedern bedeuten und $R^9$ zusätzlich Wasserstoff bedeutet,

$R^6$ für Wasserstoff, $C_1$-$C_8$-Alkyl oder $C_3$-$C_8$-Cycloalkyl und
$R^7$ für Wasserstoff oder $C_1$-$C_8$-Alkyl stehen oder wobei
$R^6$ und $R^7$ eine Methylenkette mit 4 oder 5 Gliedern bilden,
$R^3$ Wasserstoff,
gegebenenfalls durch Hydroxy, Halogen, $C_1$-$C_4$-Alkoxy, $C_1$-$C_4$-Alkylthio oder $C_1$-$C_4$-Dialkylamino substituiertes $C_1$-$C_8$-Alkyl oder gegebenenfalls durch $C_1$-$C_4$-Alkyl, Halogen oder $C_1$-$C_4$-Halogenalkyl substituiertes $C_3$-$C_8$-Cycloalkyl und
$R^4$ Wasserstoff, Hydroxyl, $C_1$-$C_4$-Alkoxy,
gegebenenfalls durch $C_1$-$C_4$-Alkoxy, $C_1$-$C_4$-Halogenalkoxy, $C_1$-$C_4$-Alkylthio, $C_1$-$C_4$-Dialkylamino, Halogen, $C_3$-$C_6$-Cycloalkyl oder Phenyl, das durch Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio substituiert sein kann, substituiertes $C_1$-$C_{10}$-Alkyl,
gegebenenfalls durch Halogen oder $C_1$-$C_4$-Alkoxy substituiertes $C_3$-$C_{10}$-Alkenyl oder $C_3$-$C_{10}$-Alkinyl,
gegebenenfalls durch $C_1$-$C_6$-Alkyl, $C_1$-$C_6$-Halogenalkyl,
$C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes $C_3$-$C_8$-Cycloalkyl,
$C_1$-$C_4$-Dialkylamino,
einen gegebenenfalls durch $C_1$-$C_6$-Alkyl, $C_1$-$C_6$-Halogenalkyl oder Halogen substitu-

ierten 3- bis 6-gliedrigen heterocyclischen Rest mit einem oder zwei Heteroatomen, ausgewählt aus der Gruppe Sauerstoff, Schwefel und gegebenenfalls durch Methyl substituierter Stickstoff, Naphthyl,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro, Cyano, Formyl, $C_1$-$C_6$-Alkanoyl oder $C_1$-$C_6$-Halogenalkanoyl substituiertes Phenyl bedeuten

oder

$R^3$ und $R^4$ gemeinsam einen Rest der Struktur $-(CH_2)_n^-\ Y_p-(CH_2)_q^-$, wobei n und q 1, 2 oder 3, p 0 oder 1 und Y Sauerstoff, Schwefel oder N-Methyl bedeuten, oder den Rest der Formel

$-(CH_2)_3-CO-$

bilden,

sowie deren umweltverträglichen Salze
mit der Maßgabe, daß im Fall von x = Sauerstoff $R^1$ nicht $CH_3$ bedeutet, wenn $R^2$ eine Gruppe COOH, $COOC_2H_5$ oder $CONH_2$ und $R^3$ und $R^4$ beide Wasserstoff bedeuten; und ferner $R^1$ nicht Phenyl bedeutet, wenn $R^2$ für $COOCH_3$ und $R^3$ und beide Wasserstoff bedeuten und daß im Fall von X = Schwefel $R^2$ keine Gruppe COOH oder $CONH_2$ darstellt, wenn $R^1$, $R^3$ und $R^4$ Wasserstoff bedeuten.

**2.** Isoxazol(Isothiazol)-5-carbonsäureamide der Formel I gemäß Anspruch 1, dadurch gekennzeichnet, daß $R^3$ Wasserstoff bedeutet.

**3.** Isoxazol(Isothiazol)-5-carbonsäureamide der Formel I gemäß Anspruch 1, dadurch gekennzeichnet, daß
$R^1$ Wasserstoff oder $C_1$-$C_4$-Alkyl,
$R^2$ $COYR^5$, wobei Y für Sauerstoff oder Schwefel und $R^5$ für Wasserstoff, $C_1$-$C_4$-Alkyl, gegebenenfalls durch $C_1$-$C_4$-Alkyl, $C_1$-$C_4$-Halogenalkyl, $C_1$-$C_4$-Alkoxy, $C_1$-$C_4$-Alkylthio oder Halogen substituiertes Phenyl oder den Rest

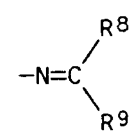

stehen,
$R^3$ Wasserstoff und
$R^4$ $C_1$-$C_4$-Alkyl oder $C_3$-$C_8$-Cycloalkyl bedeuten.

**4.** Isoxazol-5-carbonsäureamid der Formel I gemäß Anspruch 1, dadurch gekennzeichnet, daß $R^1$ n-Propyl, $R^2$ Propan-2-iminoxycarbonyl, $R^3$ Wasserstoff und $R^4$ tert.-Butyl bedeuten.

**5.** Herbizides Mittel, enthaltend neben inerten Zusatzstoffen ein Isoxazol(Isothiazol)-5-carbonsäureamid der Formel

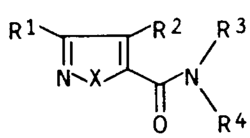

(I)

in der
X Sauerstoff oder Schwefel,
$R^1$ Wasserstoff,
gegebenenfalls durch $C_1$-$C_3$-Alkoxy, $C_1$-$C_3$-Halogenalkoxy, Halogen, Cyano oder Phenyl, das durch Halogen, $C_1$-$C_4$-Alkyl, $C_1$-$C_4$-Halogenalkyl, $C_1$-$C_4$-Alkoxy, $C_1$-$C_4$-Halo-

genalkoxy, $C_1$-$C_4$-Alkylthio, $C_1$-$C_4$-Halogenalkylthio, Cyano oder Nitro substituiert sein kann, substituiertes $C_1$-$C_{10}$-Alkyl,
$C_1$-$C_4$-Alkoxy,
gegebenenfalls durch $C_1$-$C_4$-Alkyl oder Halogen substituiertes $C_3$-$C_8$-Cycloalkyl,
einen 5- bis 6-gliedrigen heterocyclischen Rest mit einem oder zwei Heteroatomen, ausgewählt aus der Gruppe Sauerstoff, Schwefel und Stickstoff, der durch $C_1$-$C_4$-Alkyl, Carboxyl oder $C_1$-$C_4$-Alkoxycarbonyl substituiert sein kann,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes Phenyl,

$R^2$ Formyl, 4,5-Dihydro-oxazol-2-yl oder einen Rest der Formel $COYR^5$ oder $CONR^6R^7$

wobei

Y für Sauerstoff oder Schwefel,

$R^5$ für Wasserstoff,
$C_1$-$C_8$-Alkyl, das durch $C_1$-$C_4$-Alkoxy,
$C_1$-$C_4$-Alkoxy-$C_1$-$C_4$-alkoxy, Halogen, Cyano, Hydroxy, Trimethylsilyl, $C_1$-$C_4$-Alkylthio, $C_1$-$C_4$-Alkylamino, $C_1$-$C_4$-Dialkylamino, $C_1$-$C_4$-Alkylsulfinyl, $C_1$-$C_4$-Alkylsulfonyl, Carboxyl, $C_1$-$C_4$-Alkoxycarbonyl, $C_1$-$C_4$-Dialkylaminocarbonyl, $C_1$-$C_4$-Dialkoxyphosphonyl, Alkaniminoxy, Benzyloxy, gegebenenfalls durch $C_1$-$C_4$-Alkyl, $C_1$-$C_3$-Alkoxy oder Halogen substituiertes Benzoyl oder gegebenenfalls durch $C_1$-$C_4$-Alkyl, $C_1$-$C_4$-Alkoxy, $C_1$-$C_4$-Halogenalkyl, Halogen, Nitro oder Cyano substituiertes Phenyl, durch Thienyl, Furyl, Tetrahydrofuryl, Phthalimido oder Pyridyl substituiert sein kann,
gegebenenfalls durch
Phenyl, das durch $C_1$-$C_4$-Alkyl, $C_1$-$C_4$-Alkoxy, $C_1$-$C_4$-Halogenalkyl, Halogen, Nitro oder Cyano substituiert sein kann, substituiertes $C_3$-$C_8$-Alkenyl,
$C_3$-$C_6$-Halogenalkenyl,
gegebenenfalls durch Hydroxy oder $C_1$-$C_4$-Alkoxy substituiertes $C_3$-$C_8$-Alkinyl,
$C_3$-$C_6$-Cycloalkyl,
$C_5$-$C_6$-Cycloalkenyl, gegebenenfalls durch $C_1$-$C_4$-Alkyl, $C_1$-$C_4$-Alkoxy, $C_1$-$C_4$-Halogenalkyl, Halogen, Nitro, Cyano, $C_1$-$C_4$-Alkoxycarbonyl oder Acylamino substituiertes Phenyl, einen 5- bis 6-gliedrigen heterocyclischen Rest mit einem oder zwei Heteroatomen, ausgewählt aus der Gruppe Sauerstoff, Schwefel und Stickstoff, oder einen Benzotriazolrest,
$C_6$-$C_7$-Cycloalkanimino, Phthalimido, Succinimido, für die Reste

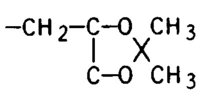
,
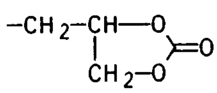
,

$-CH_2-CH(OH)-CH_2(OH)$,
für ein Äquivalent eines Kations aus der Gruppe der Alkali-, Erdalkalimetalle, Hangan, Kupfer, Eisen, Ammonium und substituiertes Ammonium oder für den Rest

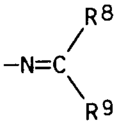
,

wobei $R^8$ und $R^9$ unabhängig voneinander $C_1$-$C_4$-Alkyl, $C_2$-$C_6$-Alkoxyalkyl, $C_3$-$C_6$-Cycloalkyl, Phenyl, Furyl oder zusammen eine Methylenkette der Formel $-(CH_2)_m-$ mit m = 4 bis 7 Kettengliedern bedeuten und $R^9$ zusätzlich Wasserstoff bedeutet,

$R^6$ für Wasserstoff, $C_1$-$C_8$-Alkyl oder $C_3$-$C_8$-Cycloalkyl und

$R^7$ für Wasserstoff oder $C_1$-$C_8$-Alkyl stehen oder wobei

$R^6$ und $R^7$ eine Methylenkette mit 4 oder 5 Gliedern bilden,

$R^3$ Wasserstoff,

gegebenenfalls durch Hydroxy, Halogen, $C_1$-$C_4$-Alkoxy, $C_1$-$C_4$-Alkylthio oder $C_1$-$C_4$-Dialkylamino substituiertes $C_1$-$C_8$-Alkyl oder gegebenenfalls durch $C_1$-$C_4$-Alkyl, Halogen oder $C_1$-$C_4$-Halogenalkyl substituiertes $C_3$-$C_8$-Cycloalkyl und

$R^4$ Wasserstoff, Hydroxyl,
gegebenenfalls durch $C_1$-$C_4$-Alkoxy, $C_1$-$C_4$-Halogenalkoxy, $C_1$-$C_4$-Alkylthio, $C_1$-$C_4$-Dialkylamino, Halogen, $C_3C_6$-Cycloalkyl oder Phenyl, das durch Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio substituiert sein kann, substituiertes $C_1$-$C_{10}$-Alkyl,
gegebenenfalls durch Halogen oder $C_1$-$C_4$-Alkoxy substituiertes $C_3$-$C_{10}$-Alkenyl oder $C_3$-$C_{10}$-Alkinyl,
gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes $C_3$-$C_8$-Cycloalkyl,
$C_1$-$C_4$-Dialkylamino,
einen gegebenenfalls durch $C_1$-$C_6$-Alkyl, $C_1$-$C_6$-Halogenalkyl oder Halogen substituierten 3- bis 6-gliedrigen heterocyclischen Rest mit einem oder zwei Heteroatomen, ausgewählt aus der Gruppe Sauerstoff, Schwefel und gegebenenfalls durch Methyl substituierter Stickstoff,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro, Cyano, Formyl, $C_1$-$C_6$-Alkanoyl oder $C_1$-$C_6$-Halogenalkanoyl substituiertes Phenyl bedeuten

oder

$R^3$ und $R^4$ gemeinsam einen Rest der Struktur $-(CH_2)_n^-\ Y_p-(CH_2)_q^-$, wobei n und q 1, 2 oder 3, p 0 oder 1 und Y Sauerstoff, Schwefel oder N-Methyl bedeuten, oder den Rest der Formel

$-(CH_2)_3-CO-$

bilden sowie deren umweltverträglichen Salze, mit der Maßgabe, daß im Fall von X = Sauerstoff $R^1$ nicht $CH_3$ bedeutet, wenn $R^2$ eine Gruppe COOH, $COOC_2H_5$ oder $CONH_2$ und $R^3$ und $R^4$ beide Wasserstoff bedeuten; und ferner $R^1$ nicht Phenyl bedeutet, wenn $R^2$ für $COOCH_3$ und $R^3$ und beide Wasserstoff bedeuten und daR im Fall von X = Schwefel $R^2$ keine Gruppe COOH oder $CONH_2$ darstellt, wenn $R^1$, $R^3$ und $R^4$ Wasserstoff bedeuten.

**6.** Herbizides Mittel gemäß Anspruch 5, dadurch gekennzeichnet, daß es als Wirkstoff ein Isoxazol-(Isothiazol)-5-carbonsäureamid der Formel I enthält, in der $R^3$ Wasserstoff bedeutet.

**7.** Herbizides Mittel gemäß Anspruch 5, dadurch gekennzeichnet, daß es als Wirkstoff ein Isoxazol-(Isothiazol)-5-carbonsäureamid der Formel I enthält, in der
$R^1$ Wasserstoff oder $C_1$-$C_4$-Alkyl,
$R^2$ $COYR^5$, wobei Y für Sauerstoff oder Schwefel und $R^5$ für Wasserstoff, $C_1$-$C_4$-Alkyl, gegebenenfalls durch $C_1$-$C_4$-Alkyl, $C_1$-$C_4$-Halogenalkyl $C_1$-$C_4$-Alkoxy, $C_1$-$C_4$-Alkylthio oder Halogen substituiertes Phenyl oder den Rest

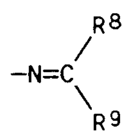

stehen,
$R^3$ Wasserstoff und
$R^4$ $C_1$-$C_4$-Alkyl oder $C_3$-$C_8$-Cycloalkyl bedeuten.

**8.** Verfahren zur Bekämpfung unerwünschten Pflanzenwuchses, dadurch gekennzeichnet, daß man die unerwünschten Pflanzen und/oder die von unerwünschten Pflanzenwuchs freizuhaltende Fläche mit einer herbizid wirksamen Menge eines Isoxazol(Isothiazol)-5-carbonsäureamids der Formel Ia

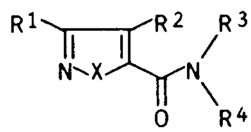

(Ia)

in der

X Sauerstoff oder Schwefel,

$R^1$ Wasserstoff,
gegebenenfalls durch $C_1$-$C_3$-Alkoxy, $C_1$-$C_3$-Halogenalkoxy, Halogen, Cyano oder Phenyl, das durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Cyano oder Nitro substituiert sein kann, substituiertes $C_1$-$C_{10}$-Alkyl,
$C_1$-$C_4$-Alkoxy,
gegebenenfalls durch $C_1$-$C_4$-Alkyl oder Halogen substituiertes $C_3$-$C_8$-Cycloalkyl,
einen 5- bis 6-gliedrigen heterocyclischen Rest mit einem oder zwei Heteroatomen, ausgewählt aus der Gruppe Sauerstoff, Schwefel und Stickstoff, der durch $C_1$-$C_4$-Alkyl, Carboxyl oder $C_1$-$C_4$-Alkoxycarbonyl substituiert sein kann,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes Phenyl,

$R^2$ Formyl, 4,5-Dihydro-oxazol-2-yl oder einen Rest der Formel $COYR^5$ oder $CONR^6R^7$

wobei

Y für Sauerstoff oder Schwefel,

$R^5$ für Wasserstoff,
$C_1$-$C_8$-Alkyl, das durch $C_1$-$C_4$-Alkoxy, $C_1$-$C_4$-Alkoxy-$C_1$-$C_4$-alkoxy, Halogen, Cyano, Hydroxy, Trimethylsilyl, $C_1$-$C_4$-Alkylthio, $C_1$-$C_4$-Alkylamino, $C_1$-$C_4$-Dialkylamino, $C_1$-$C_4$-Alkylsulfinyl, $C_1$-$C_4$-Alkylsulfonyl, Carboxyl, $C_1$-$C_4$-Alkoxycarbonyl, $C_1$-$C_4$-Dialkylaminocarbonyl, $C_1$-$C_4$-Dialkoxyphosphonyl, Alkaniminoxy, Benzyloxy, gegebenenfalls durch $C_1$-$C_4$-Alkyl, $C_1$-$C_3$-Alkoxy oder Halogen substituiertes Benzoyl oder gegebenenfalls durch $C_1$-$C_4$-Alkyl, $C_1$-$C_4$-Alkoxy, $C_1$-$C_4$-Halogenalkyl, Halogen, Nitro oder Cyano substituiertes Phenyl, durch Thienyl, Furyl, Tetrahydrofuryl, Phthalimido oder Pyridyl substituiert sein kann,
gegebenenfalls durch
Phenyl, das durch $C_1$-$C_4$-Alkyl, $C_1$-$C_4$-Alkoxy, $C_1$-$C_4$-Halogenalkyl, Halogen, Nitro oder Cyano substituiert sein kann, substituiertes $C_3$-$C_8$-Alkenyl,
$C_3$-$C_6$-Halogenalkenyl,
gegebenenfalls durch Hydroxy oder $C_1$-$C_4$-Alkoxy substituiertes $C_3$-$C_8$-Alkinyl,
$C_3$-$C_6$-Cycloalkyl,
$C_5$-$C_6$-Cycloalkenyl, gegebenenfalls durch $C_1$-$C_4$-Alkyl, $C_1$-$C_4$-Alkoxy, $C_1$-$C_4$-Halogenalkyl, Halogen, Nitro, Cyano, $C_1$-$C_4$-Alkoxycarbonyl oder Acylamino substituiertes Phenyl, einen 5- bis 6-gliedrigen heterocyclischen Rest mit einem oder zwei Heteroatomen, ausgewählt aus der Gruppe Sauerstoff, Schwefel und Stickstoff, oder einen Benzotriazolrest,
$C_6$-$C_7$-Cycloalkanimino, Phthalimido, Succinimido, für die Reste

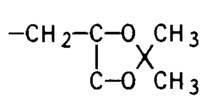 , 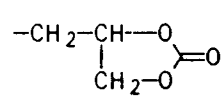 ,

$-CH_2-CH(OH)-CH_2(OH)$,
für ein Äquivalent eines Kations aus der Gruppe der Alkali-, Erdalkalimetalle, Mangan,

Kupfer, Eisen, Ammonium und substituiertes Ammonium oder für den Rest

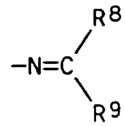
,

wobei $R^8$ und $R^9$ unabhängig voneinander $C_1$-$C_4$-Alkyl, $C_2$-$C_6$-Alkoxyalkyl, $C_3$-$C_6$-Cycloalkyl, Phenyl, Furyl oder zusammen eine Methylenkette der Formel $-(CH_2)_m-$ mit m = 4 bis 7 Kettengliedern bedeuten und $R^9$ zusätzlich Wasserstoff bedeutet,

$R^6$ für Wasserstoff, $C_1$-$C_8$-Alkyl oder $C_3$-$C_8$-Cycloalkyl und

$R^7$ für Wasserstoff oder $C_1$-$C_8$-Alkyl stehen oder wobei

$R^6$ und $R^7$ eine Methylenkette mit 4 oder 5 Gliedern bilden,

$R^3$ Wasserstoff,
gegebenenfalls durch Hydroxy, Halogen, $C_1$-$C_4$-Alkoxy, $C_1$-$C_4$-Alkylthio oder $C_1$-$C_4$-Dialkylamino substituiertes $C_1$-$C_8$-Alkyl
oder gegebenenfalls durch $C_1$-$C_4$-Alkyl, Halogen oder $C_1$-$C_4$-Halogenalkyl substituiertes $C_3$-$C_8$-Cycloalkyl und

$R^4$ Wasserstoff, Hydroxyl,
gegebenenfalls durch $C_1$-$C_4$-Alkoxy, $C_1$-$C_4$-Halogenalkoxy, $C_1$-$C_4$-Alkylthio, $C_1$-$C_4$-Dialkylamino, Halogen, $C_3$-$C_6$-Cycloalkyl oder Phenyl, das durch Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio substituiert sein kann, substituiertes $C_1$-$C_{10}$-Alkyl,
gegebenenfalls durch Halogen oder $C_1$-$C_4$-Alkoxy substituiertes $C_3$-$C_{10}$-Alkenyl oder $C_3$-$C_{10}$-Alkinyl,
gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes $C_3$-$C_8$-Cycloalkyl,
$C_1$-$C_4$-Dialkylamino,
einen gegebenenfalls durch $C_1$-$C_6$-Alkyl, $C_1$-$C_6$-Halogenalkyl oder Halogen substituierten 3- bis 6-gliedrigen heterocyclischen Rest mit einem oder zwei Heteroatomen, ausgewählt aus der Gruppe Sauerstoff, Schwefel und gegebenenfalls durch Methyl substituierter Stickstoff,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro, Cyano, Formyl, $C_1$-$C_6$-Alkanoyl oder $C_1$-$C_6$-Halogenalkanoyl substituiertes Phenyl bedeuten

oder

$R^3$ und $R^4$ gemeinsam einen Rest der Struktur $-(CH_2)_n^-\ Y_p-(CH_2)_q^-$, wobei n und q 1, 2 oder 3, p 0 oder 1 und Y Sauerstoff, Schwefel oder N-Methyl bedeuten, oder den Rest der Formel

$-(CH_2)_3-CO-$

bilden sowie deren umweltverträglichen Salzen behandelt.

**9.** Verfahren zur Herstellung von Isoxazol(Isothiazol)-5-carbonsäureamiden der Formel I gemäß Anspruch 1, dadurch gekennzeichnet, daß man ein Isoxazol(Isothioazol)-5-carbonsäurehalogenid der Formel VI

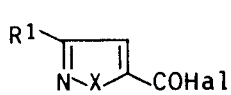
(VI)

mit einem Amin der Formel V

$HNR^3R^4$ (V)

zu einem Isoxazol(Isothiazol)amid der Formel VII

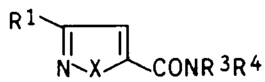

(VII)

umsetzt und dieses Amid mit Alkyllithium und Kohlendioxid zu der entsprechenden Isoxazol(Isothiazol)-4-carbonsäure der Formel Ic

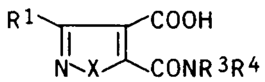

(Ic)

umsetzt und diese Säure durch Veresterung mit einem Alkohol der Formel IX

$HY-R^5$ (IX)

in Gegenwart eines wasserentziehenden Mittels in ein Isoxazol(Isothiazol)-5-carbonsäureamid der Formel I überführt.

**10.** Verfahren zur Herstellung von Isoxazol(Isothiazol)-5-carbonsäureamiden der Formel Ib

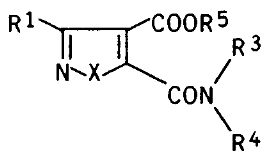

(Ib),

in der X Sauerstoff oder Schwefel bedeutet, $R^1$, $R^3$ und $R^4$ die in Anspruch 1 genannte Bedeutung haben und $R^5$ für $C_1$-$C_8$-Alkyl steht, dadurch gekennzeichnet, daß man einen Dialkylester der Formel II

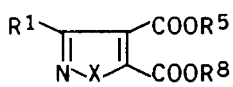

(II),

in der $R^1$, $R^5$ und X die o.g. Bedeutung haben und $R^8$ für $C_1$-$C_8$-Alkyl steht, mit etwa äquimolaren Mengen eines Amins der Formel V

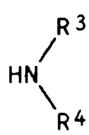

(V)

in einem organischen Lösungsmittel bei Temperaturen zwischen 0 und 100 ° C umsetzt.

**Patentansprüche für folgenden Vertragsstaat : ES**

**1.** Verfahren zur Herstellung von Isoxazol(Isothiazol)-5-carbonsäureamiden der Formel I

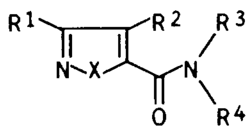 (I),

in der

| | |
|---|---|
| X | Sauerstoff oder Schwefel, |
| $R^1$ | Wasserstoff, gegebenenfalls durch $C_1$-$C_3$-Alkoxy, $C_1$-$C_3$-Halogenalkoxy, Halogen, Cyano oder Phenyl, das durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, Cyano oder Nitro substituiert sein kann, substituiertes $C_1$-$C_{10}$-Alkyl, $C_1$-$C_4$-Alkoxy, gegebenenfalls durch $C_1$-$C_4$-Alkyl oder Halogen substituiertes $C_3$-$C_8$-Cycloalkyl, einen 5- bis 6-gliedrigen heterocyclischen Rest mit einem oder zwei Heteroatomen, ausgewählt aus der Gruppe Sauerstoff, Schwefel und Stickstoff, der durch $C_1$-$C_4$-Alkyl, Carboxyl oder $C_1$-$C_4$-Alkoxycarbonyl substituiert sein kann, oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes Phenyl, |
| $R^2$ | Formyl, 4,5-Dihydro-oxazol-2-yl oder einen Rest der Formel $COYR^5$ oder $CONR^6R^7$, |

wobei

| | |
|---|---|
| Y | für Sauerstoff oder Schwefel, |
| $R^5$ | für Wasserstoff, $C_1$-$C_8$-Alkyl, das durch $C_1$-$C_4$-Alkoxy, $C_1$-$C_4$-Alkoxy-$C_1$-$C_4$-alkoxy, Halogen, Cyano, Hydroxy, Trimethylsilyl, $C_1$-$C_4$-Alkylthio, $C_1$-$C_4$-Alkylamino, $C_1$-$C_4$-Dialkylamino, $C_1$-$C_4$-Alkylsulfinyl, $C_1$-$C_4$-Alkylsulfonyl, Carboxyl, $C_1$-$C_4$-Alkoxycarbonyl, $C_1$-$C_4$-Dialkylaminocarbonyl, $C_1$-$C_4$-Dialkoxyphosphonyl, Alkaniminoxy, Benzyloxy, gegebenenfalls durch $C_1$-$C_4$-Alkyl, $C_1$-$C_3$-Alkoxy oder Halogen substituiertes Benzoyl oder gegebenenfalls durch $C_1$-$C_4$-Alkyl, $C_1$-$C_4$-Alkoxy, $C_1$-$C_4$-Halogenalkyl, Halogen, Nitro oder Cyano substituiertes Phenyl, durch Thienyl, Furyl, Tetrahydrofuryl, Phthalimido oder Pyridyl substituiert sein kann, gegebenenfalls durch Phenyl, das durch $C_1$-$C_4$-Alkyl, $C_1$-$C_4$-Alkoxy, $C_1$-$C_4$-Halogenalkyl, Halogen, Nitro oder Cyano substituiert sein kann, substituiertes $C_3$-$C_8$-Alkenyl, $C_3$-$C_6$-Halogenalkenyl, gegebenenfalls durch Hydroxy oder $C_1$-$C_4$-Alkoxy substituiertes $C_3$-$C_8$-Alkinyl, $C_3$-$C_6$-Cycloalkyl, $C_5$-$C_6$-Cycloalkenyl, gegebenenfalls durch $C_1$-$C_4$-Alkyl, $C_1$-$C_4$-Alkoxy, $C_1$-$C_4$-Halogenalkyl, Halogen, Nitro, Cyano, $C_1$-$C_4$-Alkoxycarbonyl oder Acylamino substituiertes Phenyl, einen 5- bis 6-gliedrigen heterocyclischen Rest mit einem oder zwei Heteroatomen, ausgewählt aus der Gruppe Sauerstoff, Schwefel und Stickstoff, oder einen Benzotriazolrest $C_6$-$C_7$-Cycloalkanimino, Phthalimido, Succinimido$_1$ für die Reste |

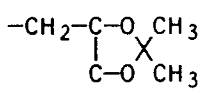 , 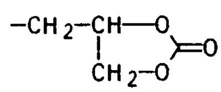 ,

$-CH_2-CH(OH)-CH_2(OH)$,

für ein Äquivalent eines Kations aus der Gruppe der Alkali-, Erdalkalimetalle, Mangan,

Kupfer, Eisen, Ammonium und substituiertes Ammonium oder für den Rest

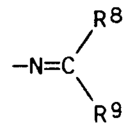 ,

wobei $R^8$ und $R^9$ unabhängig voneinander $C_1$-$C_4$-Alkyl, $C_2$-$C_6$-Alkoxyalkyl, $C_3$-$C_6$-Cycloalkyl, Phenyl, Furyl oder zusammen eine Methylenkette der Formel $-(CH_2)_m-$ mit m = 4 bis 7 Kettengliedern bedeuten und $R^9$ zusätzlich Wasserstoff bedeutet,

$R^6$ für Wasserstoff, $C_1$-$C_8$-Alkyl oder $C_3$-$C_8$-Cycloalkyl und

$R^7$ für Wasserstoff oder $C_1$-$C_8$-Alkyl stehen oder wobei

$R^6$ und $R^7$ eine Methylenkette mit 4 oder 5 Gliedern bilden,

$R^3$ Wasserstoff,
gegebenenfalls durch Hydroxy, Halogen, $C_1$-$C_4$-Alkoxy, $C_1$-$C_4$-Alkylthio oder $C_1$-$C_4$-Dialkylamino substituiertes $C_1$-$C_8$-Alkyl oder gegebenenfalls durch $C_1$-$C_4$-Alkyl, Halogen oder $C_1$-$C_4$-Halogenalkyl substituiertes $C_3$-$C_8$-Cycloalkyl und

$R^4$ Wasserstoff, Hydroxyl, $C_1$-$C_4$-Alkoxy,
gegebenenfalls durch $C_1$-$C_4$-Alkoxy, $C_1$-$C_4$-Halogenalkoxy, $C_1$-$C_4$-Alkylthio, $C_1$-$C_4$-Dialkylamino, Halogen, $C_3$-$C_6$-Cycloalkyl oder Phenyl, das durch Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio substituiert sein kann, substituiertes $C_1$-$C_{10}$-Alkyl,
gegebenenfalls durch Halogen oder $C_1$-$C_4$-Alkoxy substituiertes $C_3$-$C_{10}$-Alkenyl oder $C_3$-$C_{10}$-Alkinyl,
gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro oder Cyano substituiertes $C_3$-$C_8$-Cycloalkyl,
$C_1$-$C_4$-Dialkylamino,
einen gegebenenfalls durch $C_1$-$C_6$-Alkyl, $C_1$-$C_6$-Halogenalkyl oder Halogen substituierten 3- bis 6-gliedrigen heterocyclischen Rest mit einem oder zwei Heteroatomen, ausgewählt aus der Gruppe Sauerstoff, Schwefel und gegebenenfalls durch Methyl substituierter Stickstoff, Naphthyl,
oder gegebenenfalls durch $C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro, Cyano, Formyl, $C_1$-$C_6$-Alkanoyl oder $C_1$-$C_6$-Halogenalkanoyl substituiertes Phenyl bedeuten

oder

$R^3$ und $R^4$ gemeinsam einen Rest der Struktur $-(CH_2)_n-Y_p-(CH_2)_q-$, wobei n und q 1, 2 oder 3, p 0 oder 1 und Y Sauerstoff, Schwefel oder N-Methyl bedeuten, oder den Rest der Formel

$-(CH_2)_3-CO-$

bilden,

mit der Maßgabe, daß im Fall von X = Sauerstoff $R^1$ nicht $CH_3$ bedeutet, wenn $R^2$ eine Gruppe COOH, $COOC_2H_5$ oder $CONH_2$ und $R^3$ und $R^4$ beide Wasserstoff bedeuten; und ferner $R^1$ nicht Phenyl bedeutet, wenn $R^2$ für $COOCH_3$ und $R^3$ und beide Wasserstoff bedeuten und daß im Fall von X = Schwefel $R^2$ keine Gruppe COOH oder $CONH_2$ darstellt, wenn $R^1$, $R^3$ und $R^4$ Wasserstoff bedeuten, dadurch gekennzeichnet, daß man ein Isoxazol(Isothioazol)-5-carbonsäurehalogenid der Formel VI

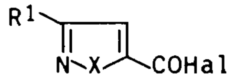 (VI)

mit einem Amin der Formel V

$HNR^3R^4$ (V)

zu einem Isoxazol(Isothiazol)amid der Formel VII

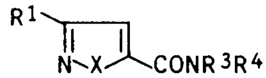

(VII)

umsetzt und dieses Amid mit Alkyllithium und Kohlendioxid zu der entsprechenden Isoxazol(Isothiazol)-4-carbonsäure der Formel Ic

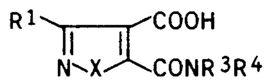

(Ic)

umsetzt und diese Säure durch Veresterung mit einem Alkohol der Formel IX

$HY-R^5$ (IX)

in Gegenwart eines wasserentziehenden Mittels in ein Isoxazol(Isothiazol)-5-carbonsäureamid der Formel I überführt.

**2.** Herbizides Mittel, enthaltend inerte Zusatzstoffe und 0,1 bis 95 Gew.% eines Isoxazol(Isothiazol)-5-carbonsäureamids der Formel I

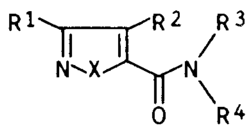

(I)

in der die Substituenten $R^1$, $R^2$, $R^3$, $R^4$ und X die gleichen Bedeutungen haben wie in Formel I gemäß Anspruch 1.

**3.** Verfahren zur Herstellung von Isoxazol(Isothiazol)-5-carbonsäureamiden der Formel Ib

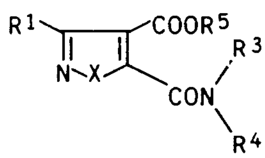

(Ib),

in der X Sauerstoff oder Schwefel bedeutet, $R^1$, $R^3$ und $R^4$ die in Anspruch 1 genannte Bedeutung haben und $R^5$ für $C_1$-$C_8$-Alkyl steht, dadurch gekennzeichnet, daß man einen Dialkylester der Formel II

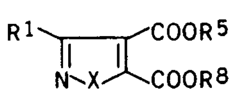

(II),

in der $R^1$, $R^5$ und X die o.g. Bedeutung haben und $R^8$ für $C_1$-$C_8$-Alkyl steht, mit etwa äquimolaren Mengen eines Amins der Formel V

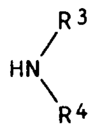

(V)

in einem organischen Lösungsmittel bei Temperaturen zwischen 0 und 100 ° C umsetzt.

## Claims

### Claims for the following Contracting States : BE, CH, DE, FR, GB, IT, LI, NL

**1.** An isoxazole(isothiazole)-5-carboxamide of the formula I

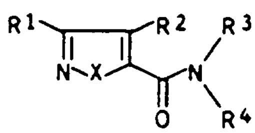

(I),

where

X is oxygen or sulfur,

$R^1$ is hydrogen,
$C_1$-$C_{10}$-alkyl which is unsubstituted or substituted by $C_1$-$C_3$-alkoxy, $C_1$-$C_3$-haloalkoxy, halogen, cyano or phenyl which may be substituted by halogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, cyano or nitro, $C_1$-$C_4$-alkoxy,
$C_3$-$C_8$-cycloalkyl which is unsubstituted or substituted by $C_1$-$C_4$-alkyl or halogen,
a 5-membered or 6-membered heterocyclic radical having one or two heteroatoms selected from the group consisting of oxygen, sulfur and nitrogen, which may be substituted by $C_1$-$C_4$-alkyl, carboxyl or $C_1$-$C_4$-alkoxycarbonyl,
or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano,

$R^2$ is formyl, 4,5-dihydrooxazol-2-yl or a radical of the formula $COYR^5$ or $CONR^6R^7$,

where

Y is oxygen or sulfur,

$R^5$ is hydrogen,
$C_1$-$C_8$-alkyl which may be substituted by $C_1$-$C_4$-alkoxy, $C_1$-$C_4$-alkoxy-$C_1$-$C_4$-alkoxy, halogen, cyano, hydroxyl, trimethylsilyl, $C_1$-$C_4$-alkylthio, $C_1$-$C_4$-alkylamino, $C_1$-$C_4$-dialkylamino, $C_1$-$C_4$-alkylsulfinyl, $C_1$-$C_4$-alkylsulfonyl, carboxyl, $C_1$-$C_4$-alkoxycarbonyl, $C_1$-$C_4$-dialkylaminocarbonyl, $C_1$-$C_4$-dialkoxyphosphonyl, alkyliminooxy, benzyloxy, benzoyl which is unsubstituted or substituted by $C_1$-$C_4$-alkyl, $C_1$-$C_3$-alkoxy or halogen, or phenyl which is unsubstituted or substituted by $C_1$-$C_4$-alkyl, $C_1$-$C_4$-alkoxy, $C_1$-$C_4$-haloalkyl, halogen, nitro or cyano, or may be substituted by thienyl, furyl, tetrahydrofuryl, phthalimido or pyridyl,
$C_3$-$C_8$-alkenyl which is unsubstituted or substituted by phenyl which may be substituted by $C_1$-$C_4$-alkyl, $C_1$-$C_4$-alkoxy, $C_1$-$C_4$-haloalkyl, halogen, nitro or cyano,
$C_3$-$C_6$-haloalkenyl,
$C_3$-$C_8$-alkynyl which is unsubstituted or substituted by hydroxy or $C_1$-$C_4$-alkoxy,
$C_3$-$C_6$-cycloalkyl,
$C_5$- or $C_6$-cycloalkenyl,
phenyl which is unsubstituted or substituted by $C_1$-$C_4$-alkyl, $C_1$-$C_4$-alkoxy, $C_1$-$C_4$-haloalkyl, halogen, nitro, cyano, $C_1$-$C_4$-alkoxycarbonyl or acylamino, a 5-membered or 6-membered heterocyclic radical having one or two heteroatoms selected from the group consisting of oxygen, sulfur and nitrogen, or a benzotriazole radical,

$C_6$- or $C_7$-cycloalkylimino, phthalimido, succinimido, or a radical

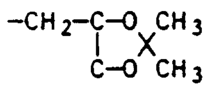 , 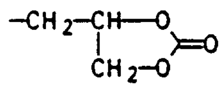 ,

$-CH_2-CH(OH)-CH_2(OH)$,
or one equivalent of a cation from the group consisting of the alkali metals, alkaline earth metals, manganese, copper, iron, ammonium and substituted ammonium or a radical

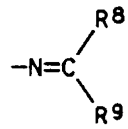 ,

where $R^8$ and $R^9$ independently of one another are $C_1$-$C_4$-alkyl, $C_2$-$C_6$-alkoxyalkyl, $C_3$-$C_6$-cycloalkyl, phenyl or furyl or together form a methylene chain of the formula $-(CH_2)_m-$, where m is from 4 to 7 chain members, and $R^9$ is additionally hydrogen,

$R^6$ is hydrogen, $C_1$-$C_8$-alkyl or $C_3$-$C_8$-cycloalkyl and
$R^7$ is hydrogen or $C_1$-$C_8$-alkyl, or
$R^6$ and $R^7$ form a methylene chain having 4 or 5 members,
$R^3$ is hydrogen,
$C_1$-$C_8$-alkyl which is unsubstituted or substituted by hydroxyl, halogen, $C_1$-$C_4$-alkoxy, $C_1$-$C_4$-alkylthio or $C_1$-$C_4$-dialkylamino, or $C_3$-$C_8$-cycloalkyl which is unsubstituted or substituted by $C_1$-$C_4$-alkyl, halogen or $C_1$-$C_4$-haloalkyl, and
$R^4$ is hydrogen, hydroxyl, $C_1$-$C_4$-alkyl,
$C_1$-$C_{10}$-alkyl which is unsubstituted or substituted by $C_1$-$C_4$-alkoxy, $C_1$-$C_4$-haloalkoxy, $C_1$-$C_4$-alkylthio, $C_1$-$C_4$-dialkylamino, halogen, $C_3$-$C_6$-cycloalkyl or phenyl which may be substituted by halogen, cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,
$C_3$-$C_{10}$-alkynyl or $C_3$-$C_{10}$-alkenyl which is unsubstituted or substituted by halogen or $C_1$-$C_4$-alkoxy,
$C_3$-$C_8$-cycloalkyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano, $C_1$-$C_4$-dialkylamino,
a 3-membered to 6-membered heterocyclic radical which is unsubstituted or substituted by $C_1$-$C_6$-alkyl, $C_1$-$C_6$-haloalkyl or halogen and has one or two heteroatoms selected from the group consisting of oxygen, sulfur and unsubstituted or methyl-substituted nitrogen, naphthyl,
or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro, cyano, formyl, $C_1$-$C_6$-alkanoyl or $C_1$-$C_6$-haloalkanoyl,

or

$R^3$ and $R^4$ together form a radical of the structure $-(CH_2)^n-Y_p-(CH_2)_q-$, where n and q are each 1, 2 or 3, p is 0 or 1 and Y is oxygen, sulfur or N-methyl, or the radical of the formula

$-CH_2)_3-CO-$

and their environmentally compatible salts, with the proviso that if X is oxygen $R^1$ is not $CH_3$ when $R^2$ is COOH, $COOC_2H_5$ or $CONH_2$ and $R^3$ and $R^4$ are both hydrogen, and furthermore $R^1$ is not phenyl when $R^2$ is $COOCH_3$ and $R^3$ and $R^4$ are both hydrogen, and that if X is sulfur $R^2$ is not COOH or $CONH_2$ when $R^1$, $R^3$ and $R^4$ are each hydrogen.

**2.** An isoxazole(isothiazole)-5-carboxamide of the formula I as claimed in claim 1, wherein $R^3$ is hydrogen.

**3.** An isoxazole(isothiazole)-5-carboxamide of the formula I as claimed in claim 1, wherein

$R^1$ is hydrogen or $C_1$-$C_4$-alkyl,

$R^2$ is $COYR^5$, where Y is oxygen or sulfur and $R^5$ is hydrogen, $C_1$-$C_4$-alkyl, phenyl which is unsubstituted or substituted by $C_1$-$C_4$-alkyl, $C_1$-$C_4$-haloalkyl, $C_1$-$C_4$-alkoxy, $C_1$-$C_4$-alkylthio or halogen, or is the radical

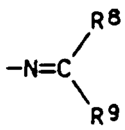

$R^3$ is hydrogen and

$R^4$ is $C_1$-$C_4$-alkyl or $C_3$-$C_8$-cycloalkyl.

**4.** An isoxazole-5-carboxamide of the formula I as claimed in claim 1, wherein $R^1$ is n-propyl, $R^2$ is propan-2-iminoxycarbonyl, $R^3$ is hydrogen and $R^4$ is tert.-butyl.

**5.** A herbicidal agent containing inert additives and an isoxazole(isothiazole)-5-carboxamide of the formula where

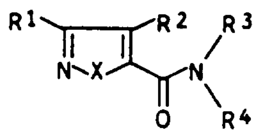

(I),

X is oxygen or sulfur,

$R^1$ is hydrogen,

$C_1$-$C_{10}$-alkyl which is unsubstituted or substituted by $C_1$-$C_3$-alkoxy, $C_1$-$C_3$-haloalkoxy, halogen, cyano or phenyl, which may be substituted by halogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, cyano or nitro, $C_1$-$C_4$-alkoxy,

$C_3$-$C_8$-cycloalkyl which is unsubstituted or substituted by $C_1$-$C_4$-alkyl or halogen,

a 5-membered or 6-membered heterocyclic radical having one or two heteroatoms selected from the group consisting of oxygen, sulfur and nitrogen, which may be substituted by $C_1$-$C_4$-alkyl, carboxyl or $C_1$-$C_4$-alkoxycarbonyl,

or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano,

$R^2$ is formyl, 4,5-dihydrooxazol-2-yl or a radical of the formula $COYR^5$ or $CONR^6R^7$,

where

Y is oxygen or sulfur,

$R^5$ is hydrogen,

$C_1$-$C_8$-alkyl which may be substituted by $C_1$-$C_4$-alkoxy, $C_1$-$C_4$-alkoxy-$C_1$-$C_4$-alkoxy, halogen, cyano, hydroxyl, trimethylsilyl, $C_1$-$C_4$-alkylthio, $C_1$-$C_4$-alkylamino, $C_1$-$C_4$-dialkylamino, $C_1$-$C_4$-alkylsulfinyl, $C_1$-$C_4$-alkylsulfonyl, carboxyl, $C_1$-$C_4$-alkoxycarbonyl, $C_1$-$C_4$-dialkylaminocarbonyl, $C_1$-$C_4$-dialkoxyphosphonyl, alkyliminooxy, benzyloxy, benzoyl which is unsubstituted or substituted by $C_1$-$C_4$-alkyl, $C_1$-$C_3$-alkoxy or halogen, or phenyl which is unsubstituted or substituted by $C_1$-$C_4$-alkyl, $C_1$-$C_4$-alkoxy, $C_1$-$C_4$-haloalkyl, halogen, nitro or cyano, or may be substituted by thienyl, furyl, tetrahydrofuryl, phthalimido or pyridyl, $C_3$-$C_8$-alkenyl which is unsubstituted or substituted by phenyl which may be substituted by $C_1$-$C_4$-alkyl, $C_1$-$C_4$-alkoxy, $C_1$-$C_4$-haloalkyl, halogen, nitro or cyano,

$C_3$-$C_6$-haloalkenyl,

$C_3$-$C_8$-alkynyl which is unsubstituted or substituted by hydroxy or $C_1$-$C_4$-alkoxy,
$C_3$-$C_6$-cycloalkyl,
$C_5$- or $C_6$-cycloalkenyl,
phenyl which is unsubstituted or substituted by $C_1$-$C_4$-alkyl, $C_1$-$C_4$-alkoxy, $C_1$-$C_4$-haloalkyl, halogen, nitro, cyano, $C_1$-$C_4$-alkoxycarbonyl or acylamino, a 5-membered or 6-membered heterocyclic radical having one or two heteroatoms selected from the group consisting of oxygen, sulfur and nitrogen, or a benzotriazole radical,

$C_6$- or $C_7$-cycloalkylimino, phthalimido, succinimido, or a radical

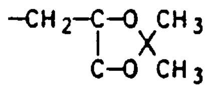 , 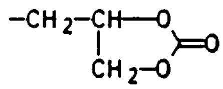 ,

$-CH_2-CH(OH)-CH_2(OH)$,
or one equivalent of a cation from the group consisting of the alkali metals, alkaline earth metals, manganese, copper, iron, ammonium and substituted ammonium or a radical

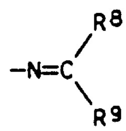 ,

where $R^8$ and $R^9$ independently of one another are $C_1$-$C_4$-alkyl, $C_2$-$C_6$-alkoxyalkyl, $C_3$-$C_6$-cycloalkyl, phenyl or furyl, or together form a methylene chain of the formula $-(CH_2)_m-$, where m is from 4 to 7 chain members, and $R^9$ is additionally hydrogen,

$R^6$ is hydrogen, $C_1$-$C_8$-alkyl or $C_3$-$C_8$-cycloalkyl and
$R^7$ is hydrogen or $C_1$-$C_8$-alkyl, or
$R^6$ and $R^7$ form a methylene chain having 4 or 5 members,
$R^3$ is hydrogen,
$C_1$-$C_8$-alkyl which is unsubstituted or substituted by hydroxyl, halogen, $C_1$-$C_4$-alkoxy, $C_1$-$C_4$-alkylthio or $C_1$-$C_4$-dialkylamino, or $C_3$-$C_8$-cycloalkyl which is unsubstituted or substituted by $C_1$-$C_4$-alkyl, halogen or $C_1$-$C_4$-haloalkyl, and
$R^4$ is hydrogen, hydroxyl,
$C_1$-$C_{10}$-alkyl which is unsubstituted or substituted by $C_1$-$C_4$-alkoxy, $C_1$-$C_4$-haloalkoxy, $C_1$-$C_4$-alkylthio, $C_1$-$C_4$-dialkylamino, halogen, $C_3$-$C_6$-cycloalkyl or phenyl, which may be substituted by halogen, cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,
$C_3$-$C_{10}$-alkynyl or $C_3$-$C_{10}$-alkenyl which is unsubstituted or substituted by halogen or $C_1$-$C_4$-alkoxy, $C_3$-$C_8$-cycloalkyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano, $C_1$-$C_4$-dialkylamino,
a 3-membered to 6-membered heterocyclic radical which is unsubstituted or substituted by $C_1$-$C_6$-alkyl, $C_1$-$C_6$-haloalkyl or halogen and has one or two heteroatoms selected from the group consisting of oxygen, sulfur and unsubstituted or methyl-substituted nitrogen,
or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro, cyano, formyl, $C_1$-$C_6$-alkanoyl or $C_1$-$C_6$-haloalkanoyl,

or

$R^3$ and $R^4$ together form a radical of the structure $-(CH_2)_n-Y_p-(CH_2)_q-$, where n and q are each 1, 2 or 3, p is 0 or 1 and Y is oxygen, sulfur or N-methyl, or the radical of the formula

$-(CH_2)_3-CO-$

or the environmentally compatible salts thereof, with the proviso that if X is oxygen $R^1$ is not $CH_3$ when $R^2$ is COOH, $COOC_2H_5$ or $CONH_2$ and $R^3$ and $R^4$ are both hydrogen, and furthermore $R^1$ is not phenyl when $R^2$ is $COOCH_3$ and $R^3$ and $R^4$ are both hydrogen, and that if X is sulfur $R^2$ is not COOH or $CONH_2$ when $R^1$, $R^3$ and $R^4$ are each hydrogen.

**6.** A herbicidal agent as claimed in claim 5, containing as active ingredient an isoxazole(isothiazole)-5-carboxamide of the formula I, $R^3$ being hydrogen.

**7.** A herbicidal agent as claimed in claim 5, containing as active ingredient an isoxazole(isothiazole)-5-carboxamide of the formula I, where

$R^1$ is hydrogen or $C_1$-$C_4$-alkyl,

$R^2$ is $COYR^5$, where Y is oxygen or sulfur and $R^5$ is hydrogen, $C_1$-$C_4$-alkyl, phenyl which is unsubstituted or substituted by $C_1$-$C_4$-alkyl, $C_1$-$C_4$-haloalkyl, $C_1$-$C_4$-alkoxy, $C_1$-$C_4$-alkylthio or halogen, or is the radical

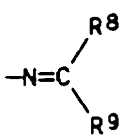

$R^3$ is hydrogen and

$R^4$ is $C_1$-$C_4$-alkyl or $C_3$-$C_8$-cycloalkyl.

**8.** A method for controlling the growth of undesirable plants, wherein the undesirable plants and/or the area to be kept free from undesirable plant growth are treated with a herbicidally effective amount of an isoxazole(isothiazole)-5-carboxamide of the formula Ia

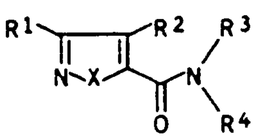

(Ia)

where

X is oxygen or sulfur,

$R^1$ is hydrogen,
$C_1$-$C_{10}$-alkyl which is unsubstituted or substituted by $C_1$-$C_3$-alkoxy, $C_1$-$C_3$-haloalkoxy, halogen, cyano or phenyl which may be substituted by halogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, cyano or nitro, $C_1$-$C_4$-alkoxy,
$C_3$-$C_8$-cycloalkyl which is unsubstituted or substituted by $C_1$-$C_4$-alkyl or halogen,
a 5-membered or 6-membered heterocyclic radical having one or two heteroatoms selected from the group consisting of oxygen, sulfur and nitrogen, which may be substituted by $C_1$-$C_4$-alkyl, carboxyl or $C_1$-$C_4$-alkoxycarbonyl,
or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano,

$R^2$ is formyl, 4,5-dihydrooxazol-2-yl or a radical of the formula $COYR^5$ or $CONR^6R^7$,

where

Y is oxygen or sulfur,

$R^5$ is hydrogen,
$C_1$-$C_8$-alkyl which may be substituted by $C_1$-$C_4$-alkoxy, $C_1$-$C_4$-alkoxy-$C_1$-$C_4$-alkoxy, halogen, cyano, hydroxyl, trimethylsilyl, $C_1$-$C_4$-alkylthio, $C_1$-$C_4$-alkylamino, $C_1$-$C_4$-

dialkylamino, $C_1$-$C_4$-alkylsulfinyl, $C_1$-$C_4$-alkylsulfonyl, carboxyl, $C_1$-$C_4$-alkoxycarbonyl, $C_1$-$C_4$-dialkylaminocarbonyl, $C_1$-$C_4$-dialkoxyphosphonyl, alkyliminooxy, benzyloxy, benzoyl which is unsubstituted or substituted by $C_1$-$C_4$-alkyl, $C_1$-$C_3$-alkoxy or halogen, or phenyl which is unsubstituted or substituted by $C_1$-$C_4$-alkyl, $C_1$-$C_4$-alkoxy, $C_1$-$C_4$-haloalkyl, halogen, nitro or cyano, or may be substituted by thienyl, furyl, tetrahydrofuryl, phthalimido or pyridyl,

$C_3$-$C_8$-alkenyl which is unsubstituted or substituted by phenyl which may be substituted by $C_1$-$C_4$-alkyl, $C_1$-$C_4$-alkoxy, $C_1$-$C_4$-haloalkyl, halogen, nitro or cyano, $C_3$-$C_6$-haloalkenyl,

$C_3$-$C_8$-alkynyl which is unsubstituted or substituted by hydroxy or $C_1$-$C_4$-alkoxy,

$C_3$-$C_6$-cycloalkyl,

$C_5$- or $C_6$-cycloalkenyl,

phenyl which is unsubstituted or substituted by $C_1$-$C_4$-alkyl, $C_1$-$C_4$-alkoxy, $C_1$-$C_4$-haloalkyl, halogen, nitro, cyano, $C_1$-$C_4$-alkoxycarbonyl or acylamino, a 5-membered or 6-membered heterocyclic radical having one or two heteroatoms selected from the group consisting of oxygen, sulfur and nitrogen, or a benzotriazole radical,

$C_6$- or $C_7$-cycloalxylimino, phthalimido, succinimido, or a radical

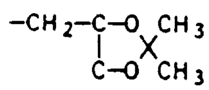 , 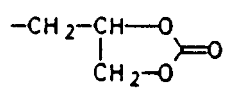 ,

$-CH_2-CH(OH)-CH_2(OH)$,

or one equivalent of a cation from the group consisting of the alkali metals, alkaline earth metals, manganese, copper, iron, ammonium and substituted ammonium or a radical

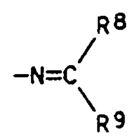 ,

where $R^8$ and $R^9$ independently of one another are $C_1$-$C_4$-alkyl, $C_2$-$C_6$-alkoxyalkyl, $C_3$-$C_6$-cycloalkyl, phenyl or furyl or together form a methylene chain of the formula $-(CH_2)_m-$, where m is from 4 to 7 chain members, and $R^9$ is additionally hydrogen,

$R^6$ is hydrogen, $C_1$-$C_8$-alkyl or $C_3$-$C_8$-cycloalkyl and

$R^7$ is hydrogen or $C_1$-$C_8$-alkyl, or

$R^6$ and $R^7$ form a methylene chain having 4 or 5 members,

$R^3$ is hydrogen,

$C_1$-$C_8$-alkyl which is unsubstituted or substituted by hydroxyl, halogen, $C_1$-$C_4$-alkoxy, $C_1$-$C_4$-alkylthio or $C_1$-$C_4$-dialkylamino, or $c_3$-$C_8$-cycloalkyl which is unsubstituted or substituted by $C_1$-$C_4$-alkyl, halogen or $C_1$-$C_4$-haloalkyl, and

$R^4$ is hydrogen, hydroxyl,

$C_1$-$C_{10}$-alkyl which is unsubstituted or substituted by $C_1$-$C_4$-alkoxy, $C_1$-$C_4$-haloalkoxy, $C_1$-$C_4$-alkylthio, $C_1$-$C_4$-dialkylamino, halogen, $C_3$-$C_6$-cycloalkyl or phenyl which may be substituted by halogen, cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,

$C_3$-$C_{10}$-alkynyl or $C_3$-$C_{10}$-alkenyl which is unsubstituted or substituted by halogen or $C_1$-$C_4$-alkoxy,

$C_3$-$C_8$-cycloalkyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano, $C_1$-$C_4$-dialkylamino,

a 3-membered to 6-membered heterocyclic radical which is unsubstituted or substituted by $C_1$-$C_6$-alkyl, $C_1$-$C_6$-haloalkyl or halogen and has one or two heteroatoms selected from the group consisting of oxygen, sulfur and unsubstituted or methyl-substituted nitrogen,

or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro, cyano, formyl, $C_1$-$C_6$-alkanoyl or $C_1$-$C_6$-haloalkanoyl,

or

$R^3$ and $R^4$ together form a radical of the structure $-(CH_2)^n-Y_p-(CH_2)_q-$, where n and q are each 1, 2 or 3, p is 0 or 1 and Y is oxygen, sulfur or N-methyl, or the radical of the formula

$-CH_2)_3-CO-$

and their environmentally compatible salts.

**9.** The process for the preparation of an isoxazole(isothiazole)-5-carboxamide of the formula I as claimed in claim 1, wherein an isoxazole(isothiazole)-5-carbonyl halide of the formula VI

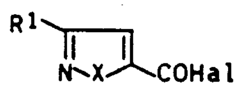

(VI)

is reacted with an amine of the formula V

$HNR^3R^4$ (V)

to give an isoxazole(isothiazole)carboxamide of the formula VII

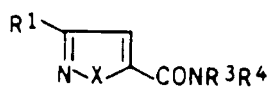

(VII)

and this amide is reacted with an alkyllithium and carbon dioxide to give the corresponding isoxazole-(isothiazole)-4-carboxylic acid of the formula Ic

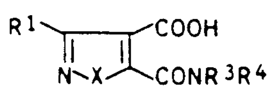

(Ic)

and this acid is converted into an isoxazole(isothiazole)-5-carboxamide of the formula I by esterification with an alcohol of the formula IX

$HY-R^5$ (IX)

in the presence of a dehydrating agent.

**10.** A process for the preparation of an isoxazole(isothiazole)-5-carboxamide of the formula Ib

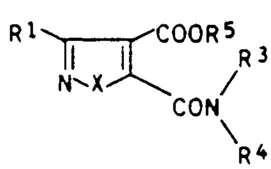

(Ib),

where X is oxygen or sulfur, $R^1$, $R^3$ and $R^4$ have the meanings stated in claim 1 and $R^5$ is $C_1$-$C_8$-alkyl,

wherein a dialkyl ester of the formula II

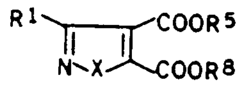

(II),

wherein $R^1$, $R^5$ and X have the abovementioned meanings and $R^8$ is $C_1$-$C_8$-alkyl, is reacted with a roughly equimolar amount of an amine of the formula V

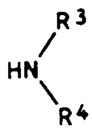

(V)

in an organic solvent at from 0 to 100 ° C.

**Claims for the following Contracting State : ES**

**1.** A process for the preparation of isoxazole(isothiazole)-5-carboxamides of the formula I

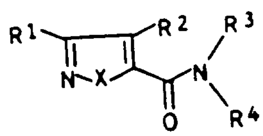

(I),

where

X is oxygen or sulfur,

$R^1$ is hydrogen,
$C_1$-$C_{10}$-alkyl which is unsubstituted or substituted by $C_1$-$C_3$-alkoxy, $C_1$-$C_3$-haloalkoxy, halogen, cyano or phenyl which may be substituted by halogen, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio, $C_1$-$C_4$-haloalkylthio, cyano or nitro, $C_1$-$C_4$-alkoxy,
$C_3$-$C_8$-cycloalkyl which is unsubstituted or substituted by $C_1$-$C_4$-alkyl or halogen,
a 5-membered or 6-membered heterocyclic radical having one or two heteroatoms selected from the group consisting of oxygen, sulfur and nitrogen, which may be substituted by $C_1$-$C_4$-alkyl, carboxyl or $C_1$-$C_4$-alkoxycarbonyl,
or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano,

$R^2$ is formyl, 4,5-dihydrooxazol-2-yl or a radical of the formula $COYR^5$ or $CONR^6R^7$,

where

Y is oxygen or sulfur,

$R^5$ is hydrogen,

$C_1$-$C_8$-alkyl which may be substituted by $C_1$-$C_4$-alkoxy, $C_1$-$C_4$-alkoxy-$C_1$-$C_4$-alkoxy, halogen, cyano, hydroxyl, trimethylsilyl, $C_1$-$C_4$-alkylthio, $C_1$-$C_4$-alkylamino, $C_1$-$C_4$-dialkylamino, $C_1$-$C_4$-alkylsulfinyl, $C_1$-$C_4$-alkylsulfonyl, carboxyl, $C_1$-$C_4$-alkoxycarbonyl, $C_1$-$C_4$-dialkylaminocarbonyl, $C_1$-$C_4$-dialkoxyphosphonyl, alkyliminooxy, benzyloxy, benzoyl which is unsubstituted or substituted by $C_1$-$C_4$-alkyl, $C_1$-$C_3$-alkoxy or halogen, or phenyl which is unsubstituted or substituted by $C_1$-$C_4$-alkyl, $C_1$-$C_4$-alkoxy, $C_1$-$C_4$-haloalkyl, halogen, nitro or cyano, or may be substituted by thienyl, furyl,

tetrahydrofuryl, phthalimido or pyridyl,
$C_3$-$C_8$-alkenyl which is unsubstituted or substituted by phenyl which may be substituted by $C_1$-$C_4$-alkyl, $C_1$-$C_4$-alkoxy, $C_1$-$C_4$-haloalkyl, halogen, nitro or cyano,
substituted $C_3$-$C_8$-alkenyl,
$C_3$-$C_6$-haloalkenyl,
$C_3$-$C_8$-alkynyl which is unsubstituted or substituted by hydroxyl or $C_1$-$C_4$-alkoxy,
$C_3$-$C_6$-cycloalkyl,
$C_5$- or $C_6$-cycloalkenyl,
phenyl which is unsubstituted or substituted by $C_1$-$C_4$-alkyl, $C_1$-$C_4$-alkoxy, $C_1$-$C_4$-haloalkyl, halogen, nitro, cyano, $C_1$-$C_4$-alkoxycarbonyl or acylamino, a 5-membered or 6-membered heterocyclic radical having one or two heteroatoms selected from the group consisting of oxygen, sulfur and nitrogen, or a benzotriazole radical,
$C_6$- or $C_7$-cycloalkylimino, phthalimido, succinimido, or a radical

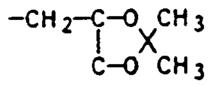 , 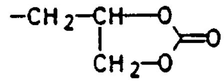 ,

$-CH_2-CH(OH)-CH_2(OH)$,
or one equivalent of a cation from the group consisting of the alkali metals, alkaline earth metals, manganese, copper, iron, ammonium and substituted ammonium or a radical

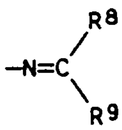 ,

where $R^8$ and $R^9$ independently of one another are $C_1$-$C_4$-alkyl, $C_2$-$C_6$-alkoxyalkyl, $C_3$-$C_6$-cycloalkyl, phenyl or furyl or together form a methylene chain of the formula $-(CH_2)_m-$, where m is from 4 to 7 chain members, and $R^9$ is additionally hydrogen,

$R^6$ is hydrogen, $C_1$-$C_8$-alkyl or $C_3$-$C_8$-cycloalkyl and

$R^7$ is hydrogen or $C_1$-$C_8$-alkyl, or

$R^6$ and $R^7$ form a methylene chain having 4 or 5 members,

$R^3$ is hydrogen,
$C_1$-$C_8$-alkyl which is unsubstituted or substituted by hydroxyl, halogen, $C_1$-$C_4$-alkoxy, $C_1$-$C_4$-alkylthio or $C_1$-$C_4$-dialkylamino,
or $C_3$-$C_8$-cycloalkyl which is unsubstituted or substituted by $C_1$-$C_4$-alkyl, halogen or $C_1$-$C_4$-haloalkyl, and

$R^4$ is hydrogen, hydroxyl, $C_1$-$C_4$-alkyl,
$C_1$-$C_{10}$-alkyl which is unsubstituted or substituted by $C_1$-$C_4$-alkoxy, $C_1$-$C_4$-haloalkoxy, $C_1$-$C_4$-alkylthio, $C_1$-$C_4$-dialkylamino, halogen, $C_3$-$C_6$-cycloalkyl or phenyl which may be substituted by halogen, cyano, nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio or $C_1$-$C_4$-haloalkylthio,
$C_3$-$C_{10}$-alkynyl or $C_3$-$C_{10}$-alkenyl which is unsubstituted or substituted by halogen or $C_1$-$C_4$-alkoxy,
$C_3$-$C_8$-cycloalkyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro or cyano, $C_1$-$C_4$-dialkylamino,
a 3-membered to 6-membered heterocyclic radical which is unsubstituted or substituted by $C_1$-$C_6$-alkyl, $C_1$-$C_6$-haloalkyl or halogen and has one or two heteroatoms selected from the group consisting of oxygen, sulfur and unsubstituted or methyl-substituted nitrogen, naphthyl,
or phenyl which is unsubstituted or substituted by $C_1$-$C_6$-alkyl, $C_1$-$C_6$-haloalkyl, $C_1$-$C_6$-alkoxy, $C_1$-$C_6$-haloalkoxy, $C_1$-$C_6$-alkylthio, $C_1$-$C_6$-haloalkylthio, halogen, nitro,

cyano, formyl, $C_1$-$C_6$-alkanoyl or $C_1$-$C_6$-haloalkanoyl,

or $R^3$ and $R^4$ together form a radical of the structure $-(CH_2)_n-Y_p-(CH_2)_q-$, where n and q are each 1, 2 or 3, p is 0 or 1 and Y is oxygen, sulfur or N-methyl, or the radical of the formula

$-CH_2)_3-CO-$

with the proviso that if X is oxygen $R^1$ is not $CH_3$ when $R^2$ is COOH, $COOC_2H_5$ or $CONH_2$ and $R^3$ and $R^4$ are both hydrogen, and furthermore $R^1$ is not phenyl when $R^2$ is $COOCH_3$ and $R^3$ and $R^4$ are both hydrogen, and that if X is sulfur $R^2$ is not COOH or $CONH_2$ when $R^1$, $R^3$ and $R^4$ are each hydrogen, wherein an isoxazole(isothiazole)-5-carbonyl halide of the formula VI

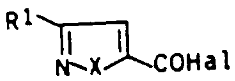

(VI)

is reacted with an amine of the formula V

$HNR^3R^4$ (V)

to give an isoxazole(isothiazole)carboxamide of the formula VII

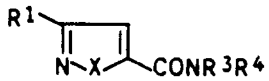

(VII)

and this amide is reacted with an alkyllithium carbon dioxide to give the corresponding isoxazole-(isothiazole)-4-carboxylic acid of the formula Ic

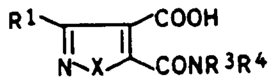

(Ic)

and this acid is converted into an isoxazole(isothiazole)-5-carboxamide of the formula I by esterification with an alcohol of the formula IX

$HY-R^5$ (IX)

in the presence of a dehydrating agent.

**2.** A herbicidal agent containing inert additives and from 0.1 to 95% by weight of an isoxazole(isothiazole)-5-carboxamide of the formula I

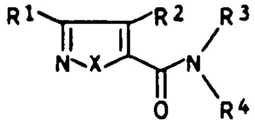

(I)

where $R^1$, $R^2$, $R^3$, $R^4$ and X have the same meanings as in formula I as claimed in claim 1.

3. A process for the preparation of an isoxazole(isothiazole)-5-carboxamide of the formula Ib

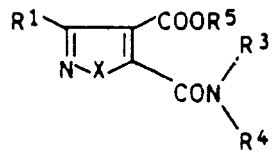
(Ib),

where X is oxygen or sulfur, $R^1$, $R^3$ and $R^4$ have the meanings stated in claim 1 and $R^5$ is $C_1$-$C_8$-alkyl, wherein a dialkyl ester of the formula II

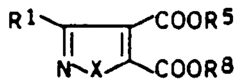
(II),

where $R^1$, $R^5$ and X have the abovementioned meaning and $R^8$ is $C_1$-$C_8$-alkyl, is reacted with a roughly equimolar amount of an amine of the formula V

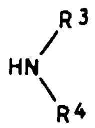
(V)

in an organic solvent at from 0 to 100 °C.

**Revendications**
**Revendications pour les Etats contractants suivants : BE, CH, DE, FR, GB, IT, LI, NL**

1. Amides d'acide isoxazol (isothiazol)-5-carboxylique, de formule I

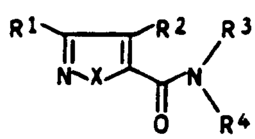
(I),

dans laquelle

X = oxygène ou soufre,

$R^1$ = hydrogène,

alkyle en C 1-C 10, éventuellement substitué par alcoxy en C 1-C 3, halogénalcoxy en C 1-C 3, halogène, cyano ou phényle pouvant être substitué par halogène, alkyle en C 1-C 4, halogénalkyle en C 1-C 4, alcoxy en C 1-C 4, halogénalcoxy en C 1-C 4, alkylthio en C 1-C 4, halogénalkylthio en C 1-C 4, cyano ou nitro,
alcoxy en C 1-C 4,
cyloalkyle en C 3-C 8, éventuellement substitué par alkyle en C 1-C 4 ou halogène,
un reste hétérocyclique à 5 ou 6 chaînons et à un ou deux hétéroatomes choisis dans le groupe constitué par oxygène, soufre et azote, qui peut être substitué par alkyle en C 1-C 4, carboxyle ou alcoxycarbonyle en C 1-C 4,
ou phényle, éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro ou cyano,

$R^2$ = formyle, 4,5-dihydro-oxazol-2-yle ou un reste de formule $COYR^5$ ou $CONR^6R^7$,

où

Y = oxygène ou soufre,

$R^5$ = hydrogène,

alkyle en C 1-C 8 pouvant être substitué par alcoxy en C 1-C 4, alcoxy-en C 1-C 4-alcoxy-en C 1-C 4, halogène, cyano, hydroxy, triméthylsilyle, alkylthio en C 1-C 4, alkylamino en C 1-C 4, dialkylamino en C 1-C 4, alkylsulfinyle en C 1-C 4, alkylsulfonyle en C 1-C 4, carboxyle, alcoxycarbonyle en C 1-C 4, dialkylaminocarbonyle en C 1-C 4, dialcoxyphosphonyle en C 1-C 4, alcaline-iminoxy, benzyloxy, benzoyle, éventuellement substitué par alkyle en C 1-C 4, alcoxy en C 1-C 3 ou par halogène, ou phényle, éventuellement substitué par alkyle en C 1-C 4, alcoxy en C 1-C 4, halogénalkyle en C 1-C 4, halogène, nitro ou cyano, par thiényle, furyle, tétrahydrofuryle, phtalimido ou pyridyle,

alcényle en C 3-C 8, éventuellement substitué par phényle pouvant être substitué par alkyle en C 1-C 4, alcoxy en C 1-C 4, halogénalkyle en C 1-C 4, halogène, nitro ou cyano,

halogénalcényle en C 3-C 6

alcynyle en C 3-C 8, éventuellement substitué par hydroxy ou alcoxy en C 1-C 4,

cycloalkyle en C 3-C 6,

cycloalcényle en C 5-C 6,

phényle, éventuellement substitué par alkyle en C 1-C 4, alcoxy en C 1-C 4, halogénalkyle en C 1-C 4, halogène, nitro, cyano, alcoxycarbonyle en C 1-C 4 ou acylamino,

un reste hétérocyclique à 5 ou 6 chaînons et à un ou deux hétéroatomes choisis dans le groupe constitué par oxygène, soufre et azote, ou un reste benzotriazole,

cycloalcane-en C 6-C 7-imino, phtalimido, succinimido, les restes

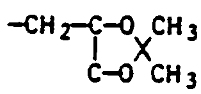 , 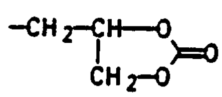 ,

$-CH_2-CH(OH)-CH_2(OH)$,

l'équivalent d'un cation choisi dans le groupe métaux alcalins, alcalino-terreux, manganèse, cuivre, fer, ammonium et ammonium substitué, ou le reste

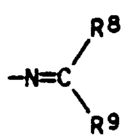 ,

où $R^8$ et $R^9$ représentent, indépendamment l'un de l'autre, alkyle en C 1-C 4, alcoxyalkyle en C 2-C 6, cycloalkyle en C 3-C 6, phényle, furyle ou forment ensemble une chaîne méthylène de formule $-(CH_2)_m-$avec m = 4 à 7 chaînons, $R^9$ pouvant aussi représenter hydrogène,

$R^6$ = hydrogène, alkyle en C 1-C 8 ou cycloalkyle en C 3-C 8, et

$R^7$ = hydrogène ou alkyle en C 1-C 8, ou
$R^6$ et $R^7$ forment une chaîne méthylène à 4 ou 5 chaînons,
$R^3$ = hydrogène,

alkyle en C 1-C 8, éventuellement substitué par hydroxy, halogène, alcoxy en C 1-C 4, alkylthio en C 1-C 4 ou dialkylamino en C 1-C 4, ou cycloalkyle en C 3-C 8, éventuellement substitué par alkyle en C 1-C 4, halogène ou halogénalkyle en C 1-C 4, et

$R^4$ = hydrogène, hydroxyle, alcoxy en C 1-C 4,
alkyle en C 1-C 10, éventuellement substitué par alcoxy en C 1-C 4, halogénalcoxy en C 1-C 4, alkylthio en C 1-C 4, dialkylamino en C 1-C 4, halogène, cycloalkyle en C 3-C 6 ou phényle, qui peut être substitué par halogène, cyano, nitro, alkyle en C 1-C 4, halogénalkyle en C 1-C 4, alcoxy en C 1-C 4, halogénalcoxy en C 1-C 4, alkylthio en C 1-C 4 ou halogénalkylthio en C 1-C 4,

alcényle en C 3-C 10 ou alcynyle en C 3-C 10, éventuellement substitués par halogéne ou alcoxy en C 1-C 4,

cycloalkyle en C 3-C 8, éventuellement substitué par alkyle en C 1-C 6, alkylthio en C 1-C 6, halogénalkylthio en C 1-C 6, alkylthio en C 1-C 6, halogénalkylthio en C 1-C 6, halogène, nitro ou cyano,

dialkylamino en C 1-C 4,

un reste hétérocyclique à 3 à 6 chaînons et à un ou deux hétéroatomes choisis dans le groupe oxygène, soufre et azote, éventuellement substitué par méthyle; naphtyle, éventuellement substitué par alkyle en C 1-C 6, halogénoalkyle en C 1-C 6 ou halogène ou phényle éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro, cyano, formyle, alcanoyle en C 1-C 6 ou halogénalcanoyle en C 1-C 6,

ou

$R^3$ et $R^4$ forment, ensemble, un reste de structure $-(CH_2)_n - Y_p- (CH_2)_q-$, n et q représentant 1, 2 ou 3, p = 0 ou 1 et
Y = oxygène, soufre ou N-méthyle, ou le reste de formule

$-(CH_2)_3-CO-$,

ainsi que leurs sels acceptables par l'environnement, étant entendu que, dans le cas où X = oxygène, $R^1$ ne représente pas $CH_3$ lorsque $R^2$ est mis pour un groupe COOH, $COOC_2H_5$ ou $CONH_2$ et $R^3$ et $R^4$ représentent, chacun, hydrogène; en outre, $R^1$ ne représente pas phényle lorsque $R^2$ est mis pour $COOCH_3$ et $R^3$ et si les deux restes représentent hydrogène et dans le cas où X = soufre, $R^2$ ne représente pas le groupe COOH ou $CONH_2$ si $R^1$, $R^3$ et $R^4$ sont mis pour hydrogène.

**2.** Amides d'acide isoxazol(isothiazol)-5-carboxylique de formule I, selon la revendication 1, caractérisés par le fait que $R^3$ représente hydrogène.

**3.** Amides d'acide isoxazol(isothiazol)-5-carboxylique de formule I, selon la revendication 1, caractérisés par le fait que
$R^1$ = hydrogène ou alkyle en C 1-C 4,
$R^2$ = $COYR^5$, où Y est mis pour oxygène ou soufre et $R^5$ pour hydrogène, alkyle en C 1-C 4, phényle éventuellement substitué par alkyle en C 1-C 4, halogénalkyle en C 1-C 4, alcoxy en C 1-C 4, alkylthio en C 1-C 4 ou halogène, ou pour le reste

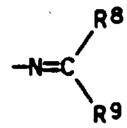

$R^3$ = hydrogène et
$R^4$ = alkyle en C 1-C 4 ou cycloalkyle en C 3-C 8.

4. Amide d'acide isoxazol-5-carboxylique de formule I, selon la revendication 1, caractérisé par le fait que $R^1$ représente n-propyle, $R^2$, propano-2-iminoxycarbonyle, $R^3$, hydrogène et $R^4$ tert-butyle.

5. Herbicide, contenant, outre des additifs inertes, un amide d'acide isoxazol(isothiazol)-5-carboxylique de formule

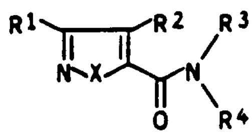

(I)

dans laquelle
X = oxygène ou soufre,
$R^1$ = hydrogène,

alkyle en C 1-C 10, éventuellement substitué par alcoxy en C 1-C 3, halogéenalcoxy en C 1-C 3, halogène, cyano ou phényle pouvant être substitué par halogène, alkyle en C 1-C 4, halogénalkyle en C 1-C 4, alcoxy en C 1-C 4, halogénalcoxy en C 1-C 4, alkylthio en C 1-C 4, halogénalkylthio en C 1-C 4, cyano ou nitro,
alcoxy en C 1-C 4,
cyloalkyle en C 3-C 8, éventuellement substitué par alkyle en C 1-C 4 ou halogène,
un reste hétérocyclique à 5 ou 6 chaînons et à un ou deux hétéroatomes choisis dans le groupe constitué par oxygène, soufre et azote, qui peut être substitué par alkyle en C 1-C 4, carboxyle ou alcoxycarbonyle en C 1-C 4,
ou phényle, éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro ou cyano,

$R^2$ = formyle, 4,5-dihydro-oxazol-2-yle ou un reste de formule $COYR^5$ ou $CONR^6R^7$,

où
Y = oxygène ou soufre,
$R^5$ = hydrogène,

alkyle en C 1-C 8 pouvant être substitué par alcoxy en C 1-C 4, alcoxy-en C 1-C 4-alcoxy-en C 1-C 4, halogène, cyano, hydroxy, triméthylsilyle, alkylthio en C 1-C 4, alkylamino en C 1-C 4, dialkylamino en C 1-C 4, alkylsulfinyle en C 1-C 4, alkylsulfonyle en C 1-C 4, carboxyle, alcoxycarbonyle en C 1-C 4, dialkylaminocarbonyle en C 1-C 4, dialcoxyphosphonyle en C 1-C 4, alcaline-iminoxy, benzyloxy, benzoyle, éventuellement substitué par alkyle en C 1-C 4, alcoxy en C 1-C 3 ou par halogène, ou phényle, éventuellement substitué par alkyle en C 1-C 4, alcoxy en C 1-C 4, halogénalkyle en C 1-C 4, halogène, nitro ou cyano, par thiényle, furyle, tétrahydrofuryle, phtalimido ou pyridyle,

alcényle en C 3-C 8, éventuellement substitué par phényle pouvant être substitué par alkyle en C 1-C 4, alcoxy en C 1-C 4, halogénalkyle en C 1-C 4, halogène, nitro ou

cyano,

halogénalcényle en C 3-C 6

alcynyle en C 3-C 8, éventuellement substitué par hydroxy ou alcoxy en C 1-C 4,

cycloalkyle en C 3-C 6,

cycloalcényle en C 5-C 6,

phényle, éventuellement substitué par alkyle en C 1-C 4, alcoxy en C 1-C 4, halogénalkyle en C 1-C 4, halogène, nitro, cyano, alcoxycarbonyle en C 1-C 4 ou acylamino,

un reste hétérocyclique à 5 ou 6 chaînons et à un ou deux hétéroatomes choisis dans le groupe constitué par oxygène, soufre et azote, ou un reste benzotriazole,

cycloalcane-en C 6-C 7-imino, phtalimido, succinimido, les restes

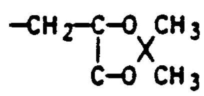 , 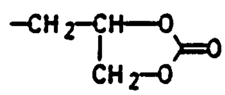 ,

$-CH_2-CH(OH)-CH_2(OH)$,
l'équivalent d'un cation choisi dans le groupe métaux alcalins, alcalino-terreux, manganèse, cuivre, fer, ammonium et ammonium substitué, ou le reste

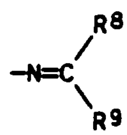 ,

où $R^8$ et $R^9$ représentent, indépendamment l'un de l'autre, alkyle en C 1-C 4, alcoxyalkyle en C 2-C 6, cycloalkyle en C 3-C 6, phényle, furyle ou forment ensemble une chaîne méthylène de formule - $(CH_2)_m$-avec m = 4 à 7 chaînons, $R^9$ pouvant aussi représenter hydrogène,

$R^6$ = hydrogène, alkyle en C 1-C 8 ou cycloalkyle en C 3-C 8, et
$R^7$ = hydrogène ou alkyle en C 1-C 8, ou
$R^6$ et $R^7$ forment une chaîne méthylène à 4 ou 5 chaînons,
$R^3$ = hydrogène,

alkyle en C 1-C 8, éventuellement substitué par hydroxy, halogène, alcoxy en C 1-C 4, alkylthio en C 1-C 4 ou dialkylamino en C 1-C 4, ou cycloalkyle en C 3-C 8, éventuellement substitué par alkyle en C 1-C 4, halogène ou halogénalkyle en C 1-C 4, et

$R^4$ = hydrogène, hydroxyle, alcoxy en C 1-C 4,
alkyle en C 1-C 10, éventuellement substitué par alcoxy en C 1-C 4, halogénalcoxy en C 1-C 4, alkylthio en C 1-C 4, dialkylamino en C 1-C 4, halogène, cycloalkyle en C 3-C 6 ou phényle, qui peut être substitué par halogène, cyano, nitro, alkyle en C 1-C 4, halogénalkyle en C 1-C 4, alcoxy en C 1-C 4, halogénalcoxy en C 1-C 4, alkylthio en C 1-C 4 ou halogénalkylthio en C 1-C 4,

alcényle en C 3-C 10 ou alcynyle en C 3-C 10, éventuellement substitués par halogéne ou alcoxy en C 1-C 4,

cycloalkyle en C 3-C 8, éventuellement substitué par alkyle en C 1-C 6, alkylthio en C 1-C 6, halogénalkylthio en C 1-C 6, alkylthio en C 1-C 6, halogénalkylthio en C 1-C 6, halogène, nitro ou cyano,

dialkylamino en C 1-C 4,

un reste hétérocyclique à 3 à 6 chaînons et à un ou deux hétéroatomes choisis dans le groupe oxygène, soufre et azote, éventuellement substitué par méthyle; naphtyle, éventuellement substitué par alkyle en C 1-C 6, halogénoalkyle en C 1-C 6 ou halogène, ou phényle éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro, cyano, formyle, alcanoyle en C 1-C 6 ou halogénalcanoyle en C 1-C 6,

ou

$R^3$ et $R^4$ forment, ensemble, un reste de structure $-(CH_2)_n - Y_p- (CH_2)_q-$, n et q représentant 1, 2 ou 3, p = 0 ou 1 et Y = oxygène, soufre ou N-méthyle, ou le reste de formule

$-(CH_2)_3-CO-$,

ainsi que leurs sels acceptables par l'environnement, étant entendu que dans le cas où X = oxygène, $R^1$ ne représente pas $CH_3$ lorsque $R^2$ est mis pour un groupe COOH, $COOC_2H_5$ ou $CONH_2$ et $R^3$ et $R^4$ représentent, chacun, hydrogène; en outre, $R^1$ ne représente pas phényle lorsque $R^2$ est mis pour $COOCH_3$ et $R^3$ et si les deux restes représentent hydrogène et dans le cas où X = soufre, $R^2$ ne représente pas le groupe COOH ou $CONH_2$ si $R^1$, $R^3$ et $R^4$ sont mis pour hydrogène.

6. Herbicide selon la revendication 5, caractérisé par le fait qu'il contient, comme principe actif, un amide d'acide isoxazol(isothiazol)-5-carboxylique de formule I, dans laquelle $R^3$ représente hydrogène

7. Herbicide selon la revendication 5, caractérisé par le fait qu'il contient, comme principe actif, un amide d'acide isoxanol(isothiazol)-5-carboxylique de formule I, dans laquelle
   $R^1$ = hydrogène ou alkyle en C 1-C 4,
   $R^2$ = $COYR^5$ où Y est mis pour oxygène ou soufre et $R^5$ pour hydrogène, alkyle en C 1-C 4, phényle, éventuellement substitué par alkyle en C 1-C 4, halogénalkyle en C 1-C 4, alcoxy en C 1-C 4, alkylthio en C 1-C 4 ou halogène, ou pour le reste

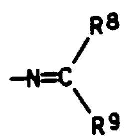

   $R^3$ = hydrogène et
   $R^4$ = alkyle en C 1-C 4 ou cycloalkyle en C 3-C 8.

8. Procédé de lutte contre la croissance de plantes indésirables, caractérisé par le fait que l'on traite les plantes indésirables et/ou la surface à maintenir exempte de la croissance de plantes indésirables avec une quantité efficace du point de vue herbicide d'un amide d'acide isoxazol(isothiazol)-5-carboxylique de formule Ia

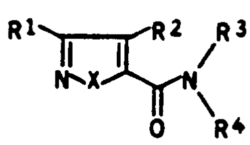

(Ia)

dans laquelle

X = oxygène ou soufre,

$R^1$ = hydrogène

halogénalcoxy en C 1-C 3, halogène, cyano ou phényle pouvant être substitué par halogène, alkyle en C 1-C 4, halogénalkyle en C 1-C 4, alcoxy en C 1-C 4, halogénalcoxy en C 1-C 4, alkylthio en C 1-C 4, halogénalkylthio en C 1-C 4, cyano ou nitro, alcoxy en C 1-C 4,
cyloalkyle en C 3-C 8, éventuellement substitué par alkyle en C 1-C 4 ou halogène,
un reste hétérocyclique à 5 ou 6 chaînons et à un ou deux hétéroatomes choisis dans le groupe constitué par oxygène, soufre et azote, qui peut être substitué par alkyle en C 1-C 4, carboxyle ou alcoxycarbonyle en C 1-C 4,
ou phényle, éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro ou cyano,

$R^2$ = formyle, 4,5-dihydro-oxazol-2-yle ou un reste de formule $COYR^5$ ou $CONR^6R^7$,

où

Y = oxygène ou soufre,

$R^5$ = hydrogène,

alkyle en C 1-C 8 pouvant être substitué par alcoxy en C 1-C 4, alcoxy-C 1-C 4-alcoxy-C 1-C 4, halogène, cyano, hydroxy, triméthylsilyle, alkylthio en C 1-C 4, alkylamino en C 1-C 4, dialkylamino en C 1-C 4, alkylsulfinyle en C 1-C 4, alkylsulfonyle en C 1-C 4, carboxyle, alcoxycarbonyle en C 1-C 4, dialkylaminocarbonyle en C 1-C 4, dialcoxyphosphonyle en C 1-C 4, alcaline-iminoxy, benzyloxy, benzoyle, éventuellement substitué par alkyle en C 1-C 4, alcoxy en C 1-C 3 ou par halogène, ou phényle, éventuellement substitué par alkyle en C 1-C 4, alcoxy en C 1-C 4, halogénalkyle en C 1-C 4, halogène, nitro ou cyano, par thiényle, furyle, tétrahydrofuryle, phtalimido ou pyridyle,

alcényle en C 3-C 8, éventuellement substitué par phényle pouvant être substitué par alkyle en C 1-C 4, alcoxy en C 1-C 4, halogénalkyle en C 1-C 4, halogène, nitro ou cyano,

halogénalcényle en C 3-C 6

alcynyle en C 3-C 8, éventuellement substitué par hydroxy ou alcoxy en C 1-C 4,

cycloalkyle en C 3-C 6,

cycloalcényle en C 5-C 6,

phényle, éventuellement substitué par alkyle en C 1-C 4, alcoxy en C 1-C 4, halogénalkyle en C 1-C 4, halogène, nitro, cyano, alcoxycarbonyle en C 1-C 4 ou acylamino,

un reste hétérocyclique à 5 ou 6 chaînons et à un ou deux hétéroatomes choisis dans le groupe constitué par oxygène, soufre et azote, ou un reste benzotriazole,

cycloalcane-C 6-C 7-imino, phtalimido, succinimido, les restes

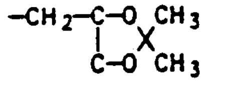 , 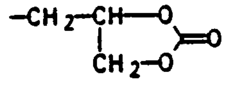 ,

$-CH_2-CH(OH)-CH_2(OH)$,

l'équivalent d'un cation choisi dans le groupe métaux alcalins, alcalino-terreux, manganèse, cuivre, fer, ammonium et ammonium substitué, ou le reste

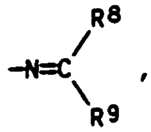

où $R^8$ et $R^9$ représentent, indépendamment l'un de l'autre, alkyle ne C 1-C 4, alcoxyalkyle en C 2-C 6, cycloalkyle en C 3-C 6, phényle, furyle ou forment ensemble une chaîne méthylène de formule - $(CH_2)_m$-avec m = 4 à 7 chaînons, $R^9$ pourvant aussi représenter hydrogène,

$R^6$ = hydrogène, alkyle en C 1-C 8 ou cycloalkyle en C 3-C 8, et

$R^7$ = hydrogène ou alkyle en C 1-C 8, ou

$R^6$ et $R^7$ forment une chaîne méthylène à 4 ou 5 chaînons,

$R^3$ = hydrogène,

alkyle en C 1-C 8, éventuellement substitué par hydroxy, halogène, alcoxy en C 1-C 4, alkylthio en C 1-C 4 ou dialkylamino en C 1-C 4, ou cycloalkyle en C 3-C 8, éventuellement substitué par alkyle en C 1-C 4, halogène ou halogénalkyle en C 1-C 4, et

$R^4$ = hydrogène, hydroxyle, alcoxy en C 1-C 4,

alkyle en C 1-C 10, éventuellement substitué par alcoxy en C 1-C 4, halogénalcoxy en C 1-C 4, alkylthio en C 1-C 4, dialkylamino en C 1-C 4, halogène, cycloalkyle en C 3-C 6 ou phényle, qui peut être substitué par halogène, cyano, nitro, alkyle en C 1-C 4, halogénalkyle en C 1-C 4, alcoxy en C 1-C 4, halogénalcoxy en C 1-C 4, alkylthio en C 1-C 4 ou halogénalkylthio en C 1-C 4,

alcényle en C 3-C 10 ou alcynyle en C 3-C 10, éventuellement substitués par halogéne ou alcoxy en C 1-C 4,

cycloalkyle en C 3-C 8, éventuellement substitué par alkyle en C 1-C 6, alkylthio en C 1-C 6, halogénalkylthio en C 1-C 6, alkylthio en C 1-C 6, halogénalkylthio en C 1-C 6, halogène, nitro ou cyano,

dialkylamino en C 1-C 4,

un reste hétérocyclique à 3 à 6 chaînons et à un ou deux hétéroatomes choisis dans le groupe oxygène, soufre et azote, éventuellement substitué par méthyle; naphtyle, ou phényle éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro, cyano, formyle, alcanoyle en C 1-C 6 ou halogénalcanoyle en C 1-C 6,

ou

$R^3$ et $R^4$ forment, ensemble, un reste de structure $-(CH_2)n - Y_p- (CH_2)_q-$, n et q représentant 1, 2 ou 3, p = 0 ou 1 et Y = oxygène, soufre ou N-méthyle, ou le reste de formule

$-(CH_2)_3-CO-$,

ainsi que leurs sels acceptables par l'environnement.

**9.** Procédé de préparation d'amides d'acide isoxazol(isothiazol)-5-carboxylique de formule I, selon la revendication 1, caractérisé par le fait que l'on fait réagir un halogénure d'acide isoxazol(isothiazol)-5-carboxylique de formule VI

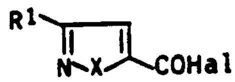

(VI)

avec une amine de formule V

$HNR^3R^4$ (V)

pour obtenir un isoxazol(isothiazol) amide de formule VII

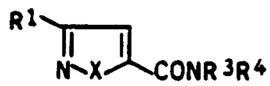

(VII)

et on met à réagir cet amide avec de l'alkyllithium et de l'anhydride carboxylique pour obtenir l'acide isoxazol(isothiazol)-4-carboxylique correspondant de formule Ic

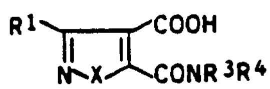

(Ic)

et on transforme cet acide, par estérification avec un alcool de formule IX

$HY-R^5$ (IX),

en présence d'un agent déshydratant, en amide d'acide isoxazol(isothiazol)-5-carboxylique de formule I.

**10.** Procédé de préparation d'amides d'acide isoxazol(isothiazol)-5-carboxylique de formule Ib

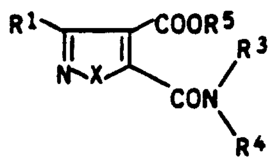

(Ib),

dans laquelle X représente oxygène ou soufre, $R^1$, $R^3$ et $R^4$ ont les significations données dans la revendication 1 et $R^5$ est mis pour alkyle en C 1-C 8, caractérisé par le fait que l'on met à réagir dans un solvant organique, à des températures comprises entre 0 et 100 degrés C, un ester dialkylique de formule II

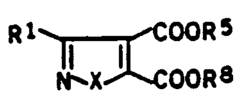

(II),

dans laquelle $R^1$, $R^5$ et X ont les significations données plus haut et $R^8$ est mis pour alkyle en C 1-C 8, avec des quantités environ équimoléculaires d'une amine de formule V.

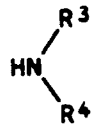

(V)

**Revendications pour l'Etat contractant suivant : ES**

1. Procédé de préparation d'amides acide isoxazol (isothiazol)-5-carboxylique, de formule I

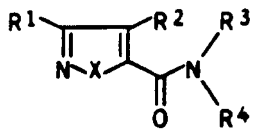

(I),

dans laquelle

X = oxygène ou soufre,

$R^1$ = hydrogène,

alkyle en C 1-C 10, éventuellement substitué par alcoxy en C 1-C 3, halogénalcoxy en C 1-C 3, halogène, cyano ou phényle pouvant être substitué par halogène, alkyle en C 1-C 4, halogénalkyle en C 1-C 4, alcoxy en C 1-C 4, halogénalcoxy en C 1-C 4, alkylthio en C 1-C 4, halogénalkylthio en C 1-C 4, cyano ou nitro,

alcoxy en C 1-C 4,

cyloalkyle en C 3-C 8, éventuellement substitué par alkyle en C 1-C 4 ou halogène,

un reste hétérocyclique à 5 ou 6 chaînons et à un ou deux hétéroatomes choisis dans le groupe constitué par oxygène, soufre et azote, qui peut être substitué par alkyle en C 1-C 4, carboxyle ou alcoxycarbonyle en C 1-C 4,

ou phényle, éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro ou cyano,

$R^2$ = formyle, 4,5-dihydro-oxazol-2-yle ou un reste de formule $COYR^5$ ou $CONR^6R^7$,

où

Y = oxygène ou soufre,

$R^5$ = hydrogène,

alkyle en C 1-C 8 pouvant être substitué par alcoxy en C 1-C 4, alcoxy-en C 1-C 4-alcoxy-en C 1-C 4, halogène, cyano, hydroxy, triméthylsilyle, alkylthio en C 1-C 4, alkylamino en C 1-C 4, dialkylamino en C 1-C 4, alkylsulfinyle en C 1-C 4, alkylsulfonyle en C 1-C 4, carboxyle, alcoxycarbonyle en C 1-C 4, dialkylaminocarbonyle en C 1-C 4, dialcoxyphosphonyle en C 1-C 4, alcaline-iminoxy, benzyloxy, benzoyle, éventuellement substitué par alkyle en C 1-C 4, alcoxy en C 1-C 3 ou par halogène, ou phényle, éventuellement substitué par alkyle en C 1-C 4, alcoxy en C 1-C 4, halogénalkyle en C 1-C 4, halogène, nitro ou cyano, par thiényle, furyle, tétrahydrofuryle, phtalimido ou pyridyle,

alcényle en C 3-C 8, éventuellement substitué par phényle pouvant être substitué par alkyle en C 1-C 4, alcoxy en C 1-C 4, halogénalkyle en C 1-C 4, halogène, nitro ou cyano,

halogénalcényle en C 3-C 6

alcynyle en C 3-C 8, éventuellement substitué par hydroxy ou alcoxy en C 1-C 4,

cycloalkyle en C 3-C 6,

cycloalcényle en C 5-C 6,

phényle, éventuellement substitué par alkyle en C 1-C 4, alcoxy en C 1-C 4, halogénalkyle en C 1-C 4, halogène, nitro, cyano, alcoxycarbonyle en C 1-C 4 ou acylamino,

un reste hétérocycligue à 5 ou 6 chaînons et à un ou deux hétéroatomes choisis dans le groupe constitué par oxygène, soufre et azote, ou un reste benzotriazole,

cycloalcane-en C 6-C 7-imino, phtalimido, succinimido, les restes

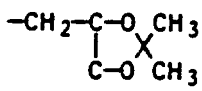 , 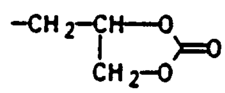 ,

$-CH_2-CH(OH)-CH_2(OH)$,
l'équivalent d'un cation choisi dans le groupe métaux alcalins, alcalino-terreux, manganèse, cuivre, fer, ammonium et ammonium substitué, ou le reste

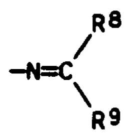 ,

où $R^8$ et $R^9$ représentent, indépendamment l'un de l'autre, alkyle en C 1-C 4, alcoxyalkyle en C 2-C 6, cycloalkyle en C 3-C 6, phényle, furyle ou forment ensemble une chaîne méthylène de formule - $(CH_2)_m$-avec m = 4 à 7 chaînons, $R^9$ pouvant aussi représenter hydrogène,

$R^6$ = hydrogène, alkyle en C 1-C 8 ou cycloalkyle en C 3-C 8, et
$R^7$ = hydrogène ou alkyle en C 1-C 8, ou
$R^6$ et $R^7$ forment une chaîne méthylène à 4 ou 5 chaînons,
$R^3$ = hydrogène,

alkyle en C 1-C 8, éventuellement substitué par hydroxy, halogène, alcoxy en C 1-C 4, alkylthio en C 1-C 4 ou dialkylamino en C 1-C 4, ou cycloalkyle en C 3-C 8, éventuellement substitué par alkyle en C 1-C 4, halogène ou halogénalkyle en C 1-C 4, et

$R^4$ = hydrogène, hydroxyle, alcoxy en C 1-C 4,
alkyle en C 1-C 10, éventuellement substitué par alcoxy en C 1-C 4, halogénalcoxy en C 1-C 4, alkylthio en C 1-C 4, dialkylamino en C 1-C 4, halogène, cycloalkyle en C 3-C 6 ou phényle, qui peut être substitué par halogène, cyano, nitro, alkyle en C 1-C 4, halogénalkyle en C 1-C 4, alcoxy en C 1-C 4, halogénalcoxy en C 1-C 4, alkylthio en C 1-C 4 ou halogénalkylthio en C 1-C 4,

alcényle en C 3-C 10 ou alcynyle en C 3-C 10, éventuellement substitués par halogéne ou alcoxy en C 1-C 4,

cycloalkyle en C 3-C 8, éventuellement substitué par alkyle en C 1-C 6, alkylthio en C 1-C 6, halogénalkylthio en C 1-C 6, alkylthio en C 1-C 6, halogénalkylthio en C 1-C 6, halogène, nitro ou cyano,

dialkylamino en C 1-C 4,

un reste hétérocyclique à 3 à 6 chaînons et à un ou deux hétéroatomes choisis dans le groupe oxygène, soufre et azote, éventuellement substitué par méthyle; naphtyle, éventuellement substitué par alkyle en C 1-C 6, halogénoalkyle en C 1-C 6 ou halogène ou phényle éventuellement substitué par alkyle en C 1-C 6, halogénalkyle en C 1-C 6, alcoxy en C 1-C 6, halogénalcoxy en C 1-C 6, alkylthio en C 1-C 6, halogénalkylthio en C 1-C 6, halogène, nitro, cyano, formyle, alcanoyle en C 1-C 6 ou halogénalcanoyle en C 1-C 6,

ou

$R^3$ et $R^4$ forment, ensemble, un reste de structure $-(CH_2)_n - Y_p- (CH_2)_q-$, n et q représentant 1, 2 ou 3, p = 0 ou 1 et
Y = oxygène, soufre ou N-méthyle, ou le reste de formule

$-(CH_2)_3-CO$,

ainsi que leurs sels acceptables par l'environnement, étant entendu que, dans le cas où X = oxygène, $R^1$ ne représente pas $CH_3$ lorsque $R^2$ est mis pour un groupe COOH, $COOC_2H_5$ ou $CONH_2$ et $R^3$ et $R^4$ représentent, chacun, hydrogène; en outre, $R^1$ ne représente pas phényle lorsque $R^2$ est mis pour $COOCH_3$ et $R^3$ et si les deux restes représentent hydrogène et dans le cas où X = soufre, $R^2$ ne représente pas le groupe COOH ou $CONH_2$ si $R^1$, $R^3$ et $R^4$ sont mis pour hydrogène, caractérisé par le fait que l'on fait réagir un halogénure d'acide isoxazol(isothiazol)-5-carboxylique de formule VI

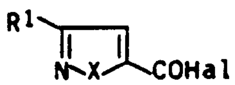

(VI)

avec une amine de formule V

$HNR^3R^4$ (V)

pour obtenir un isoxazol(isothiazol) amide de formule VII

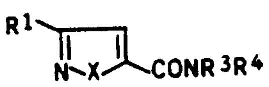

(VII)

et on met à réagir cet amide avec de l'alkyllithium et de l'anhydride carboxylique pour obtenir l'acide isoxazol(isothiazol)-4-carboxylique correspondant de formule IC

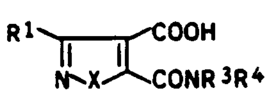

(Ic)

et on transforme cet acide, par estérification avec un alcool de formule IX

$HY-R^5$ (IX),

en présence d'un agent déshydratant, en amide d'acide isoxazol(isothiazol)-5-carboxylique de formule I.

**2.** Herbicide contenant des additifs niertes et 0,1 à 95 % en poids d'un amide d'acide isoxazol(isothiazol)-5-carboxylique de formule I

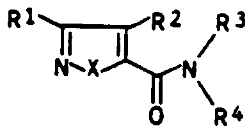

(I)

dans laquelle les substituants $R^1$, $R^2$, $R^3$ et X ont les mêmes significations que dans la formule I selon la revendication 1.

**3.** Procédé de préparation d'amides d'acide isoxazol(isothiazol)-5-carboxylique de formule Ib

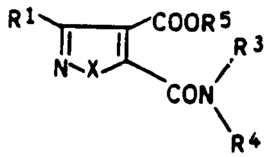

(Ib),

dans laquelle X représente oxygène ou soufre, $R^1$, $R^3$ et $R^4$ ont les significations données dans la revendication 1 et $R^5$ est mis pour alkyle en C 1-C 8, caractérisé par le fait que l'on met à réagir dans un solvant organique, à des températures comprises entre 0 et 100 degrés C, un ester dialkylique de formule II

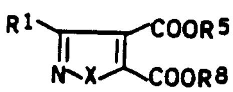

(II),

dans laquelle $R^1$, $R^5$ et X ont les significations données plus haut et $R^8$ est mis pour alkyle en C 1-C 8, avec des quantités environ équimoléculaires d'une amine de formule V.

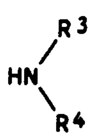

(V)